(19) Europäisches Patentamt
European Patent Office
Office européen des brevets

(11) Publication number: 0 162 738
A1

(12) # EUROPEAN PATENT APPLICATION

(21) Application number: 85400704.4

(22) Date of filing: 09.04.85

(51) Int. Cl.4: C 12 N 15/00
C 12 P 21/00, C 07 K 15/14
C 12 N 7/00, C 12 N 1/20
A 61 K 39/205, G 01 N 33/53
//C12R1:19

(30) Priority: 09.04.84 US 598073
03.04.85 US 719773

(43) Date of publication of application:
27.11.85 Bulletin 85/48

(84) Designated Contracting States:
AT BE CH DE FR GB IT LI LU NL SE

(71) Applicant: MOLECULAR GENETICS RESEARCH & DEVELOPMENT LIMITED PARTNERSHIP
10320 Bren Road East
Minnetonka Minnesota 55343(US)

(72) Inventor: Robbins, Alan K.
902 North Vanburen
Wilmington Delaware(US)

(72) Inventor: Watson, Roger J.
23 Arthur Road
St. Albans Hertfordshire AL1 4SZ(GB)

(72) Inventor: Enquist, Lynn W.
2403 Kentmere Parkway
Wilmington Delaware(US)

(74) Representative: Warcoin, Jacques et al,
Cabinet Régimbeau 26, avenue Kléber
F-75116 Paris(FR)

(54) **Production of pseudorabies virus subunit vaccines.**

(57) Methods and compositions are provided for the identification, cloning and expression of Pseudorabies Virus (PRV) glycoprotein genes in procaryotic and eucaryotic expression systems. Also described are methods for culturing these novel host cell organisms to produce PRV gene products. The PRV related polypeptides produced by the recombinant DNA- techniques described herein may be formulated for use as immunogens in vaccines to protect against PRV infection.

EP 0 162 738 A1

# PRODUCTION OF PSEUDORABIES VIRUS SUBUNIT VACCINES

## TABLE OF CONTENTS


| | Page |
|---|---|
| 1. Field of the Invention | 7 |
| 2. Background of the Invention | 8 |
| 2.1. Recombinant DNA Technology and Gene Expression | 8 |
| 2.1.1. ORI Mutations | 12 |
| 2.1.2. Vaccinia Virus as an Expression Vector | 13 |
| 2.2. Vaccines | 15 |
| 2.3. Pseudorabies | 18 |
| 3. Summary of the Invention | 20 |
| 4. Brief Description of the Figures | 22 |
| 5. Description of the Invention | 25 |
| 5.1. Identification and Isolation of PRV Glycoprotein Genes | 27 |
| 5.2. Insertion of the PRV DNA Fragments Into Cloning Expression Vectors | 30 |

5.3. Preparation of Fusion Proteins.............. 36

5.4. Preparation of Unfused Protein.............. 37

5.4.1. Co-transformation of Host Cells...... 38

5.4.2. Transformation of Host Cells With One Plasmid Carrying Two Genes........... 39

5.4.3. Modification of the Fusion Protein Gene......................... 39

5.5. Identification of the Proteins Produced..... 42

5.6. Purification of the Proteins Produced....... 44

5.7. Formulation of a Vaccine.................... 50

6. Example........................................ 53

6.1. General Procedures Used for Preparation of the Plasmids............................ 54

6.1.1. Plasmid DNA Isolation................ 54

6.1.2. Conditions For Restriction Enzyme Digestions........................... 56

6.1.3. Restriction Enzyme Buffers........... 56

6.1.4. Modification of DNA.................. 57

6.1.5. DNA Polymerase Reaction.............. 58

6.1.6. Gel Purification of DNA Fragments.... 59

6.1.7. DNA Ligation......................... 60

6.2. Production of PRV Stock Infection Pools..... 60

6.3. Extraction of DNA From PRV Nucleocapsids.... 61

6.4. Identification of PRV-Specific Glycoproteins 62

6.4.1. Preparation of Cell Extracts and Immunoprecipitation.................. 62

6.5. Production of Rabbit Antisera Against PRV Glycoproteins.................... 64

6.5.1. Preparation of Goat Anti-PRV Antibody Affinity Column...................... 64

6.5.2. Purification of PRV-Specific Proteins............................ 64

6.5.3. Immunoprecipitation and Western Blot Analysis of PRV-Specific Proteins.... 66

6.6. Cloning of PRV DNA into an Expression Vector.................................. 67

6.6.1. The Expression Vectors pJS413 and pHK412......................... 68

6.6.2. Cloning of Random PRV DNA Fragments.. 69

6.6.3. Identification of Transformants...... 70

6.6.4. Screening for Bacterial Synthesis of PRV Antigens Using Western Blot Analysis.............................. 70

6.6.5. Mapping of pDPR7 and pDPR123 DNA on the PRV Genome....................... 71

6.6.6. Production and Analysis of Antisera Raised Against Cro/PRV/β-Galactosidase Fusion Proteins Expressed by Plasmids pDPR7 and pDPR123.................... 72

6.6.7. Insertion of BamHI 2 Fragment of PRV Genomic DNA Into pBR322.............. 74

6.6.8. Localization and Characterization of the PRV gIII Gene mRNA Coding Sequence............................. 75

6.6.9. Cloning and Expression of the PRV gIII Gene Represented by p7/123...... 78

6.6.10. Identification and Analysis of Transformants that Express the PRV gIII Gene........................... 79

6.7. Construction of ptac Clones................. 80

6.7.1. Construction of ptacNB............... 80

6.7.2. Western Blot Analysis of the Cro/PRV gIII β-galactosidase Fusion Protein Expressed by ptacNB.................. 81

6.7.3. Cloning and Expression of the PRV gIII Gene with its Natural Terminator........................... 82

6.7.4. Construction of p7/123AM Which Produced Both Cro/PRV gIII Fusion Protein and Cro/PRV gIII β-Galactosidase Fusion Protein.................. 83

6.7.5. p7/123AM Transformants............... 84

6.7.6. Insertion of PRV DNA Sequences into a High Copy Number Plasmid............. 85

6.8. Construction of Recombinant Vaccinia Viruses..................................... 86

6.8.1. Construction of an *E. coli* Plasmid Containing the Vaccinia/PRV gIII Recombinant Gene...................... 86

6.8.2. Construction of Recombinant Vaccinia Virus Expressing the PRV gIII Gene of PRV................................. 87

6.8.3. Analysis of Recombinant VPRI Virus... 89

6.9 Use of Heterologous DNA Probes to Localize Glycoprotein Genes Within the PRV Genome..... 90

6.9.1. Cloning of the PRV gII Gene Represented by Plasmid pssgB.............. 93

6.9.2. Cloning and Expression of PRV gII Gene Sequences ...................... 94

6.9.3. Western Blot Analysis of Cro/PRV gII Fusion Proteins...................... 98

7. Vaccination..................................... 99

7.1. Protection of Mice Against Lethal PRV Infection Using Fusion Protein Expressed by ptacNB In a Vaccine Formulation............. 99

7.2. Inoculation of Pigs Using Fusion Protein Expressed By ptacNB in a Vaccine Formulation................................ 100

7.3. Protection of Mice Against Lethal PRV Infection Using the Cro/PRV gIII Fusion Protein Expressed by p7/123AM in a Vaccine Formulation................................ 101

7.4. Protection of Swine Using the Cro/PRV gIII Fusion Protein Expressed by p7/123AM in a Vaccine Formulation..................... 104

8. Deposit of Microorganisms......................... 108

## 1. FIELD OF THE INVENTION

This invention is directed to processes for the production of proteins related to any of the pseudorabies virus (PRV) envelope glycoproteins and to processes and compositions for making and using novel DNA sequences, plasmids and microorganisms (both eucaryotic and procaryotic) to produce such proteins. The present invention comprehends expression of these glycoproteins.

The present invention utilizes recombinant DNA techniques to insert a DNA sequence coding for pseudorabies protein or a portion thereof, into a DNA vector, such as viral DNA, plasmid DNA or bacteriophage DNA, such that the vector is capable of replicating and directing expression of the envelope protein gene in a bacterial host or other single cell system. The resulting recombinant DNA molecule is introduced into host cells to thereby enable production of the PRV protein, or a portion or molecular variant thereof, by the host cells. Recombinant DNA technology may also be used to express a gene in a stably transformed cell line. The protein produced is then isolated, purified and used as an immunogen in a vaccine against infection of pigs by pseudorabies virus.

The present invention also provides a method of producing pseudorabies virus antigens of general importance in veterinary medicine and in microbiological research. This includes use of the pseudorabies virus proteins produced by the present invention as highly reproducible standard antigens for ultrasensitive assays such as radioimmunoassays. These antigens may also be used as diagnostic tools for detection of antibodies to pseudorabies virus in biological samples. Finally, the pseudorabies proteins produced by the present invention will be invaluable in elucidating the mechanisms of infection and pathogenesis of the various isolates of pseudorabies virus.

The structural PRV glycoproteins have been tentatively classified by Hampl et al. (1984, J. Virol. 52: 583-590) in which the major structural glycoproteins are identified by Roman numerals (gI-gV). To be consistent with the literature, glycoproteins referred to in this patent application are identified according to the Hampl nomenclature.

## 2. BACKGROUND OF THE INVENTION

### 2.1. RECOMBINANT DNA TECHNOLOGY AND GENE EXPRESSION

Recombinant DNA technology involves insertion of specific DNA sequences into a DNA vehicle (vector) to form a recombinant DNA molecule which is capable of replication in a host cell. Generally, the inserted DNA sequence is foreign to the recipient DNA vehicle, i.e., the inserted DNA sequence and the DNA vector are derived from organisms which do not exchange genetic information in nature, or the inserted DNA sequence may be wholly or partially synthetically made. In recent years several general methods have been developed which enable construction of recombinant DNA molecules. For example, U.S. Pat. No. 4,237,224 to Cohen and Boyer describes production of such recombinant plasmids using restriction enzymes and methods known as ligation. These recombinant plasmids are then introduced and replicated in unicellular organisms by means of transformation. Because of the general applicability of the techniques described therein, U.S. Pat. No. 4,237,224 is hereby incorporated by reference into the present specification.

Another method for introducing recombinant DNA molecules into unicellular organisms is described by Collins and Hohn in U.S. Pat. No. 4,304,863 which is also incorporated herein by reference. This method utilizes a packaging/transduction system with bacteriophage vectors (cosmids).

Recombinant genes may also be introduced into viruses, such as vaccina virus. Recombinant viruses can be generated by transfection of plasmids into cells infected with virus.

Regardless of the method used for construction, the recombinant DNA molecule must be compatible with the host cell, i.e., capable of autonomous replication in the host cell. The recombinant DNA molecule or virus (i.e., a vaccinia virus recombinant) should also have a marker function which allows the selection of the desired recombinant DNA molecule(s) or virus(es). In addition, if all of the proper replication, transcription and translation signals are correctly arranged on the recombinant DNA molecule, the foreign gene will be properly expressed in the transformed bacterial cells, as is the case with bacterial expression plasmids, or in permissive cell lines infected with a recombinant virus.

As is characteristic of all viruses which infect eucaryotic cells, Pseudorabies virus (PRV) requires a eucaryotic host cell system in which to replicate its genome, express its viral genes and generate its progeny. The signals and control elements for DNA replication, gene expression and virus assembly in eucaryotes differ from those of procaryotes. This is of critical importance when attempts are made to express in procaryotic host cells a gene which is naturally expressed only in eucaryotic cells.

These different genetic signals and processing events control many levels of gene expression; for instance, DNA transcription and messenger RNA translation. Transcription of DNA is dependent upon the presence of a promoter which is a DNA sequence that directs the binding of RNA polymerase and thereby promotes transcription. The DNA sequences of

eucaryotic promoters differ from those of procaryotic promoters. Furthermore, eucaryotic promoters and accompanying genetic signals may not be recognized in or may not function in a procaryotic system.

Similarly, translation of messenger RNA (mRNA) in procaryotes depends upon the presence of the proper procaryotic signals which differ from those of eucaryotes. Efficient translation of mRNA in procaryotes requires a ribosome binding site called the Shine-Dalgarno (SD) sequence on the mRNA. This sequence is a short nucleotide sequence of mRNA that is located before the start codon (AUG) which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, 1979, Methods in Enzymology 68:473.

In addition to these different control elements, many eucaryotic genes contain intervening sequences (introns) which are not present in the mature mRNA or the corresponding double stranded complementary DNA (cDNA) coding for the gene (for review see Chambon, 1981, Sci. Amer. 244:60-71). During mRNA processing in eucaryotes, these introns are spliced out of the mRNA prior to translation (for reviews see Crick, 1979, Science 204:264; Sharp, 1981, Cell 23:643).

Expression of eucaryotic genes cloned in procaryotic hosts requires that the coding sequence of the gene of interest be available in a form uninterrupted by intervening sequences. Therefore, when eucaryotic genetic sequences which contain intervening sequences are to be cloned in a procaryotic host, isolation of mRNA as the source of the genetic information offers an advantage over the isolation of the relevant DNA sequence. Accordingly, the isolated mRNA is transcribed into single stranded complementary DNA (cDNA) copies which are then enzymatically processed into double-stranded DNA molecules which can be inserted into cloning vectors.

Many other factors complicate the expression of eucaryotic genes in procaryotes even after the proper signals are inserted and appropriately positioned. A clear understanding of the nature of these factors and the mechanisms by which they operate is presently lacking. However, one such factor is the presence of an active proteolytic system in E. coli and other bacteria. This protein-degrading system appears to selectively destroy "abnormal" or foreign proteins such as eucaryotic proteins. A tremendous utility, therefore, would be afforded by the development of a means to protect eucaryotic proteins expressed in bacteria from proteolytic degradation. One strategy is to construct hybrid genes in which the eucaryotic sequence is ligated in phase (i.e., in the correct reading frame) with a procaryotic gene. Expression of this hybrid gene results in a fusion protein product (a protein that is a hybrid of procaryotic and foreign or eucaryotic amino acid sequences).

Construction of hybrid genes was the approach used in the molecular cloning of genes encoding a number of eucaryotic proteins, such as somatostatin (Itakura et al., 1977, Science 198:1056), rat pre-proinsulin (Villa-Komaroff et al., 1978, Proc. Natl. Acad. Sci., U.S.A. 75:3727), growth hormone (Seeburg et al., Nature 276:795), and ovalbumin-like protein (Mercereau-Puijalon et al., 1978, Nature 275:505). Additionally, procaryotic promoters have been ligated to such fusion gene sequences in the case of ovalbumin (Fraser et al., 1978, Proc. Natl. Acad. Sci., U.S.A. 75:5936) and β-globin (Guarente et al., 1980, Cell 20:543). The Guarente et al. system involves inserting the lac promoter, including the SD sequence, at varying distances in front of the ATG of the fusion gene. Although the molecular cloning and expression of several eucaryotic genes has been accomplished, this has not heretofore been done for any pseudorabies virus genes

encoding structural glycoproteins. Nor is the state of the art such that expression of foreign or eucaryotic genes in procaryotic host cells may be routinely performed.

Successful expression of a cloned gene requires efficient transcription of DNA, translation of the mRNA and in some instances post-translational modification of the protein. Expression vectors have been used to express genes in a suitable host and to increase protein production. The cloned gene should be placed next to a strong promoter which is controllable so that transcription can be turned on when necessary. Cells can be grown to a high density and then the promoter can be induced to increase the number of transcripts. These, if efficiently translated will result in high yields of protein. This is an especially valuable system if the foreign protein is deleterious to the host cell.

### 2.1.1. <u>ORI MUTATIONS</u>

Most plasmid cloning vectors commonly used in <u>E. coli</u> are derivatives of ColE1-type replicons (for additional information see Oka <u>et al</u>., 1979, Mol. Gen. Genet. <u>172</u>:151-159). The ColE1 plasmids are stably maintained in <u>E. coli</u> strains as monomeric molecules with a copy number of about 15-20 copies per chromosome. Various levels of expression of human and animal health-care protein products of foreign genes inserted into these plasmids have been obtained. However, very high expression levels should be obtained in order for the system to become economically feasible to produce foreign protein products.

One way to obtain large amounts of a given gene product is to clone a gene on a plasmid which has a very high copy number within the bacterial cell. In theory, by increasing the number of copies of a particular gene, mRNA levels should also increase which should lead to increased production of protein. Muesing <u>et al</u>. (1981, Cell <u>24</u>: 235-242) have

isolated the recessive copy-number mutant, Cop plasmid pOP1Δ6 from ColE1. This plasmid, which exists at levels of about 200-300 copies per chromosome, is characterized by a mutation on the plasmid genome about 400 b.p. from the origin of replication of a single base-pair alteration--a G-C to T-A transversion in the copA gene. The copA sequence is thought to code for a small RNA transcript (RNA1) which may act as a negative modulator of the ColE1 DNA replication machinery. It is believed that a disruption of the secondary structure of the RNA by the mutation is responsible for the loss of its negative controlling effects.

Uhlin et al. (1983, Gene 22:255-265) have reported a series of plasmids which are double cop (copy number) mutants of the plasmid R1. These plasmids are unique in that they carry a temperature sensitive mutation which allows the plasmid to be present in moderate numbers per cell at temperature below 30°C. Above 35°C, all control of plasmid replication is apparently lost and the number of plasmid copies per cell increases continuously to nearly 2000. Cell growth and protein synthesis continue at normal rates for 2-3 hours at the higher temperature. During this period, products of genes present on the plasmid may be overproduced. Inhibition of cell growth occurs after 2-3 hours and the cell loses viability. Eventually, plasmid DNA may account for as much as 75% of the total DNA present in the cell.

### 2.1.2. VACCINIA VIRUS AS AN EXPRESSION VECTOR

Vaccinia virus may be used as a cloning and expression vector. The virus contains a linear double-stranded DNA genome of approximately 187 kb pairs and replicates within the cytoplasm of infected cells. These viruses contain a complete transcriptional enzyme system (including capping, methylating and polyadenylating enzymes) within the virus core which are necessary for virus infectivity. Vaccinia virus transcriptional regulatory sequences (promoters) allow

for initiation of transcription by vaccinia RNA polymerase but not by eucaryotic RNA polymerase.

Expression of foreign DNA in recombinant viruses requires the fusion of vaccinia promoters to protein coding sequences of the foreign gene. Plasmid vectors, also called insertion vectors have been constructed to insert the chimeric gene into vaccina virus. One type of insertion vector is composed of: (1) a vaccinia virus promoter including the transcriptional initiation site; (2) several unique restriction endonuclease cloning sites downstream from the transcriptional start site for insertion of foreign DNA fragments; (3) nonessential vaccinia virus DNA (such as the TK gene) flanking the promoter and cloning sites which direct insertion of the chimeric gene into the homologous nonessential region of the virus genome; and (4) a bacterial origin of replication and antibiotic resistance marker for replication and selection in E. coli. Examples of such vectors are described by MacKett (1984, J. Virol. 49: 857-864).

Recombinant viruses are produced by transfection of recombinant bacterial insertion plasmids containing the foreign gene into cells infected with vaccinia virus. Homologous recombination takes place within the infected cells and results in the insertion of the foreign gene into the viral genome. Recombinant viruses can be screened for and subsequently isolated using immunological techniques, DNA plaque hybridization, or genetic selection. These vaccinia recombinants retain their essential functions and infectivity and can be constructed to accomodate approximately 35 kb of foreign DNA.

Foreign gene expression can be detected by enzymatic or immunological assays (immunoprecipitation, radioimmunoassay, or immunoblotting). Additionally, naturally occurring membrane glycoproteins produced from recombinant vaccinia infected cells are glycosylated and may be transported to the cell surface. High expression levels can be obtained by

using strong promoters or cloning multiple copies of a single gene.

## 2.2. VACCINES

There are at least four basic approaches for the control and therapy of viral infections: (1) vaccines that elicit an active immune response; (2) chemotherapeutic agents that inhibit viral replication; (3) agents that induce the synthesis of interferon; and (4) administration of antibodies for passive immunity. All four can be used to prevent infection, but are more effective when applied early in infection. Vaccination, or active immunization is usually ineffective after infection has begun.

Viruses are small and compared to cells, relatively simple in structure. Viruses contain a central core of nucleic acid (either DNA or RNA) surrounded by a protein coat called a capsid. In many viruses, the capsid is surrounded by a loose membranous envelope which is similar to a cellular membrane in composition. Viral nucleic acid contains the information needed for replicating the virus genome and producing the protein components of the virion particle within infected cells.

When an animal is infected by a virus, it reacts to the capsid or envelope protein as a foreign protein. The host may develop immunity, which is manifested by the formation of antibodies and lymphocytes which are capable of recognizing the viral proteins. This may result in inactivation of the virus and lysis of infected cells. Thus, conventional vaccines providing active immunity are prepared by producing large amounts of virus in cell tissue culture or in laboratory animals and then rendering the virus harmless without destroying its immunological properties.

Traditionally this is accomplished either by inactivating the infectivity of the virus (i.e., "killing" the virus, usually by treatment with various chemical agents

such as formaldehyde) for use in inactivated vaccines, or by selecting an avirulent or attenuated (modified) virus for use in live vaccines (attenuated vaccines). Attenuation is obtained by adapting the virus to unusual conditions; for example, to different animal hosts and cell cultures by frequent passages of large viral inocula to select mutants which have lost virulence, or by genetically modifying the virus, and thus to produce safer vaccines. A few vaccines still used in veterinary practice consist of fully virulent infectious virus injected in a site where virus multiplication is of little consequence.

Attenuated virus used in live vaccines are generally excellent immunogens because the attenuated virus multiplies in the host thus eliciting long-lasting immunity. Attenuated virus vaccines induce humoral antibodies directed against both virion and nonvirion proteins and also induce cell-mediated immunity. The vaccines are generally stable if freeze dried, easy to administer, and because of their potency, require only one inoculation. There are, however, a number of problems encountered with the use of live vaccines, such as insufficient attenuation of the virus, genetic instability of the virus, contamination by adventitious viruses in cell cultures used to grow the vaccine virus, and, finally, the vaccine may be unstable (e.g., heat-labile).

While the use of inactivated vaccines (employing "killed" or inactivated virus that does not multiply) avoids the difficulties encountered with the use of live vaccines, killed viruses do not multiply in the immunized animal and usually produce antibodies directed against only virion structural components. There are two major difficulties with inactivated vaccines. Firstly, it is frequently not possible to inactivate all the virus particles. Secondly there is the difficulty of producing enough virus to provide the necessary quantity of the relevant antigen. Other difficulties encountered with the use of inactivated vaccines are that the immunity achieved is brief and often requires additional

immunizations, the antigenic sites may be altered by the inactivating chemical treatment, and thus be less immunogenic or may induce antibodies that are less effective at neutralizing viral infections, and these vaccines frequently do not induce satisfactory levels of secretory antibody.

Subunit vaccines contain only the necessary and relevant immunogenic material, such as the capsid proteins of nonenveloped icosahedral viruses or the peplomers (glycoprotein spikes) of enveloped viruses. Subunit vaccines can be made by isolating the relevant subunit from highly purified viral fractions, or by synthesizing the relevant polypeptide. A major advantage of subunit vaccines is the exclusion of genetic material of viral origin and of toxic, contaminating or otherwise undesirable substances of the host or donor. However, at present, production of subunit vaccines using these methods is too expensive for widespread commercial veterinary use. Recombinant DNA technology has much to offer in the production of subunit vaccines; the molecular cloning and controlled expression of viral genes which encode the relevant immunogenic portions of the virus or its proteins can produce sufficient quantities of the relevant polypeptide for use as an immunogen in a subunit vaccine.

Vaccines are often administered in conjunction with various adjuvants. The adjuvants aid in attaining a more durable and higher level of immunity using smaller amounts of antigen in fewer doses than if the immunogen were administered alone. The mechanism of adjuvant action is complex and not completely understood. However, it may involve the stimulation of phagocytosis and other activities of the reticuloendothelial system as well as a delayed release and degradation of the antigen. Examples of adjuvants include Freund's adjuvant (complete or incomplete), Adjuvant 65 (containing peanut oil, mannide monooleate and aluminum monostearate), the pluronic polyol L-121, Avridine, DDA and mineral gels such as aluminum hydroxide, aluminum

phosphate, or alum. Freund's adjuvant is no longer used in vaccine formulations for humans or for food animals because it contains nonmetabolizable mineral oil and is a potential carcinogen; however, the mineral gels are widely used in commercial veterinary vaccines.

## 2.3. PSEUDORABIES

Pseudorabies is an infectious, naturally occurring viral disease affecting many animal species. The disease is also known as Aujesky's disease, mad itch, and infectious bulbar paralysis. In swine the virus affects the respiratory tract and nervous and reproductive systems. The clinical disease in pigs varies with the age of the animals. The morbidity and mortality rate among young suckling pigs is close to 100%, whereas the mortality rate is usually not as high in pigs over 6 weeks of age. There has been a marked increase in the incidence of the disease in the United States, Mexico, and some Western European countries in recent years.

Swine are the natural hosts of the virus and the primary source of infection for other animals. Infected swine can transmit the virus through nasal secretions, saliva, milk, placenta, and by boars during service.

Swine which survive the disease develop a lasting antibody. These animals are subject to latent infections and become carriers. The infections may become reactivated spontaneously, under certain stress, corticosteroid administrations, or tissue explantation.

It was originally believed that infection was the result of direct contact with pigs. However, infections in cattle and sheep indicate that these species become infected through the respiratory tract. The virus has been found in nasal mucosa, lungs and vagina of cattle. The source of infection in other wildlife (badgers, racoons, skunks, rats) is thought to be from feeding on infected swine carcasses.

The illness in very young and weaning pigs is sudden and progresses rapidly towards death. Depression, tremors, uncoordinated movements, and convulsions may be observed before death. The main clinical features are respiratory signs. The animals develop high fevers and may cough and sneeze. Diarrhea and vomiting may also be observed. Intense pruritus is a characteristic sign of the disease. Additionally, breeding problems with some sows and gilts may occur following an outbreak of the disease.

There is no treatment for the disease once an infection has been initiated and little can be done to reverse the course of the disease after it appears. Anti-PRV serum given to swine exposed to the virulent virus have limited usefulness at this time since it fails to prevent viral replication and shedding of virulent virus, and thus these swine can spread the virus to susceptible animals and are subject to latent infections. At present every effort is made to avoid infection, although administration of a hyperimmune serum to piglets is effective in reducing death losses during an outbreak.

Pseudorabies is caused by a herpesvirus (Herpetoviridae). The components of the complete virus (virion) are the core containing the genome, an icosohedral capsid composed of 162 capsomeres, each of which has subunits, variable amounts of globular material asymmetrically arranged around the capsid (i.e., the tegument) and an outer membrane or envelope that encloses the entire structure. The core is about 75 nm in diameter, the nucleocapsid is 105-110 nm and the complete enveloped virion is about 180 nm in diameter. The genome consists of a linear double-stranded DNA duplex of approximately 90 x $10^6$ daltons in size and can be divided into two covalently linked components, the L and S components. The DNA of PRV consists of a short unique sequence bounded by inverted complementary repeats and a long unique sequence. The short unique

sequence inverts so that two isomeric forms of the PRV genome exist.

The sequence of biochemical and physical events of the infective cycle is similar to that of many DNA viruses and typical of herpesviruses affecting mammalian species. The infective cycle requires from 15 to 19 hours and begins with attachment of the virus to the cell and ends with the cessation of viral replication and cellular lysis. Nascent virus particles are released from the cell beginning 6 to 9 hours post-infection (Gustafson, 1981, Disease of Swine, 5th Edition, Eds: A.D. Leman et al., Iowa State University Press, Ames).

## 3. SUMMARY OF THE INVENTION

Methods and compositions are provided for the cloning and expression of PRV genes or portions thereof encoding viral structural proteins in single-cell host organisms. Also described are methods for culturing these novel single-cell organisms to produce the pseudorabies virus gene product and methods for purifying the gene product. The PRV-related proteins produced by the recombinant DNA techniques described herein may be formulated for use as immunogens in vaccines to protect against viral infection. Alternatively, these PRV-related proteins may be used to generate antisera which can be employed in the detection of virus for diagnostic purpose as well as being used directly to screen sera of pigs for exposure to PRV virus or previous vaccination.

The isolated pseudorabies virus genes or gene fragments were inserted into bacterial plasmids and a vector to form recombinant DNA molecules which serve as biologically functional replication units. The recombinant DNA molecules were constructed so as to facilitate both the replication and expression of the PRV genetic sequences upon transformation of a suitable bacterial cell or alternatively, as with virus vectors, infection of compatible host cells. Additionally,

the present invention contemplates the introduction of a pseudorabies virus gene into a eucaryotic cell line such that the gene is constitutively expressed in the transformed cell.

Bacterial expression plasmids were constructed so that transformed microorganisms actively expressing the PRV genetic sequences could be identified in one step. Methods are described for the isolation, stabilization, and purification of the expressed gene products. These methods take advantage of the fact that many fusion proteins produced in bacterial cells will be resistant to degradation in the host cell. Methods are provided for the co-production of both the fusion and the unfused product. Methods are also provided for the isolation and purification of the co-aggregate forming proteins. Methods are also described for isolating the expressed product and for its use in formulating a vaccine. Finally, methods are provided for using live recombinant vaccinia virus as immunological tools and as live vaccines against infectious agents.

The proteins thus produced may be used in vaccine formulations. Vaccines produced by recombinant DNA technology can address many of the problems involved in the production of conventional vaccines. First, very little virus is needed in order to isolate the relevant genetic sequences which are used in the construction of recombinant DNA molecules. The use of such recombinant molecules to transform appropriate host cells allows for the production of effective vaccines against viruses that cannot easily be grown in cell tissue culture or in laboratory animals. Second, recombinant DNA vaccines are a type of subunit vaccine, and contain, at worst, incomplete viral genetic material. Third, since large scale bacterial fermentation can be used, the vaccines can be produced economically in large and used quantities. Fourth, recombinant viruses can be used as live vaccines and for the construction of polyvalent vaccines, as in the case with vaccinia virus.

## 4. BRIEF DESCRIPTION OF THE FIGURES

The present invention may be more fully understood by reference to the following detailed descriptions of the invention, examples of specific embodiments of the invention, and the appended figures.

In all figures within this text, restriction endonuclease enzyme recognition sites are indicated by the following abbreviations: BamHI (Bm); BglII (Bg) BstEII (Bs); HindIII (H3) KpnI (Kp); NcoI (Nc); PstI (Ps); PvuII (Pv); SacI (Sc); SalI (Sa); SmaI (Sm); SphI (Sp); XhoI (Xh); XmaI (Xm) Restriction endonuclease enzymes themselves are indicated by their typical abbreviations.

Unless otherwise indicated, the figures which follow are not drawn to scale.

Figure 1 shows: (A) a generalized restriction map of PRV genomic DNA for the enzymes BamHI and KpnI and the genome location of PRV DNA sequences represented in plasmids pDPR7 and pDPR123; (B) the map loci of PRV DNA inserts of plasmids pDPR7 and pDPR123 are indicated. The genome location of PRV gIII glycoprotein gene and direction of transcription of PRV gIII mRNA is indicated by the arrow.

Figure 2 represents the construction of plasmid pPRV49, a recombinant plasmid derived from the BamHI 2 fragment of genomic PRV DNA; and the construction of p7/123 a recombinant plasmid derived from a portion of pPRV49 DNA and pBR322.

Figure 3 represents the nucleotide sequence of the coding region of the PRV gIII gene. The DNA sequence of plasmid p7/123 and pK64 was determined for both strands; represented here is the sequence of the noncoding strand only. Nucleotides are numbered to the right of the DNA sequence, which is transcribed from left to right. The location of PRV DNA sequences contained by plasmids pDPR7 and pDPR123 are indicated. The positions of the KpnI, NcoI, PvuII, SacI, SalI, and XhoI cleavage sites are indicated. The predicted PRV gIII amino acid sequence is numbered at the

left. Overscored are putative transcriptional regulatory CAT and TATA signals, respectively. Underscored are relevant restriction endonuclease recognition sites. A number of amino acids that show homology with HSV-1 gC (at the 3' end) are underscored. The percent homology between HSV-1 gC and PRV gIII over the entire gene is estimated to be 28.5%.

Figure 4 represents a comparison of the predicted amino acid sequences of PRV gIII and HSV-1 gC using the PROTHOM program of Conrad and Mount (1982, Nucl. Acid. Res. 10:21) and the dot alignment program developed by Wilbur and Lipman (1983, Wilbur and Lipman, Proc. Natl. Acad. Sci., U.S.A. 80:726; 1984 SIAM (Soc. Inc. Appl. Math.) J. Appl. Math., 44:557). The window size was set at 10 in each program for each sequence, with k-tup=1 and gap penalty=1 in the case of the Wilbur and Lipman algorithm. An approximately 28.5% identity exists between the two protein sequences for the 520 amino acids sequence compared. The upper line in each set is the HSV-1 gC sequence and the lower line is the predicted PRV gIII amino acid sequence. Amino acid sequences are represented by the single-letter code. Amino acid residues which are homologous in PRV gIII and HSV-1 gC are indicated by (:). A number of amino acid homologies indicated on Figure 3 are underscored to show their location.

Figure 5 represents the construction of plasmid Polink 26 from pBR328 and a DNA fragment containing multiple restriction endonuclease cleavage sites; and the construction of pK64 derived from Polink 26 and the KpnI J fragment of genomic DNA.

Figure 6 represents the construction of plasmid pNB412, a recombinant plasmid derived from a portion of p7/123 and pHK412, an expression vector which carries the lac promoter. The coding sequences are transcribed in the direction of the arrow and are under the control of the E.coli lac promoter/operator (plac).

Figure 7 represents the construction of plasmid ptacNB, a recombinant plasmid derived from pNB412 and the expression

clone p830 containing the tac promoter. The PRV gIII gene coding sequences are transcribed in the direction indicated by the arrow and are under the control of the ptac promoter (ptac).

Figure 8 represents the construction of plasmid pNT7/123, derived from ptacNB and pK64. The PRV gIII gene coding sequences are under the control of the tac promoter and contain the natural terminator sequences for the gene.

Figure 9 represents the construction of plasmid pHK7/123AM + ori which contains PRV DNA sequences and the high copy number origin from pOP1Δ6.

Figure 10 represents a scheme for construction of vaccinia virus recombinants expressing the PRV gIII gene. The PRV gIII gene sequences from plasmid pNT7/123 are inserted into the insertion vector downstream from the vaccinia promoter and flanked by the vaccinia virus TK; the TK gene is non-functional. This plasmid is transfected into tissue culture cells infected with wild-type virus. Homologous recombination should result in the insertion of the PRV gIII gene into the viral genome of the TK locus. $TK^-$ vaccinia virus containing the PRV gIII gene are then selected.

Figure 11 represents the construction of plasmid pPRS3 containing the SalI 3 fragment of genomic DNA which hybridizes to HSV-1 gB DNA; and the construction of pssgB which was derived from pPRS3 and pBR322.

Figure 12 is a restriction map of plasmid pssgB. The location of the 5' end of the gene and direction of transcription is indicated by the arrow.

Figure 13 represents the partial nucleotide sequence of the PRV gII gene and the predicted amino acid sequence of the gII protein. The position of several pertinent restriction sites are indicated. Underscored are potential glycosylation sites. Nucleotides are numbered to the right of the DNA sequence, which is transcribed from left to right. The predicted amino acid sequence is numbered at the left. The

upstream control sequences (i.e., CAT, TATA) and the putative ATG are underlined. There are approximately 300 bps at the 3' end of this gene which are missing.

Figure 14 represents a comparison of the predicted amino acid sequences of the PRV gII and HSV-1 gB using the PROTHOM program and the PRTALN program as described in Figure 14. An approximately 49.2% homology was found between the two protein sequences for the 900 amino acids compared. The upper line in each set is the predicted amino acid sequence for HSV-1 gB and the lower line is the predicted PRV gII amino acid sequence. Amino acid residues are represented by the single-letter code. Amino acid residues which are homologous in PRV gII and HSV-1 gB are indicated by (:).

Figure 15 represents the construction of plasmid pXXgB, a recombinant plasmid derived from pJSXma and the expression vector pHK414.

Figure 16 represents the construction of plasmid pPR10 in which PRV gIII gene sequences are under the control of the tac promoter, and contains the artificial translation initiation complex (TIC), the triple terminator sequence (TTS), and the terminator (TI TERM) from the *E. coli* rrn B operon.

Figure 17 represents the construction of plasmids pR14 and pR15 in which PRV gIII gene sequences are under the control of the tac promoter, and contain TIC, TTS, and TI Term sequences.

## 5. DESCRIPTION OF THE INVENTION

This invention relates to the use of recombinant DNA techniques to produce pseudorabies virus (PRV) polypeptides which can be used as immunogens in vaccine formulations. More specifically, the production of polypeptides related to structural proteins of PRV is described. Because immunity to PRV is produced by humoral and cellular responses to the surface proteins of the viral envelope, a subunit vaccine may

be formulated which contains only those viral surface proteins (subunits) responsible for evoking the immune response. All other portions of the virus can be excluded from the vaccine. The subunit vaccine formulation will efficiently protect the recipient animal against PRV infection. For instance, the PRV structural proteins or any antigenically significant portion thereof may be used in a subunit vaccine which would efficiently protect a recipient animal against infection; if the recipient animal is a pregnant sow, the fetus or embryo will also be protected from PRV infections *in utero*.

The polypeptides and antisera produced according to the method of the present invention may also be used for diagnostic purposes. For instance, the polypeptides may be used as antigens for *in vitro* immunoassays to determine antisera titers in animals; such *in vitro* assays are currently known and used by those skilled in the art. Alternatively, antisera, antibodies, or monoclonal antibodies directed against the polypeptides produced herein may be used in immunoassays for the detection of PRV which may be present in the body fluids or feces of infected animals.

The recombinant bacterial plasmids, constructed as described herein, provide for host cell production of a protein which is a PRV-related polypeptide, and which is stable and resistant to host cell degradation; such plasmids enable the generation of large quantities of a PRV-related protein or fusion protein containing immunological and antigenic determinants of a PRV structural protein. Similarly, recombinant viruses constructed as described herein, provide for expression of foreign genes to be used in another embodiment of the present invention.

Since the techniques described herein may be used to produce polypeptides related to any of the PRV structural proteins, it can readily be seen that various immunogens and vaccine formulations can be prepared. Vaccinia virus

recombinants can also be used as live vaccines against infectious agents.

In the present invention, protein components of purified PRV virions indicated the presence of at least four glycoproteins. Because of the large size of the PRV genome (approximately 150,000 base pairs) and the prior lack of any data on localization of glycoprotein genes, a simple and effective strategy was utilized in the present invention to locate a PRV glycoprotein that involved use of antibodies against authentic PRV glycoproteins, cloning of random genomic PRV DNA fragments in expression vectors and Western blot analysis.

The method of this invention may be divided into the following stages for the purpose of description: (1) identification and isolation of the relevant PRV gene or gene fragment; (2) insertion of the gene or gene fragment into a cloning expression vector to form a recombinant DNA molecule which is used to transform single-cell organisms; (3) identification and growth of the transformed single-cell organisms which are capable of replicating and expressing the gene; (4) identification and purification of the gene product; (5) construction and isolation of vaccinia virus recombinants expressing the PRV gene; (6) determination of the immunopotency of the gene product by assessment of its ability to elicit the production of neutralizing antibodies; and (7) formulation and use of subunit vaccines.

## 5.1. IDENTIFICATION AND ISOLATION OF PRV GLYCOPROTEIN GENES

The PRV glycoprotein gene may be obtained from any strain of PRV virus. Isolation of a PRV glycoprotein gene (or portion thereof) involves first isolating PRV DNA, generating DNA fragments, and identifying the fragments which contain the glycoprotein gene sequences. Before identification, the PRV DNA fragments are usually ligated into cloning vectors that are used to transform the host

cells; this enables generation of multiple copies of the PRV DNA fragments so that an ample supply is available for analysis and identification procedures.

The PRV DNA can be obtained either (a) by isolating DNA directly from virus purified from eucaryotic cells infected with the virus or (b) from bacteriophage or plasmids which contain portion(s) of the viral genome encompassing the PRV gene.

In order to generate the PRV DNA fragments, the DNA may be cleaved at specific sites using restriction enzymes; alternatively, one can use DNase to fragment the DNA; or the DNA can be physically sheared, as, for example, by sonication. The linear DNA fragments can then be separated according to size by standard techniques, including, but not limited to, agarose and polyacrylamide gel electrophoresis and column chromatography.

Any restriction enzyme or combination of restriction enzymes may be used to generate the PRV DNA fragment(s) containing the PRV glycoprotein gene sequence provided the enzymes do not destroy the immunopotency of the PRV gene protein product. For example, the antigenic site of a protein can consist of approximately 7 to 14 amino acids. Thus, a protein of the size of the PRV glycoproteins may have many discrete antigenic sites, possibly thousands considering overlapping sequences, secondary and tertiary structure considerations, and processing events such as acetylation, glycosylation or phosphorylation. Therefore, many partial PRV polypeptide gene sequences could code for an antigenic site. Consequently, many restriction enzyme combinations may be used to generate DNA fragments which, when inserted into an appropriate vector, are capable of directing in a host cell the production of PRV specific amino acid sequences comprising different antigenic determinants.

Transformation of host cells with these recombinant DNA molecules incorporating the PRV DNA fragments enables the generation of multiple copies of viral DNA which can then be

analyzed to identify the fragment that contains the PRV gene sequence. Insertion of DNA restriction fragments into a cloning vector is easily accomplished when the cloning vector has complementary cohesive termini. However, if the complementary restriction sites used to fragment the PRV DNA are not present in the cloning vector, the ends of the PRV DNA or the cloning vector may be modified. Such modifications include producing blunt ends by digesting back single-stranded DNA termini or by filling in single-stranded termini with the appropriate enzyme(s) so that the ends can be blunt end ligated. Alternatively, any site desired may be produced by ligating nucleotide sequences (linkers) onto the DNA termini; these ligated linkers may comprise specific chemically synthesized oligonucleotides containing restriction enzyme recognition sites. According to other methods, the cleaved vector and the PRV DNA fragment may be modified by homopolymeric tailing. (For a review see Morrow, 1979, Methods in Enzymology 68:3).

Identification of the specific DNA fragment containing the PRV gene may be accomplished in a number of ways. Firstly, it is possible to sequence the viral DNA fragments (Maxam and Gilbert, 1980, Methods in Enzymology 65:49) and then identify the fragment containing the PRV protein gene sequence based upon a comparison of the predicted amino acid sequence to the amino acid sequence of the PRV protein. Secondly, the fragment containing the PRV protein gene may be identified by mRNA selection. In this procedure the PRV DNA fragments are used to isolate complementary mRNA's by hybridization. Immunoprecipitation analysis of the *in vitro* translation products of the isolated mRNAs identifies the complementary PRV DNA fragments encoding for a particular gene product(s). Thirdly, PRV specific mRNAs may be selected by adsorption of mRNA associated polysomes isolated from PRV-infected cells, to immobilized antibodies directed against PRV proteins. A radiolabeled PRV cDNA (complementary DNA) can be synthesized using the selected mRNA (extracted

from the adsorbed polysomes) as a template. The radiolabeled cDNA may then be used as a probe to localize on the PRV genome the gene encoding this particular gene product (Alwine et al., Methods in Enzymology 65:499). Fourthly, as described in the embodiment of the present invention, DNA fragments can be identified by the shotgun cloning/expression procedure described herein. Finally, cloned Herpes simplex virus (HSV) genes may be used as heterolgous DNA probes in a Southern blot analysis of PRV genomic DNA to localize regions of homology.

Alternatives to isolating the PRV gene include but are not limited to, chemically synthesizing the gene sequence itself (provided the sequence is known) or making cDNA to the mRNA which encodes the PRV gene. To accomplish this, mRNA specific for PRV is isolated from virus-infected cells. By standard techniques the isolated mRNA is then converted to a cDNA copy and inserted directly into a cloning vector.

Transformation of host cells with recombinant DNA molecules that incorporate the isolated gene, cDNA or synthesized DNA sequence enables generation of multiple copies of the gene. Thus, the gene may be obtained in microgram quantities by growing transformants, isolating the recombinant DNA molecules from the transformants and retrieving the inserted gene from the isolated recombinant DNA. Alternatively, microgram quantities of the PRV protein gene may be obtained from the source of the PRV gene, e.g., PRV in infected animal cells, or bacteria or phage containing the viral gene in recombinant expression plasmids. The viral or plasmid DNA may be isolated, fragmented, fractionated and sized by the same methods which may be used to locate the gene.

## 5.2. INSERTION OF THE PRV DNA FRAGMENTS INTO CLONING EXPRESSION VECTORS

Once isolated, the PRV gene or gene fragment is inserted into an appropriate expression vector, i.e., a vector which

contains the necessary elements for transcription and translation of the inserted gene (see Roberts and Lauer, 1979, Methods in Enzymology, 68:473).

In order to obtain efficient expression of the gene (or a portion of the gene), a promoter must be present in the expression vector. RNA polymerase normally binds to the promoter and initiates transcription of a gene or a group of linked genes and regulatory elements (called an operon). Promoters vary in their "strength", i.e., their ability to promote transcription. For the purpose of molecular cloning it is desirable to use strong promoters in order to obtain a high level of transcription and, hence, expression of the gene. Depending upon the host cell system utilized, any one of a number of suitable promoters may be used. For instance, when cloning in E. coli, its bacteriophages, or plasmids, promoters such as the lac promoter, trp promoter, recA promoter, ribosomal RNA promoter, the $P_R$ and $P_L$ promoters of coliphage lambda and others including but not limited to lacUV5, ompF, bla, lpp and the like, may be used to direct high levels of transcription of adjacent DNA segments. Additionally, a hybrid trp-lacUV5 (tac) promoter or other E. coli promoters produced by recombinant DNA or other synthetic DNA techniques may be used to provide for transcription of the inserted gene.

When cloning in a eucaryotic host cell, enhancer sequences (e.g., the 72 bp tandem repeat of SV40 DNA or the retroviral long terminal repeats or LTRs, etc.) may be inserted to increase transcriptional efficiency. Enhancer sequences are a set of eucaryotic DNA elements that appear to increase transcriptional efficiency in a manner relatively independent of their position and orientation with respect to a nearby gene. Unlike the classic promoter elements (e.g., the polymerase binding site and the Goldberg-Hogness "TATA" box) which must be located immediately 5' to the gene, enhancer sequences have a remarkable ability to function upstream from, within, or downstream from eucaryotic genes;

therefore, the position of the enhancer sequence with respect to the inserted PRV gene is less critical.

Specific initiation signals are also required for efficient gene transcription and translation in procaryotic cells. These transcription and translation initiation signals may vary in "strength" as measured by the quantity of gene specific messenger RNA and protein synthesized, respectively. The DNA expression vector, which contains a promoter, may also contain any combination of various "strong" transcription and/or translation initiation signals. Thus, any SD-ATG combination that can be utilized by host cell ribosomes may be employed; such combinations include but are not limited to the SD-ATG combination from the cro gene or the N gene of coliphage lambda, or from the E. coli tryptophan E, D, C, B or A genes. Additionally, any SD-ATG combination produced by recombinant DNA or other synthetic techniques may be used.

Any of the methods previously described for the insertion of DNA fragments into a vector may be used to ligate a promoter and other control elements into specific sites within the vector.

Accordingly, PRV genetic sequences containing those regions coding for the structural glycoproteins can be ligated into an expression vector at a specific site in relation to the vector promoter and control elements so that when the recombinant DNA molecule is introduced into a host cell the PRV genetic sequence can be expressed (i.e., transcribed and translated) by the host cell. The recombinant DNA molecule may be introduced into appropriate host cells (including but not limited to bacteria, virus, yeast, mammalian cells or the like) by transformation, transduction or transfection (depending upon the vector/host cell system). Transformants are selected based upon the expression of one or more appropriate gene markers normally present in the vector, such as ampicillin resistance or tetracycline resistance in pBR322, or thymidine kinase

activity in eucaryotic host systems. Expression of such marker genes should indicate that the recombinant DNA molecule is intact and is replicating. Expression vectors may be derived from cloning vectors, which usually contain a marker function; such cloning vectors may include, but are not limited to the following: SV40 and adenovirus, vaccinia virus vectors, yeast vectors, bacteriophage vectors such as lambda gt-WES-lambda B, Charon 28, Charon 4A, lambda gt-1-lambda BC, lambda gt-1-lambda B, M13mp7, M13mp8, M13mp9, or plasmid DNA vectors such as pBR322, pAC105, pVA51, pACYC177, pKH47, pACYC184, pUB110, pMB9, pBR325, Col E1, pSC101, pBR313, pML21, RSF2124, pCR1, RP4, pBR328 and the like.

Expression of viral genes in a eucaryotic host systems may result in the production of a polypeptide that serves as a better immunogen than those produced by procaryotic transformants because the protein may be expressed in a more natural form; similarly expression in eucaryotic host cell systems may be advantageous due to secondary protein modifications which occur during eucaryotic biosynthesis. Such secondary protein modifications include but are not limited to acylation, glycosylation, methylation, phosphorylation, sulfation and the like. The eucaryotic host cell might modify (i.e., phosphorylate or glycosylate) the clonally derived polypeptide product at sites identical to those of the natural protein.

In addition, bacterial host cell strains may be chosen which inhibit the action of the promoter unless specifically induced. In this way greater than 95% of the promoter-directed transcription may be inhibited in uninduced cells. In certain operons the addition of specific inducers is necessary for efficient transcription of the inserted DNA; for example, the lac operon is induced by the addition of lactose or IPTG (isopropylthio-β-D-galactoside). A variety of other operons, such as trp, pro, etc., are under different controls. The trp operon is induced when tryptophan is absent in the growth media; and the $P_L$ promoter of lambda can

be induced by an increase in temperature in host cells containing a temperature sensitive lambda represor, e.g., cI857. Thus, expression of the genetically engineered PRV protein may be controlled. This is important if the protein product of the cloned gene is lethal or detrimental to host cells. In such cases, transformants may be cultured under conditions such that the promoter is not induced, and when the cells reach a suitable density in the growth medium, the promoter can be induced for production of the protein.

One such promoter/operator system is the so-called "tac" or trp-lac promoter/operator system (referred to as "tac") (Russell and Bennett, 1982, Gene 20:231-243; DeBoer, European Patent Application, 67,540 filed May 18, 1982). This hybrid promoter is constructed by combining the -35 b.p. (-35 region) of the trp promoter and the -10 b.p. (-10 region or Pribnow box) of the lac promoter (the sequences of DNA which are the RNA polymerase binding site). In addition to maintaining the strong promoter characteristics of the tryptophan promoter, tac is also controlled by the lac repressor. This construction may explain the higher efficiency of expression of this hybrid promoter with respect to either one of the parental promoters.

Gene expression can also be controlled at the level of translation. In non-suppressor cells transformed with amber, ochre, or opal modified plasmids (See Section 5.4.3.), translation of the mRNA produced from the plasmid DNA stops at the nonsense codon and only the desired protein (unfused) is expressed. In an appropriate host (one that is able to "suppress" the nonsense codon), some of the ribosomes, "read through" the nonsense codon and produce fusion protein. Since the level of suppression varies in different host cells, it is possible to regulate the ratio of (unfused) protein to fusion protein by choosing the appropriate suppresor host cell.

The expression of genes maybe regulated in several other ways. For example, in order to improve the initiation of

translation, an A/T rich DNA segment may be inserted between the $SD^{cro}$ and the ATG initial codon for the start of translation. An oligomer which contain an A/T rich sequence may be synthesized using state-of-the-art nucleic acid chemistry. A particularly useful method is described in U.S. Patent Application Serial No. 491,099 by Kempe, et al., filed May 5, 1983 which is incorporated herein by reference. This A/T rich segment may be needed to destabilize the secondary structure of the mRNA within this region and thereby facilitate ribosome binding and subsequent translation along the message.

In many recombinant clones studied only one translational stop codon (e.g., TAG, TAA or TGA) is present for termination of translation, and no transcriptional-stop like sequences are found in the 3' untranslated region of the clones. Therefore a triple translational stop sequence, with all 3 nonsense codons may be inserted in phase with the reading frame of the protein. Again, this linker is synthesized using methods referred to above. This construction should then provide for efficient termination of translation by assuring proper translational termination with little chance of read-through into the 3' non-coding region of the mRNA.

Recent studies have shown that the tryptophan attenuator region of the tryptophan operon provides for the efficient termination of mRNA transcription from the DNA. This extremely stable stem and loop structure is characteristically GC-rich and posseses dyad symmetry, followed by an oligo-(T) stretch at the site of termination similar to the well known features of rho-independent transcription termination sites in procaryotes. By taking advantage of this system, a sequence comprising the attenuator portion of the tryptophan operon may be placed 3' of the translational stop codons of the genome. This construction may now allow for the effective termination of transcription of the mRNA.

The regulation of expression of any gene includes, but is not limited to the construction and use of promoter/operator systems, A/T rich sequences, transcriptional and translational termination sequences, and any other modified gene construct (either naturally occurring or synthetically made) which contributes to an "optimal" system for expression of a protein in a compatible host cell.

## 5.3. PREPARATION OF FUSION PROTEINS

To maximize the level of expression of the cloned PRV DNA sequences it may be desirable to ligate these sequences to a gene encoding another protein, such as a host cell protein. If the host cell protein is large and inherently contains an assayable function additional advantages are obtained because expression of the ligated genes may result in a fusion protein product that can be identified on the basis of its large molecular weight and assayable function.

For example, production of a PRV-related polypeptide fused to β-galactosidase, hereinafter referred to as PRV/β-galactosidase fusion protein offers several advantages for cloning and expression of a PRV genetic sequence in an E. coli host. First, fusion protein production simplifies the identification and isolation of the protein product. The unfused PRV-related protein (i.e., unfused to β-galactosidase) produced by E. coli transformants is smaller than the fusion protein and as such might co-migrate with several other host proteins when analyzed by SDS-polyacrylamide gel electrophoresis. However, a fusion protein can often be easily detected and identified by SDS-polyacrylamide electrophoresis (SDS-PAGE) due to its unique high molecular weight. Second, retention of enzymatic function of the host protein can be used to identify transformants and may also allow for an approximation of the level of protein production (hence expression) directed by the vector using a colorimetric assay specific for

β-galactosidase activity according to the method of Miller (pages 47-55, and 352-355 in Experiments in Molecular Genetics, Cold Spring Harbor Press, New York, NY, 1972). Third, as discussed in Section 2.1., hybrid gene sequences, e.g., procaryotic nucleotide sequences ligated to eucaryotic nucleotide sequences, when expressed in procaryotes result in production of fusion proteins which may be protected from host cell proteolysis.

The present invention is not limited to the production of a PRV/β-galactosidase fusion protein; any gene of either eucaryotic or procaryotic origin may be ligated in phase with a PRV genetic sequence to provide advantages similar to the PRV/β-galactosidase fusion protein product. Examples include, but are not limited to: galactokinase; trp D, E or leader; pilus genes; and eucaryotic genes, such as thymidine kinase, β-globin, SV-40 T-antigen, bovine growth hormone or Rous Sarcoma Virus transforming gene.

In order to construct a hybrid gene which encodes a fusion protein, the two genes must be joined within open coding sequences such that the correct reading frame of each is maintained. Also, as previously explained, if the host cell is a strain which represses or inhibits the action of the promoter, the fusion protein will be produced only in response to induction.

## 5.4. PREPARATION OF UNFUSED PROTEIN

As previously explained, transformants which produce unfused proteins, generally do so in smaller quantities than transformants which produce fusion proteins; this is true even when the gene sequence for the unfused protein is under the control of an inducible promoter. In addition, the unfused proteins produced by bacterial transformants may be less stable than fusion proteins.

In an alternate embodiment of the present invention, a host cell transformant can be engineered to produce, for

example, large quantities of both fused PRV/β-galactosidase fusion protein and unfused PRV related proteins which will coaggregate and can be purified easily. (See U.S. Patent Application Serial No. 573,642 filed January 25, 1984 to George et al. which is herein incorporated by reference.) Three embodiments of this mode of the invention are described in the subsections below.

### 5.4.1. CO-TRANSFORMATION OF HOST CELLS

Any appropriate host cell can be co-transformed with two or more plasmids. One plasmid carries the desired unfused protein gene and one plasmid carries a fusion protein gene. Each gene should be under the control of a promoter and translational control elements. The same or different promoter systems may be used to control expression of each gene. If a different promoter is used for each gene sequence then the ratio of the synthesis of the sequences may be controlled by varying the degree of induction of each promoter.

In addition, the two plasmids may carry different selectable drug markers for antibiotic resistance (e.g., tetracycline and ampicillin resistance) and may be maintained within the same cell if grown in the presence of the two antibiotics. With both plasmids in the cell, synthesis of the fusion protein and the labile unfused protein can occur.

These transformed cells produce insoluble aggregates which contain both the unfused proteins and the large fusion protein. There is no requirement that the fusion protein be a PRV fusion protein. In fact, any combination of fusion protein with unfused protein will have similar aggregation and stabilization properties.

For example, a significant stabilization of the unfused bovine growth hormone (hereinafter referred to as BGH) protein was observed when two plasmids were used to transform one host cell, each plasmid directing the synthesis of its

respective protein: unfused BGH and porcine parvovirus/β-galactosidase fusion protein (PPV/β-galactosidase). Pulse-chase experiments indicated that the half-life of the unfused modified-BGH proteins had increased from a 1-2 minute level to between 30 minutes and 1 hour. Additionally, when these proteins were produced in large amounts within the cell, they form insoluble aggregates which contain both the unfused BGH protein and the large PPV/β-galactosidase fusion protein. Subsequent purification of the aggregate proteins indicated that the protein could be readily obtained in gram quantities. The cloning and expression of nucleotide sequences encoding BGH is described in U.S. Pat. Application Serial No. 548,917 to George et al., filed November 7, 1983.

### 5.4.2. TRANSFORMATION OF HOST CELLS WITH ONE PLASMID CARRYING TWO GENES

In an alternate embodiment of the present invention, plasmids which carry both the unfused protein gene and the larger fusion protein gene on a single vector are designed and constructed. As described supra for the co-infection scheme, this embodiment of the invention is suitable for any combination of fusion protein and any unfused protein.

The plasmids are constructed such that each gene on the plasmid has its own promoter and expression control elements. The same or different promoter systems may be used to control expression of each gene, therefore this embodiment of the present invention also allows for the regulation of the ratio of the fusion protein and the desired gene product.

### 5.4.3. MODIFICATION OF THE FUSION PROTEIN GENE

In one embodiment of the present invention, a recombinant plasmid which encodes a PRV fusion protein is altered at the junction of the two gene sequences which comprise the fusion protein gene. A chain termination sequence such as amber (TAG), ochre (TAA), or opal (TGA) is

located between the two gene sequences; the chain terminator must be in phase with the translational reading frames of both gene sequences. Such an alteration may be accomplished by cleaving the plasmid at a restriction site (or sites) located between the two gene sequences and then ligating a nucleotide linker sequence encoding a chain terminator such as amber, ochre, or opal into the cleaved site on the plasmid so that the chain terminator is in phase with the translational reading frames of both gene sequences. (See for example U.S. Pat. Application Serial No. 510,551, to Watson et al., filed July 6, 1983 which is herein incorporated by reference.)

Introduction of these amber, ochre, or opal modified plasmids into a host cell containing the appropriate tRNA suppressors results in the synthesis of both an unfused protein as well as a fusion protein (since suppression is significantly less than 100%). A tRNA suppressor is a tRNA that has undergone a modification (generally the result of a mutation of the tRNA gene) that allows the tRNA to recognize the termination codon and results in the occasional but not regular insertion of an amino acid at the site of the termination codon. Therefore, host cells carrying the suppressor tRNA characteristically produce both the unfused protein and a fusion protein of normal length. Every nonsense or termination suppressor has a characteristic efficiency, indicated by the ratio of the two protein chains produced.

There are at least two ways to introduce the amber, ochre or opal modified plasmids into a suppressor cell background: (1) the transformant (i.e., a host cell transformed with amber, ochre or opal modified plasmid) can be infected with a lysogenic transducing phage that (TBS,9,14,19,24) carries the appropriate suppressor tRNA gene (e.g.,ϕ80/pSU3 carries the supF suppressor of amber mutations); or (2) the amber, ochre or opal modified plasmids can be used to transform cell lines which contain suppressor

tRNAs of amber, ochre, or opal respectively. Examples of such strains include but are not limited to LE392 (containing *sup*E and *sup*F suppressors of amber mutations), YMC (containing *sup*F), and C600 (*sup*E). The various amber suppressor tRNA genes in *E. coli* include but are not limited to: *sup*B, *sup*C, *sup*D, *sup*E, *sup*F, *sup*G, *sup*L, *sup*M, *sup*N, *sup*O, *sup*P, *sup*U, *sup*V; the various ochre suppressor tRNA genes in *E. coli* include but are not limited to: *sup*B, *sup*C, *sup*G, *sup*L, *sup*M, *sup*N, *sup*O, *sup*V; and the various opal suppressor tRNA genes in *E. coli* include but are not limited to: *sup*K (see Bachmann and Low, 1980, Microbiological Reviews 44(1): 1-56).

The host cells containing the appropriate suppressor tRNA gene are transformed with the amber, ochre, or opal modified plasmids which can produce the protein as both a fusion protein and as an unfused protein (the proportions of fused to unfused protein produced depends upon the extent of suppression in the host cell).

The amber, ochre, or opal modified plasmids may be further modified to ensure that translation of the corresponding mRNA transcript will terminate at the 3'-terminus of the fusion protein gene. Proper chain termination is important because it contributes to the overall efficiency of translation. A number of approaches which may be used to effect chain termination are described below. In one embodiment, all three chain termination sequences may be inserted in tandem at the 3'- region of the fusion protein gene so that they are in phase with the translational reading frame of the gene sequence. The presence of the chain terminator sequences in tandem will reduce the chance of read-through, consequently, translation will terminate at the chain termination codons on the mRNA transcript.

Alternatively, one or more chain termination sequences of the appropriate type may be inserted into the 3'- region of the fusion protein gene. For example, an amber modified

plasmid may be further modified by the insertion (in phase) of one or more opal or ochre sequences at the 3'- end of the fusion protein gene. When this plasmid is inserted into a host cell containing an amber tRNA suppressor (such as supD, supE, supF, supP, supU, supV in *E. coli*) that does not suppress ochre or opal, the host cell will produce both the fusion protein and the unfused protein and translation of the fusion protein will terminate at the location of the opal and/or ochre codons located at the 3'- end of the mRNA transcript.

In a similar fashion, an opal modified plasmid may be further modified by the insertion (in phase) of one or more amber or ochre sequences at the 3'- end of the fusion protein gene. When this plasmid is inserted into a host cell containing an opal tRNA suppressor (such as supK in *E. coli*) that does not suppress amber or ochre, the host cell will produce both the fusion protein and the unfused protein and translation of the fusion protein will terminate at the location of the amber and/or ochre codons located at the 3'- end of the mRNA transcript.

Similarly, an ochre modified plasmid may be further modified by the insertion (in phase) of one or more opal sequences at the 3'- end of the fusion protein gene. When this plasmid is inserted into a host cell containing an ochre tRNA suppressor (such as supB, supC, supG, supL, supM, supN, supO, supV in *E. coli*) that does not suppress opal, the host cell will produce both the fusion protein and the unfused protein and translation of the fusion protein will terminate at the location of the opal codon (or codons) located at the 3'- end of the mRNA transcript.

### 5.5. IDENTIFICATION OF THE PROTEINS PRODUCED

Once transformants which contain the correct DNA construction are identified, analyses of the immunoreactivity and antigenicity of the products expressed by the

transformants (e.g., fusion proteins or PRV-related polypeptides) are required. Immunological analyses of these expression products of the PRV DNA sequences are especially important because the ultimate goal is to use the PRV-related products (e.g., the fusion proteins, PRV-related polypeptides or proteins) so produced in vaccine formulations. The analysis of immunoreactivity is most easily carried out using antisera directed against the PRV structural protein, whereas immunogenicity may be evaluated by determining test animal antisera titers which develop in response to immunization with the PRV-related product and the ability of the antisera so produced to neutralize PRV infectivity.

Identification of the PRV-related products described in this invention is, therefore, based upon two requirements. First, the PRV-related product can be produced in response to induction of the promoter (i.e., if the promoter is inducible). It is also possible that promoters used to express PRV genes in other systems (such as yeast) will be constitutive rather than inducible. For example, the PRV gene can be transferred to cell lines that constituitively synthezise the PRV-gene product. Second, the PRV-related product should be reactive with antibodies directed against the relevant PRV glycoprotein; the PRV-related product should be immunoreactive whether it results from the expression of an entire PRV gene sequence, a portion of a PRV gene sequence or from two or more gene sequences which are ligated to direct the production of a fusion protein. This reactivity may be demonstrated by standard immunological techniques, such as immunoprecipitations, immunodiffusion, radio-immune competition, immunoelectrophoresis or the immunological detection of proteins which were separated by polyacrylamide gel electrophoresis and then transferred to nitrocellulose.

## 5.6. PURIFICATION OF THE PROTEINS PRODUCED

Crude vaccines may be prepared using intact cells, cell extracts (*E. coli* or the like) or cell lysates. Recombinant viruses such as the recombinant vaccinia virus, baculovirus (insect viruses), and the like may also be used advantageously to produce vaccines. Live or killed virus recombinants can be made against infectious agents such as PRV. These vaccines can be made to express only a single PRV antigen.

Certain vaccines might require the use of purified proteins. Furthermore, the purified proteins may be used in diagnostic procedures. In order to purify the proteins produced, cells containing the cloned gene are grown up in a large volume, and the protein produced after induction of the promoter is isolated from such cells or from the medium if the protein is excreted. The protein may be isolated and purified either by standard chromatography, including ion exchange, affinity or sizing resins, by centrifugation, or by any other standard technique for the purification of proteins.

Fusion proteins can form aggregates when overproduced in *E. coli* and unfused proteins (i.e., unfused to β-galactosidase) may co-aggregate with the fusion protein within the host cell. An efficient method for isolating these aggregate-forming proteins (for example, separating the Cro/PRV proteins from the Cro/PRV/β-galactosidase proteins) is particularly useful for isolating the fusion proteins produced in the present invention. These proteins or the isolated aggregates can then be used in vaccine formulations. Indeed, the aggregates which may be used in vaccine formulations are also a significant intermediate product in the purification of proteins. These aggregates may be isolated by the methods described in the embodiment of the present invention. Purification, hereinafter referred to as aggregate purification, involves the extraction, separation and/or purification of aggregate-forming proteins from

protein mixtures such as lysates of cell cultures by disruption of cells followed by washing the aggregated material. Additionally, the aggregated material may be solubilized by the addition of a solvent that is both a strong hydrogen acceptor and a strong hydrogen donor (or a combination of solvents each having one of these properties); the aggregate-forming proteins may then be precipitated by dilution with a compatible buffer or may be further purified by column chromatography. Suitable protein solvents include, but are not limited to urea (from about 4M to about 8M), formamide (at least about 80%, volume/volume basis), guanidine hydrochloride (from about 4M to about 8M). Other solvents which are capable of solubilizing aggregate-forming proteins are, for example SDS (sodium dodecyl sulfate), 70% formic acid. In some instances these solvents may be inappropriate for use due to the possibility of irreversible denaturation of the proteins, hence a lack of immunogenicity and lack of activity. Although guanidine hydrochloride and other similar agents are denaturants, studies indicate that this denaturation is not irreversible and that renaturation may occur upon removal (by dialysis, for example) or dilution of the denaturant, allowing re-formation of immunologically and/or biologically active protein. It should be emphasized that many variations of these procedures are possible and may be used in the present invention.

One embodiment of the fusion protein isolation technique is outlined as follows (hereinafter referred to as the non-denaturing aggregate purification procedure): the cell pellet is quick frozen using dry ice/methanol, weighed, and 3-4 g of cells are resuspended in at least 25 mℓ of a buffer solution [e.g., 50 mM Tris-HCl (tris hydroxymethylaminomethane-hydrochloride), pH 8.0, 2 mM EDTA (ethylenediaminetetraacetic acid) and 200 mM NaCl]. That is, the cells are suspended in a buffer solution at an approximate concentration of from about 100 to 200 grams of cells/liter. Concentrations less than about 160 grams/liter are preferred. To this suspension

lysozyme is added to a final concentration of about 130 μg/mℓ and the resulting mixture is allowed to stand at 4°C for 20 minutes with occasional shaking. Nonidet P40 (NP-40, Shell trademark, polyoxyethylene (9) p-tert-octylphenol), a non-ionic detergent used to solubilize membranes, is added to a final concentration of about 0.1% and the solution mixed. Then, the suspension is homogenized for approximately 1 minute using a Polytron grinder (Brinkman Instruments, Westbury, NY) or equivalent.

The suspension is centrifuged at 8,000 x g for 30 minutes, and the pellet resuspended in a wash buffer, e.g., 20 mM Tris-HCl (pH 7.2), 1 mM EDTA, 150 mM NaCl and 2% Triton-X 100 [polyoxyethylene (9-10) p-tert-octylphenol, a non-ionic detergent], and homogenized with the Polytron grinder. This step of centrifugation, washing, and grinding may be repeated to further wash the pellet and remove as much cellular debris as possible.

In another embodiment, the cell pellet may also be quick frozen. Cells are resuspended in 50 mℓ of lysis buffer (50mM Tris, pH7.9, 200mM NaCl, 2mM EDTA, 2mM β-ME). To this suspension, approximately 200 μg/mℓ (10mg) of lysozyme is added and the mixture is allowed to stand on ice for 10 minutes. Zwittergent 3-14 (Calbiochem-Behring Corp, LaJolla, CA) is added, to a final concentration of 0.5% and the suspension incubated on ice for 10 minutes. The suspension is then sonicated using a soni-probe for about 3 x 10 pulses at a setting of 3, to reduce the viscosity of the suspension. After sonication $MgCl_2$ is added to 5mM and DNase is added to 50μg/mℓ for 15 minutes at 37°C. The sonicated suspensions are overlayed in two 50 mℓ round bottom plastic centrifuge tubes onto 10 mℓ of 40% sucrose defind in PBS or saline tris EDTA buffer (STE) and centrifuged in a Sorvall swinging bucket rotor for 30 minutes at 13,000 r.p.m. The supernatant is removed and, PBS or STE is added to the pellet which is resuspended by vortexing or by sonication.

Alternatively, the pellet of aggregates may be further treated by the addition of a denaturant (hereinafter referred to as the denaturing aggregate purification procedure) as follows: The suspension is centrifuged, the supernatant removed completely and the pellet resuspended in about one-fifth volume of 6M guanidine hydrochloride (in distilled water). For instance, aggregate protein derived from 3 g of cells washed with 25 mℓ of buffer should be resuspended at this step in 5 mℓ of 6M guanidine hydrochloride solution. It may be difficult to resuspend the pellet at this stage and sonication or homogenization may be required in order to obtain a homogenous solution. The solution is allowed to stand at 22°C for 20 minutes and is then centrifuged at 8,000 x g for 30 minutes to remove debris, saving the supernatant which at this point contains the fusion protein.

The fusion protein is precipitated from the guanidine hydrochloride supernatant by the addition of about four volumes of aqueous buffer. Almost any aqueous buffer may be used at this stage; however, the addition of a small amount of non-ionic detergent, such as 0.5% NP-40 or Triton X-100 may be helpful. This suspension is allowed to stand at 4°C for 30 minutes and is then centrifuged at 8,000 x g for 10 minutes. The supernatant is discarded, the pellet (containing the fusion protein precipitate) is resuspended in an appropriate buffer, e.g., Phosphate Buffered Saline (PBS) in any appropriate volume. Brief sonication or homogenization may aid in obtaining a homogenous suspension or slurry.

It may be desirable to remove any remaining DNA from the suspension of aggregates or the fusion protein precipitate before using either as an immunogen. To this end the suspension of aggregates or fusion protein precipitate is pelleted and resuspended in wash buffer containing no detergent. To either suspension a final concentration of about 10 mM $MgCl_2$ and 2 µg/mℓ Deoxyribonuclease I (P.L. Biochemicals, Milwaukee, WI) is added. After incubation at

37°C for 30 minutes, the suspension of aggregates or fusion protein precipitate is pelleted by centrifugation, washed with buffer, repelleted, and resuspended in fresh buffer, thus removing most of the deoxyribonuclease.

According to the denaturing aggregate purification procedure, fusion proteins may also be solubilized in 8M urea and subsequently dialysed to remove the urea. This is carried out by adding solid urea to a final urea concentration of 8M. Also added are EDTA to 10 mM, DTT to 1 mM and Tris, pH 7.5 to 50 mM. The urea is then removed by dialysis usually against PBS. The pH and concentration of DTT can be adjusted to enhance solubilization.

Another embodiment of the fusion protein isolation technique involves purification of aggregate proteins from larger volumes of culture. This method is a modification of the aggregate purification procedure described supra. The cell pellet is quick frozen in a dry ice/ ethanol bath, weighed, and approximately 30-60 g of cells are resuspended in 125 mℓ of B1P buffer (50 mM Tris pH 8.0, 10 mM EDTA) at 37°C with shaking for 10 minutes. The partially resuspended cells are homogenized using a Polytron grinder (Brinkman Instruments, Westbury, NY). To this suspension 50 mg of lysozyme (10 mg/mℓ in B1P) is added while the cells are further homogenized and the cells are placed at room temperature for 20-30 minutes. Nonidet P40 (described supra) is added to a final concentration of 0.1% and the mixture is homogenized using a Polytron grinder for 5 minutes.

The cell lysate is centrifuged at 26,890 x g for 20 minutes using a SS-34 Sorvall rotor and the supernatant is discarded. The pellet is resuspended in 150 mℓ B2P buffer (10 mM Tris pH 7.5, 1 mM EDTA, 1% Triton X-100), homogenized for 2-5 minutes, and centrifuged for 20 minutes at 26,890 x g. This step is repeated, and the pellet (containing the aggregate protein) is then washed two times with water. Finally, the aggregate protein is resuspended in water using the Polytron grinder.

In an alternate embodiment of the present invention, aggregates purified as described supra containing fused and unfused protein are solubilized, and soluble unfused viral protein is isolated. Aggregate protein (4 mg/mℓ) is suspended in 20 mℓ of buffer (75 mM Tris-HCl, 10M urea, and 0.5M β-mercaptoethanol), sonicated for 2 minutes, and heated at 65°C for 30 minutes. the suspension is centrifuged at 20,000 x g for 20 minutes, and the supernatant is diluted by the addition of an equal volume of water.

The sample is applied to a 30 mℓ DE52 (Diethylaminoethyl cellulose, Whatman Chemical Separation Ltd., Kent, England) column. The column is washed with several volumes of buffer (30 mM Tris-HCl, 4M urea, 50 mM β-mercaptoethanol, pH 9.0). The protein is eluted from the column using a gradient from 0 to 150 mM NaCl (400 mℓ of buffer containing 30 mM Tris-HCl, 4M urea, 50 mM β-mercaptoethanol, pH 9.0).

Fractions are collected and an aliquot of each fraction is analyzed by SDS-PAGE for the presence of unfused protein. Fractions containing unfused protein are pooled and dialyzed against 30 mM Tris-HCl, pH 9.0. Finally, the unfused protein is neutralized and concentrated at least 10 fold by ultrafiltration using an Amicon Model #8050 Ultrafiltration Cell (Amicon Corp., Danvers, MA).

Alternatives to purification of the protein from genetically engineered recombinants include synthesis of polypeptides of PRV which are immunogenic and antigenic. Once the PRV glycoprotein genes are identified by the techniques described herein, their amino acid sequences can be deduced. Then, any method such as solid phase protein synthesis and the like can be used to synthesize these polypeptides or relevant portions thereof. These polypeptides maybe used in vaccine formulations or in diagnostic assays.

## 5.7. FORMULATION OF A VACCINE

The purpose of this invention is to produce a polypeptide related to a PRV structural protein which may be used as an immunogen in a subunit vaccine to protect against PRV infections. If the PRV-related protein produced is immunoreactive with specific neutralizing antibodies, it would be expected that the PRV-related protein would elicit an immune response capable of neutralizing the PRV virus *in vivo*. Vaccines made from genetically engineered immunogens should be safer than conventional vaccines made from attenuated or killed virus because there is no risk of infection of the recipient.

For pseudorabies virus, the subunit vaccine offers several advantages. First, the subunit vaccine is safer than live attenuated vaccines. Infectious material is eliminated and thus acute or latent infections are avoided. Furthermore, subunit vaccines can be designed to be compatible with control and eradication programs. Subunit vaccines need contain only those viral antigens required to elicit a protective immune response. Other viral antigens that are not required for such a protective response can be excluded from the vaccine. These latter antigens can thus serve as diagnostic markers to enable differentiation of subunit vaccinated animals from PRV-infected animals. Subunit vaccinated animals will not have antibodies against these excluded (diagnostic) antigens, whereas PRV-infected animals will. With conventional vaccines, on the other hand, animals that have been vaccinated cannot be distinguished from those that have been infected, since both should have antibodies against most if not all viral antigens.

The serologic test to distinguish vaccinates from infected animals would be a test for an excluded antigen. An infected animal would react positively with this "Infection Test" whereas subunit vaccinates would remain negative. The excluded antigen would thus serve as a diagnostic reagent to

be used to distinguish those animals that have been vaccinated with the subunit vaccine from those animals that have been PRV-infected.

Therefore 3 classes of animals can be distinguished: (1) seronegative animals which have never been infected or vaccinated would be negative by standard serum neutralization (SN) tests; (2) animals which have been given the subunit vaccine will be positive by the SN test. However, a serologic test for one of the excluded (diagnostic) antigens (an antigen present in whole virus but absent from the vaccine) would indicate that subunit vaccinated animals are negative for the diagnostic antigen; and (3) infected animals will be SN positive and serologically positive for the excluded (diagnostic) antigen.

Presumably, vaccinated animals are more resistant to infection than are non-vaccinated animals. Current control programs require maintenance of totally susceptible seronegative animals in order to maintain a herd certified to be free of virus. In such a herd, introduction of the virus results in disease and seroconversion in most if not all of the herd. In one embodiment of the present invention, the subunit vaccine would allow maintenance of "Infection-Test" - negative, subunit - vaccinated animals which are protected from disease and partially or largely resistant to infection. If infection did occur in the herd, the "Infection Test" would detect these animals which are infected, and these can be eliminated.

Alternatively, the genetically engineered PRV product may be used to produce antibodies for use in passive immunotherapy or as a diagnostic tool. For instance the PRV specific antibody may be labeled with a dye, an enzyme such as peroxidase, ferritin, a fluorescent compound, or a radioactive compound. Such a labeled antibody may be used to detect PRV antigens (and, therefore, pseudorabies virus) in various body fluids obtained from animals suspected to be infected with PRV. Such techniques for immunoassay <u>in vitro</u>

diagnostic assays are well known and may be applied to body fluids obtained from mucous membranes, semen, blood and excrement. Finally, the genetically engineered product may be used as antigen in an in vitro immunoassay to determine and monitor the titers of antisera in vaccinated animals.

Although the PRV/β-galactosidase fusion protein product itself may be useful in a vaccine formulation, it may be advantageous to first remove and/or modify all or part of the β-galactosidase moiety in order to produce a more effective PRV-related immunogen.

While whole cells or crude extracts of induced transformants may be used to vaccinate animals, genetically engineered PRV-related protein may also be isolated and purified from the host cells using standard protein isolation techniques or techniques previously described (see Section 5.6). In fact, any of the methods used to isolate aggregates from host cells may be used in vaccine formulation. The final purified product may then be adjusted to an appropriate concentration and formulated with any suitable vaccine adjuvant and packaged for use. Suitable adjuvants include but are not limited to: surface active substances, e.g., hexadecylamine, octadecylamine, lysolecithin, dimethyl-dioctadecylammonium bromide, N,N-dioctadecyl-N'-N-bis(2-hydroxyethyl-propane diamine), methoxyhexadecylglycerol, and pluronic polyols; polyanions, e.g., pyran, dextran sulfate, poly IC, polyacrylic acid, carbopol; peptides, e.g., muramyl dipeptide, dimethylglycine, tuftsin; oil emulsions; and alum. Freund's adjuvant is no longer used in vaccine formulations for human or for food animals because it contains nonmetabolizable mineral oil and is a carcinogen, however, the mineral oils are widely used in commercial veterinary vaccines. Finally, the protein product may be incorporated into liposomes for use in a vaccine formulation, or may be conjugated to polysaccharides or other polymers.

The genetically engineered DNA molecules described herein allow great flexibility for vaccine production. For

example, a vaccine could be formulated using a PRV-related protein produced by transformants containing any portion of the genetic sequences of PRV encoding a structural protein or multiple copies of such sequences in tandem. The PRV genetic sequences (or portion thereof) may be ligated to genes that encode other immunogens so that the fused protein product could be used in the preparation of multivalent vaccines. Additionally, the PRV genetic sequences (or portion thereof) could be ligated to other PRV gene sequences (or portions thereof) in any combination. Finally, the genetic sequences of PRV may be reorganized to increase the immunogenicity of the vaccine. For example, the PRV genetic sequences may be altered so that the protein product presents specific epitopes to the immune system (e.g., an antigenic site of the protein that is normally only partially exposed may be presented to the immune system); or the regions of the genetic sequences that encode immunosuppressive portions of the protein can be deleted; or the regions that separate epitopes can be deleted.

## 6. EXAMPLE

According to the method of the present invention, a combined cloning/expression protocol was utilized to identify a pseudorabies virus (PRV) glycoprotein gene within the PRV genome. Antibodies were made against authentic PRV glycoproteins. PRV genomic DNA was isolated from purified nucleocapsids and fragmented into approximately 500 base pair lengths. These random PRV DNA fragments were inserted into an expression vector and the PRV DNA sequences were expressed as proteins fused to β-galactosidase. PRV specific glycoprotein sequences were identified in these PRV-β-galactosidase fusion proteins by Western blot analysis. The PRV DNA sequences represented in the expression plasmids were mapped to the 20.3 kb BamHI 2 fragment within PRV genomic DNA. Further subcloning of the PRV DNA genome more precisely

located these sequences to a 3.6 Kb SphI/NcoI fragment. Appropriate PRV DNA sequences were cloned into expression vectors and analyzed by restriction enzyme mapping and DNA sequencing. The gene product which was isolated as a fusion protein was then formulated for use as an immunogen. The procedures are described in detail below.

### 6.1. GENERAL PROCEDURES USED FOR PREPARATION OF THE PLASMIDS

The following subsections describe the general procedures and materials used for DNA isolation, enzyme reactions and ligation reactions.

#### 6.1.1. PLASMID DNA ISOLATION

Host cell bacteria *Escherichia coli*, (*E. coli*) were transformed (Gautier and Bonewald, 1980, Molec. Gen. Genet. 178: 375) and large (microgram) quantities of plasmid DNA were isolated from cultures of *E. coli* transformants grown in L-broth. Briefly, overnight cultures of cells (10 mℓ) were prepared in L-broth containing 100 μg/mℓ ampicillin. Ten mℓ cultures were added to one liter of L-broth containing ampicillin in a 2 liter flask. Cultures were incubated at 37°C with shaking until the $OD_{600}$ was approximately 0.8 to 1.0. Cells were amplified by addition of 300 μg/mℓ spectinomycin to the medium and further incubated for at least 16 hours. Cells were pelleted in a GSA-rotor (Sorvall, Wilmington, DE) at 6,000 RPM for 15 minutes at 4°C. Cells were then resuspended in 30 mℓ of 25% sucrose, 0.05 M Tris, pH 8.0 and 10 mℓ of lysozyme [5 mg/mℓ in 0.25 M Tris, pH 8.0, 10 mℓ 0.25 M, EDTA, pH 8.0] were added and incubated on ice for 15 minutes. 50 mℓ of detergent mix (5 mℓ 10% Triton X-100 in 0.25 M Tris, pH 8.0, 125 mℓ 0.25mℓ EDTA, 25 mℓ 1.0 M Tris, pH 8.0, 345 mℓ water) was added quickly, mixed gently and incubated on ice for 30 minutes. The cells were centrifuged in Ultraclear tubes (Beckman Instruments,

Fullerton, CA; 1 inch X 3.5 inch) in a SW28 rotor (Beckman Instruments) at 27,000 RPM for 1.5 hours at 15°C. Predigested Pronase (Sigma, St. Louis, MO) was added to the supernatant at a final concentration of 250 µg/mℓ and the mixture was incubated at 37°C for 30 minutes.

The DNA was phenol extracted by adding one-half the volume of STE [TE-1 (0.01M Tris, pH 8.0 and 0.001 M EDTA, pH 8.0) plus 0.15 M NaCl] saturated phenol. After mixing, the mixture was centrifuged in a Beckman TJ-6 rotor at 1,000 RPM for 25 minutes at 20°C. The aqueous layer was removed and ethanol precipitated by adding one-tenth the volume of 3 M Na Acetate and 2 times the volume of -20°C ethanol. DNA was precipitated at -20°C overnight or -70°C for 30 minutes. The DNA was pelleted by centrifugation in a GSA-4 rotor at 6,000 RPM for 20 minutes at 4°C. Air dried pellets were resuspended in a total volume of 19 mℓ in TE-10 (0.02 M Tris, pH 8.0, 0.01 M EDTA, pH 8.0). The plasmid DNA was then purified by centrifugation to equilibrium in cesium chloride-ethidium bromide density gradients (Maniatis _et al._, Molecular Cloning, 1982, Cold Spring Harbor Laboratory p. 93). The cesium chloride solution was centrifuged at 48,000 rpm for 42 hours or 40,000 rpm for 72 hours in a 70.1 Ti or 50 Ti rotor. The plasmid DNA band was collected, the ethidium bromide extracted using isoamyl alcohol and the DNA was then ethanol precipitated. Ethanol precipitates were centrifuged in a SS34 rotor (Sorvall) for 20 minutes at 10,000 RPM to pellet the DNA. Pellets were air-dried and resuspended in TE-1 buffer.

Small quantities of plasmid DNA free from chromosomal DNA and excess protein were quickly prepared by the following alkaline minipreparation.

Host cell bacteria were grown to saturation. The cells (1 mℓ) were centrifuged for 20 seconds in an Eppendorf centrifuge and the supernatant was removed. Cells were resuspended by vortexing in 100 µℓ GTE buffer (50 mM glucose, 25 mM Tris, pH 8.0, 10 mM EDTA) and then incubated for 5

minutes at room temperature. 200 μℓ of alkali-SDS solution (0.2N NaOH, 1% SDS) was added, mixed well, and incubated for on ice for 5 minutes; 150 μℓ of 5 M acetate solution was then added. The sample was vortexed vigorously for 2 seconds and placed on ice for a further 5 minutes. The mixture was then centrifuged for 1 minute in an Eppendorf centrifuge and supernatants transferred to new Eppendorf tubes. 0.9 mℓ of 100% ethanol was added, mixed well and incubated for 1 minute at room temperature. The mixture was then centrifuged for 5 minutes and the supernatant discarded. The pellet was washed with 0.9 mℓ 70% ethanol and centrifuged in an Eppendorf centrifuge for 20 seconds. Pellets were dried under vacuum and resuspended in 20 μℓ of water.

### 6.1.2. CONDITIONS FOR RESTRICTION ENZYME DIGESTIONS

Restriction enzymes used in the present application were obtained from New England Biolabs, Inc., Beverly, MA, unless otherwise indicated. An enzyme unit is defined as the amount required to digest 1.0 μg of lambda DNA in 1 hour at 37°C in a total reaction mixture of 50 μℓ.

All restriction enzyme total digestions were accomplished under the following conditions: each 1 μg DNA was incubated with 2 units of enzyme at 37°C for 60 minutes in 20 μℓ buffer.

### 6.1.3. RESTRICTION ENZYME BUFFERS

The buffer used for *KpnI*, *SacI* or *SmaI* digestions consisted of: 6.6 mM Tris-HCl (pH 7.4), 6.6 mM $MgCl_2$ and 6.6 mM β-ME (β-mercaptoethanol).

The buffer used for *BglII*, *EcoRI*, *PstI* or *PvuII* digestions consisted of: 60 mM NaCl, 6.6 mM Tris-HCl (pH 7.4), 6.6 mM $MgCl_2$ and 6.6 mM (β-ME).

The buffer used for BamHI, BstEII, NcoI, SalI, SphI, or XhoI digestions consisted of: 150 mM NaCl, 6.6 mM Tris-HCl (pH 7.4), 6.6 mM $MgCl_2$ and 6.6 mM β-ME.

It should be noted that whenever two or more restriction endonuclease reactions are performed on DNA, the reaction in low salt buffer is accomplished before the reaction in high salt buffer. If two enzymes require the same buffer, then the reactions may be performed simultaneously.

### 6.1.4. MODIFICATION OF DNA

S1 nuclease degrades RNA or denatured DNA (i.e., single-stranded DNA) into mononucleotides, but will not (under proper conditions) degrade double-stranded DNA or DNA/RNA hybrids. One unit of S1 nuclease (Boehringer Mannheim, Indianapolis, IN) is defined as the amount of enzyme required to acid solubilize 1 μg of denatured calf thymus DNA in 30 minutes at 37°C. The reaction buffer used for S1 nuclease consisted of 30 mM sodium acetate (pH 4.6), 250 mM NaCl, 1 mM $ZnSO_4$ and 5% glycerol. S1 digestions were accomplished by incubating 2000 units of enzyme with 0.1 μg DNA and 10 μg RNA at 45°C for 30 minutes.

Exonuclease VII (Exo VII) degrades single-stranded DNA (ssDNA). The mechanism of action of Exo VII appears to be that of a processive exonuclease. One unit of Exo VII (Bethesda Research Laboratories, Rockville, MD) is defined as the amount of enzyme which produces 1 nmol of nucleotide monomers in 30 minutes at 37°C using linear, denatured [$^3$H]-SV40 DNA as a substrate. The reaction buffer used for Exo VII consisted of 10 mM Tris-HCl (pH 7.9), 100 mM NaCl, 10 mM β-ME and 8 mM EDTA. Exo VII digestions were accomplished using 4 units of Exo VII per 0.1 μg DNA and 10 μg RNA in a 250 μℓ reaction volume for 1 hour at 45°C.

### 6.1.5. DNA POLYMERASE REACTION

DNA polymerases catalyze the stepwise elongation of a DNA chain. Chain growth is in the 5' to 3' direction (i.e., addition of nucleotides occurs at the 3'-terminus) and the sequence of the polymer is determined by that of the template because the incoming nucleotide must be complementary to the template, i.e., form the correct base pair with the template strand. The known DNA polymerases cannot initiate chain synthesis, thus DNA synthesis requires a primer strand with a free 3'-hydroxyl terminus annealed to a template strand. Chain elongation proceeds by the nucleophilic attack of the 3'-hydroxyl terminus of the primer on the 5'-phosphate of the incoming nucleotide. The new chain is base paired and antiparallel to the template strand. As a result of the DNA polymerase reaction the single-stranded template strand is "filled-in" or converted to a double-stranded DNA molecule.

A second important feature of the DNA polymerases is the "proof-reading" function. A 3' to 5' exonuclease activity associated with DNA polymerase I acts on nonbase-paired termini and can degrade both single- and double-stranded DNA in the 3' to 5' direction yielding mononucleotides. Thus an incorrectly added nucleotide is removed before polymerization continues, and the fidelity of the enzyme is further enhanced. DNA polymerase I also has a 5' to 3' exonuclease activity specific for double-stranded DNA (yielding 5'-mononucleotides and oligonucleotides) which can also excise mismatched regions in DNA.

Proteolytic treatment of DNA polymerase I by the method of Klenow (Klenow et al., 1971, Eur. J. Biochem. 22: 371) yields two fragments, large and small. The large fragment or Klenow fragment (76,000 daltons) retains the polymerase and 3'-5' exonuclease activity whereas the small fragment retains the 5'-3' exonuclease activity. One unit of DNA polymerase I or DNA polymerase I-large fragment (New England Biolabs, Beverly, MA) is defined as the amount converting 10 nmoles of

deoxyribonucleotides to an acid insoluble form in 30 minutes at 37°C. The assay conditions for both enzymes are the same except that the reaction mixture for DNA polymerase I contains 67 mM $KPO_4$ (pH 7.5) while the reaction mixture for the Klenow fragment contains 40 mM $KPO_4$ (pH 7.5) in the following buffer: 6.6 mM $MgCl_2$, 1.0 mM β-ME, 2 mM dAT copolymer, 33 μM dATP, 33 μM $^3$H-dTTP and enzyme.

In the present application, when DNA polymerase large (Klenow) fragment was used to fill in single-stranded DNA or cohesive ends that result from restriction enzyme cleavage, the following procedure was employed: restriction enzyme reactions were terminated by heating at 68°C for 10 minutes. To the same reaction mixture a final concentration of 50 μM of each deoxynucleotide triphosphate was added and for each microgram of DNA in the reaction mixture 10-100 units of DNA polymerase Klenow fragment was added. In other words an excess of DNA polymerase Klenow fragment was used to ensure that single-stranded ends were completely filled in.

6.1.6. <u>GEL PURIFICATION OF DNA FRAGMENTS</u>

After restriction enzyme or nuclease treatment, DNA fragments of varying sizes were separated by gel electrophoresis in either agarose or polyacrylamide slab gels at low voltage (approximately 2 volts/cm for agarose gels and 10 volts/cm for polyacrylamide gels), stained with ethidium bromide and visualized under ultraviolet light (Southern, 1979, Methods in Enzymology <u>68</u>: 152).

In order to recover particular DNA fragments from gels, the appropriate bands were sliced out of the gel and the DNA was electroeluted into dialysis tubing. The DNA was then isolated on DEAE-cellulose, appropriate fractions pooled, or ethanol precipitated, and resuspended in the appropriate buffer (Smith, 1980, Methods in Enzymology <u>65</u>: 371). DNA fragments were also separated on low melting point agarose (LMP agarose, Bethesda Research Laboratories, Inc.,

Gaithersburg, MD). The appropriate DNA bands were excised and melted at 65°C, diluted with 10 volumes of TEN (10mM Tris, pH 7.5, 1 mM EDTA, 100 mM NaCl) and then applied to a DEAE-cellulose column as described above.

## 6.1.7. DNA LIGATION

All ligations were accomplished using T4 DNA ligase (New England Biolabs, Inc., Beverly, MA). One unit of T4 DNA ligase is defined as the amount required to yield 50% ligation of *Hind*III fragments of bacteriophage lambda DNA in 30 minutes at 16°C in 20 µℓ of ligase buffer at a 5'-DNA termini concentration of 0.12µM (300 µg/mℓ).

DNA ligations were carried out in ligase buffer consisting of: 60 mM Tris-HCl (pH 7.8), 10 mM $MgCl_2$, 20 mM dithiothreitol(DTT), 1.0 mM ATP and a DNA concentration ranging from 15-50 µg/mℓ. Ligation reactions were incubated 4 to 24 hours at room temperature using approximately 300 units of T4 DNA ligase per 10 µℓ reaction volume.

## 6.2. PRODUCTION OF PRV STOCK INFECTION POOLS

The Becker strain of Pseudorabies (PRV) was used to infect PK15 cells (derived from porcine kidney, National Veterinary Services Laboratories, Ames, Iowa). Infected PK15 cells were propagated in Dulbecco's modified Eagle's medium supplemented with 10% fetal calf serum (GIBCO, Grand Island, NY). 10-20 100 mM tissue culture plates of PK15 cells were infected with 0.1 plaque forming units (pfu/cell) of PRV and the virus was allowed to absorb for 1 hour. Culture medium containing 5% fetal calf serum was added and the plates were incubated for 48 hours at 37°C. The plates were then scraped to dislodge the cells, pooled, sonicated and aliquots frozen at -70°C. The average titer for virus produced in this way was 5 X $10^8$pfu/mℓ.

### 6.3. EXTRACTION OF DNA FROM PRV NUCLEOCAPSIDS

PRV stock infection pools were produced as described *supra*. The following method for extraction of DNA from nucleocapsids was used:

(1) Cells from 10 infected roller bottles were resuspended in 10 mℓ LCM buffer (0.5% NP40, 30 mM Tris-HCl, pH 7.5, 3.6 mM $CaCl_2$, 5 mM $MgCl_2$, 125 mM KCl, 0.5 mM EDTA and 6 mM β-ME). Nucleocapsids were extracted by addition of 1 mℓ Freon (1,1,2-Trichloro-1,2,2,-trifluoroethane), shaken and subsequently centrifuged at low-speed for approximately 5 minutes. The upper aqueous phase was removed and re-extracted with 1 mℓ freon.

(2) The aqueous phase was split in two and layered onto 3 mℓ each of a 5% and 45% glycerol (in LCM buffer) step gradient in Beckman SW27 or SW28 centrifuge tubes. Nucleocapsids were pelleted by centrifugation at 25,000 RPM for 1 hour at 4°C.

(3) Each nucleocapsid pellet was resuspended by mild sonication after addition of 10 mℓ TNE (50 mM Tris, HCl pH 7.5, 100 mM NaCl, 10 mM EDTA). SDS (to give a final concentration of 0.5%) and an equal volume of phenol were added and the mixture was gently phenol extracted. After low-speed centrifugation, the upper aqueous phase was removed and re-extracted 2 times with phenol/chloroform/isoamyl alcohol (25:24:1) and 1 time with chloroform isoamyl/alcohol (24:1). Two volumes of ethanol were added to the aqueous phase and the DNA was precipitated at -20°C for at least 3 hours.

(4) The DNA was recovered by centrifugation and dissolved in a total of 12 mℓ TE-8 (10 mM Tris, pH 8.0, 1 mM EDTA). The final volume was measured and cesium chloride was added in ratio of 3.2 mℓ solution per 4 g cesium chloride. The DNA was then centrifuged to equilibrium at 45,000 RPM in a Beckman Type 50 rotor.

(5) Fractions were collected and subjected to electrophoresis on a 1% agarose gel. Fractions containing high molecular weight PRV DNA were pooled and dialyzed against 2-3 changes of TE-8 buffer. The DNA was then used directly or after ethanol precipitation and resuspension in an appropriate buffer. The yield of DNA after this procedure was approximately 200 - 400 µg.

## 6.4. IDENTIFICATION OF PRV-SPECIFIC GLYCOPROTEINS

To identify the glycoproteins present in the PRV virion, the virus structural proteins present in PRV-infected PK15 cells were examined using goat antibodies raised against Triton X-100 disrupted PRV virions (goat anti-PRV serum).

### 6.4.1. PREPARATION OF CELL EXTRACTS AND IMMUNOPRECIPITATION

100 mm plates of confluent PK15 cells were infected with 10 pfu/cell of PRV. Four hours post infection the culture medium was removed, and infected cell proteins were radioisotopically labeled by the addition of medium containing 50 µCi/mℓ of $^{35}$S-methionine or $^{3}$H-glucosamine (Amersham, England) and continued incubation at 37°C. The medium used for $^{35}$S-methionine labeling was methionine-free Dulbecco's E4 medium containing 2% fetal calf serum, while labeling with $^{3}$H-glucosamine was accomplished in Dulbecco's Modified Eagles Medium (E4 medium) containing 2% fetal calf serum. At 20 hours post infection, cells were harvested by removing the culture fluid, washing the cell monolayer with phosphate buffered saline (PBS) and scraping the cells into 5 mℓ of PBS. The cells were centrifuged (3000 X g for 5 minutes), resuspended in 5 mℓ of IP buffer (20 mM Tris, pH 8.0, 100 mM NaCl, 1 mM EDTA, 1% NP40, 1% deoxycholate (DOC), 0.1% SDS) and the lysate cleared by centrifugation at 40,000 RPM in a SW50.1 Beckman rotor for 30 minutes at 4°C.

For immunoprecipitations, approximately one-tenth of the total infected cell lysate (400 µℓ) was incubated for 1 hour on ice with 20 µℓ of either normal goat serum or goat anti-PRV serum. Immune complexes were collected by adsorption to protein A bearing Staphylococcus aureus (Pansorbin, Calbiochem-Behring Corp., La Jolla, CA) as described by S. W. Kessler (1975, J. Immunol. 115: 1617-1624). Following a 10 minute incubation on ice, the bacteria were pelleted by centrifugation in an Eppendorf centrifuge (Brinkman Instruments, Inc., Westbury, NY) and the pellets were washed consecutively in IP buffer, IP buffer containing 0.5 M LiCl, and finally IP buffer. The pellets were resuspended in SDS-PAGE sample buffer (70 mM Tris-HCl, pH 6.8, 11% glycerol, 3% SDS, 5% β-ME, 0.01% bromophenol blue) (Laemmli, 1970, Nature 227: 680-5). The extract was boiled for 2 minutes and centrifuged for 5 minutes in an Eppendorf centrifuge to remove insoluble material and loaded onto a 7-17% SDS-PAGE gradient gel. Immunoprecipitated proteins were visualized by fluorography following treatment of the gel with 1 M sodium salicylate. Films were exposed overnight at -70°C using Kodak XAR5 film and an intensifying screen.

A number of $^{35}S$-labeled proteins were found to be specifically precipitated by goat anti-PRV serum as compared to normal goat serum. To identify which of these proteins were glycosylated and hence probably PRV virion envelope proteins, the proteins in PRV-infected cells were labeled using $^{3}H$-glucosamine. Labeled proteins were identified by immunoprecipitation as described supra. The immunoprecipitated proteins were resolved by SDS-PAGE and visualized by fluorography. The goat anti-PRV sera specifically precipitated at least five glycoproteins. The molecular weights of these PRV-specific glycoproteins were estimated to be 110,000, 92,000, 74,000, 55,000 and 22,000 and were designated according to their electrophoretic mobility (i.e., g110, g92, g74, g55, g22).

## 6.5. PRODUCTION OF RABBIT ANTISERA AGAINST PRV GLYCOPROTEINS

PRV glycoproteins identified by $^3$H-glucosamine labeling were enriched by preparative affinity chromatography of infected cell extracts and separated by polyacrylamide gel electrophoresis. Regions corresponding to major groups of glycoproteins were excised from the gel and used to immunize rabbits.

A requirement of the cloning/expression strategy utilized in this invention was that antibodies raised against PRV glycoproteins recognize linear or denatured determinants. Therefore, antibodies were raised against PRV glycoproteins denatured with heat and SDS, as described in the following sections.

### 6.5.1. PREPARATION OF GOAT ANTI-PRV ANTIBODY AFFINITY COLUMN

Immunoglobulin from 5 mℓ of goat anti-PRV serum was purified using two ammonium sulphate precipitations followed by extensive dialysis against phosphate buffered saline (PBS, 40 mM $NaPO_4$, pH 7.0, 150 mM NaCl). Resultant immunoglobulins were covalently bound to 3 mℓ of Affigel 10 resin (Bio-Rad, Richmond, CA) at 4°C for 2 hours. The affigel was then incubated in 0.1 M ethanolamine at room temperature for 1 hour and subsequently washed with 4 column volumes of IP buffer followed by IP buffer containing 1 M NaCl and IP buffer containing 1.5 M potassium thiocyanate. The column was stored in IP buffer containing 0.02% azide at 4°C.

### 6.5.2. PURIFICATION OF PRV-SPECIFIC PROTEINS

Three roller bottles of PK15 cells were infected with 0.1 pfu/cell of PRV and were incubated for 36 hours at 37°C. The cells were then scraped into 20 mℓ of IP buffer, sonicated briefly and centrifuged in an SW41 Beckman rotor

(Beckman Instruments, Fullerton, CA) for 30 minutes at 40,000 rpm at 4°C.

The supernatant was incubated for 60 minutes at 4°C with the goat anti-PRV antibody affinity resin. The resin was then washed under stringent conditions with 4 resin volumes each of IP buffer, IP buffer containg 1M NaCl and then reequilibrated in IP buffer. The antibody affinity resin was poured into a glass column and proteins were eluted with IP buffer containing 1.5 M potassium thiocyanate and 1% NP40. The appropriate fractions containing PRV specific proteins were pooled and the proteins precipitated using 10% trichloracetic acid (TCA). Precipitates were spun in an Eppendorf centrifuge for 15 minutes at 4°C.

Protein samples selected by binding to the goat anti-PRV antibody column were lyophilized and resuspended in SDS-PAGE sample buffer and applied to a 10% SDS-PAGE gel. Electrophoresis was carried out for 3.5 hours at 40 mA. Side strips of the gel containing marker proteins were cut from the gel and stained using Coomassie blue dye. The stained side strips were used to locate PRV proteins in the remainder of the unstained gel. Regions of the gel containing the major groups of PRV glycoproteins (i.e., g110 and g92, g74, g55 and g22) were excised and macerated.

New Zealand white rabbits were inoculated subcutaneously with polyacrylamide gel slices containing approximately 30 µg of PRV protein emulsified in Freund's adjuvant. For each of the four groups of PRV specific glycoproteins, rabbits received five inoculations; only the initial inoculation was in complete Freund's adjuvant, whereas the remainder were in Freund's incomplete adjuvant. Serum from all rabbits was obtained prior to inoculation and subsequently five days after each boost by bleeding from the marginal ear vein. Rabbit sera were analyzed by immunoprecipitation and Western blot analysis. An example of such an analysis is described below for rabbit sera directed against PRV g74 protein.

### 6.5.3. IMMUNOPRECIPITATION AND WESTERN BLOT ANALYSIS OF PRV-SPECIFIC PROTEINS

The specificity and reactivity of the resulting rabbit sera immunized with PRV g74 protein was assessed by immunoprecipitation and Western blot analysis.

PRV infected PK15 cells were labeled with $^{35}$S-methionine and cell extracts (prepared as described supra) were reacted with either normal rabbit serum or rabbit serum raised against the PRV g74 protein. Immune complexes were collected by adsorbtion to Staphylococcus aureus and subjected to electrophoresis on a 7-17% SDS-PAGE gel. $^{35}$S-labeled proteins were detected by fluorography. The predominant PRV protein precipitated using anti-PRV g74 serum was a protein of 74,000 apparent molecular weight, although other PRV proteins were detected to a greatly reduced extent.

For Western blot analysis, uninfected and PRV infected PK15 cell lysates were subjected to electrophoresis on a 7-17% gradient SDS-PAGE gel and transferred to nitrocellulose according to the method of Towbin et al. (1979, Proc. Natl. Acad. Sci. USA 76:4350-4).

After transfer, the filter was incubated at 37°C for 2 hours in a solution of 5% BSA (bovine serum albumin) in binding buffer (0.1 M NaCl, 0.01 M $NaPO_4$, pH 7.5) to saturate all available protein binding sites (i.e. blocking buffer). The immobilized proteins were then reacted with anti-PRV g74 serum prepared in the blocking buffer described above. Reactions were performed in sealed plastic bags at 4°C for 12-18 hours on a rotary shaker. Following this incubation, the filter was washed three times for 20 minutes at room temperature in binding buffer. The second wash contained 0.5 M NaCl and 0.05% Nonidet P-40 and the third wash contained 0.05% Nonidet P-40. To visualize antigen-antibody interactions, the nitrocellulose was then incubated with $^{125}$I-labeled goat anti-rabbit IgG antibodies. The second antibody was iodinated using Bio-Rad enzymobeads (Richmond, CA) and $^{125}$I from New England Nuclear (Boston, MA) according

to the recommended conditions. The reaction with the iodinated antibody was carried out at 4°C for 4 hours in the blocking buffer. The nitrocellulose was washed as described *supra* followed by autoradiography at -70°C using Kodak XAR5 film with an intensifying screen. Antiserum raised against PRV g74 protein reacted with PRV specific proteins of 74,000, 76,000, 92,000, and 98,000 apparent molecular weight.

The results indicated that anti-PRV g74 serum was not monospecific and that the method used for isolation of PRV glycoproteins probably resulted in co-purification of PRV-specific proteins of similar molecular weights (*i.e.* 74,000 an 76,000). In addition the methods used in immunoprecipitation and Western blot analysis to determine the specificity of the sera were not analogous. Immunoprecipitation involved the reaction of antibodies with undenatured proteins, whereas the Western blot analysis involved the reaction of antibodies with denatured proteins. Furthermore, the method of raising antibodies involved inoculation with denatured PRV proteins and therefore the antisera produced may react preferentially in the Western blot analysis.

Antisera raised in rabbits against the combined g110 and g92 proteins reacted in immunoprecipitation and Western blot analysis predominantly with proteins of 110,000, 92,000 and 55,000 apparent molecular weight. Antisera against proteins contained in gel slices including the PRV g55 and g22 proteins have not been produced yet in sufficient titer in rabbits.

### 6.6. CLONING OF PRV DNA INTO AN EXPRESSION VECTOR

A library of *E. coli* plasmid bearing strains each expressing PRV DNA sequences was constructed by insertion of random segments of PRV DNA into an *E. coli* expression plasmid vector. Expression plasmids producing PRV antigens as

proteins fused to β-galactosidase that reacted with rabbit anti-g74 sera (described supra) were identified.

### 6.6.1. THE EXPRESSION VECTORS pJS413 AND pHK412

The expression vectors pJS413(6483 bp or about 6.5 kilobases, kb) and pHK412 (6485 bp or about 6.5 kb), are pBR322 derivatives which contain the $amp^r$ (β-lactamase) gene, a lac promoter (plac), lac and Cro ribosome binding sites ($SD^{lacZ}$ and $SD^{Cro}$ are represented in figures as $SD^z$ and $SD^{Cro}$, respectively), a chain initiation ATG derived from Cro followed by 65 nucleotides of the Cro gene, and a modified β-galactosidase gene (the i-z gene, hereinafter referred to as the z-gene). These expression vectors are described in detail in a U.S. Patent Application Serial No. 449,187 to J. Salstrom et al., filed December 13, 1982 which is incorporated herein by reference. Each plasmid has unique cloning sites which span the Cro-z junction: the pJS413 cloning sites are BglII, SmaI, and BamHI; the pHK412 cloning sites are BglII, HindIII, SmaI, and BamHI. In plasmid pJS413, the z-gene is not in phase with the Cro ATG, thus, the intact plasmid does not direct the production of β-galactosidase. However, when a DNA fragment is inserted in the correct orientation and reading frame into these cloning sites on the plasmid, the reading frame of the z-gene may be readjusted with respect to the Cro ATG and, provided that the inserted DNA sequence contains no termination signals (e.g., TGA, TAA, or TAG) that are in phase with the Cro ATG or z gene, a PRV/β-galactosidase fusion protein having β-galactosidase activity will be produced by host cells transformed by such recombinant plasmids. The β-galactosidase activity can be aserted by color reaction on indicator plates. Accordingly, insertion of a 3n+1 b.p. DNA fragment between the Cro ATG and z-gene of pJS413, results in a readjustment of the reading frames and production of a fusion protein in the host cell transformant, provided the

inserted sequences do not contain termination signals in the same reading frame. Of course, the inserted DNA sequence and/or the cloning sites of pJS413 and pHK412 may be altered in any fashion in order to readjust the reading frame across the Cro-z junction.

### 6.6.2. CLONING OF RANDOM PRV DNA FRAGMENTS

The plasmid expression library was constructed by first fragmenting PRV DNA isolated from purified nucleocapsids using limited digestion with pancreatic deoxyribonuclease I in the presence of $Mn^{+2}$ ions. Briefly, 10 μg of PRV DNA isolated from purified nucleocapsids as described in Sections 6.2 and 6.3 was digested with 2ng DNaseI for 10, 20 and 30 minutes at 25°C. The digests were pooled and run on a 5% polyacrylamide gel. A region corresponding to 0.5-0.6 kb DNA was excised, and the DNA fragments were eluted and ethanol precipitated.

The ends of the PRV DNA fragments were repaired using the Klenow fragment of DNA polymerase I and all four deoxynucleotides. $^{32}P$ labeled DNA linkers, (GGAGATCTCC), which contained the BglII recognition sequence were added to the PRV fragments using T4 DNA ligase. The complementary strands of DNA linker were synthesized by the solid phase method of Chow et al. (1981, Nucleic Acids Res. 9(12):2807-2827).

After addition of the BglII linkers, the DNA was digested with BglII and run on a 5% polyacrylamide gel. PRV DNA with the BglII linkers attached were located by autoradiography, excised from the gel, eluted and ethanol precipitated. Subsequently the PRV DNA was ligated into the BglII site of the expression vector pJS413 (Weis et al., 1983, Nature 302:72-74).

### 6.6.3. IDENTIFICATION OF TRANSFORMANTS

Plasmid DNA resulting from the ligation of the 500-600 bp random PRV DNA fragments to the BglII site of pJS413 were used to transform competent *E. coli* strain NF1829. The *E. coli* strain NF1829 is a K-12 MC1000 derivative carrying an F'-*lac* episome with the *lacI*$^q$ mutation for *lac* repressor overproduction. (The *lac* z-gene encoding β-galactosidase present on the F'-*lac* episome is inactivated by a Tn5 [transposon] insertion.) Thus, in strain NF1829, the *lac* promoter must be induced in order to obtain expression of a gene inserted into the pJS413 plasmid.

Primary transformants were detected on L-broth agar plates containing ampicillin. Transformants were replica-plated on lactose MacConkey (Mac-Lac) agar plates using nitrocellulose filters. Specific transcription from the pJS413 *lac* promoter was induced by the lactose in Mac-Lac plates, and the β-galactosidase activity of the hybrid gene products was assessed by colony color. Red color on Mac-Lac plates indicated β-galactosidase activity as a result of insertion of a PRV DNA fragment into pJS413 in the correct translational reading frame. Single colonies were picked for further analysis from the duplicate L-broth agar plates and screened for the bacterial synthesis of PRV antigens.

### 6.6.4. SCREENING FOR BACTERIAL SYNTHESIS OF PRV ANTIGENS USING WESTERN BLOT ANALYSIS

Transformants were picked and grown overnight (stationary phase) at 30°C in L-broth containing 100 μg/mℓ ampicillin. To obtain synthesis of fusion proteins, 50 μℓ of each overnight culture was used to inoculate 1 mℓ fresh L-broth containing 100 μg/mℓ ampicillin. After incubation at 38°C for 90 minutes, the cultures were induced by the addition of IPTG to a final concentration of 1 mM in the growth medium. After further incubation at 38°C for 1 hour,

bacteria were collected by centrifugation and lysed by boiling in 200 μl of SDS-PAGE sample buffer described supra.

In all, the proteins specified by 278 independent transformants were screened by Western blot analysis. Proteins were resolved by electrophoresis and analyzed by the Western blot procedure as described in Section 6.5.3. After immobilization of proteins on nitrocellulose paper, Cro-PRV-β-galactosidase proteins were reacted with rabbit anti-PRV g74 serum. As a control, duplicate blots of proteins were reacted with normal rabbit serum. Binding of rabbit antibodies was detected by reaction with $^{125}$I-labelled goat anti-rabbit IgG antibodies and autoradiography. Of the 278 colonies screened, 118 were found to synthesize a fusion protein of a size predicted for a PRV DNA insert of approximately 500-600 bp. Two of these fusion proteins were found to react specifically with anti-PRV g74 serum. The plasmids contained by these positive clones were designated pDPR7 and pDPR123. The remaining 160 colonies either synthesized a smaller protein of the size of β-galactosidase or synthesized no visible inducible protein.

### 6.6.5. MAPPING OF pDPR7 AND pDPR123 DNA ON THE PRV GENOME

The PRV DNA inserts contained by the two plasmids (pDPR7 and pDPR123) that specified fusion proteins reactive with anti-PRV g74 serum, were mapped on the PRV genome using the Southern blot hybridization method.

Restriction enzyme analysis of plasmid DNA from clones pDPR7 and pDPR123 indicated that the PRV DNA inserted in pJS413 was approximately 450 bp and 370 bp in size respectively. Each plasmid DNA was isolated and purified as described in Section 6.1.1. and labeled with $^{32}$P by nick translation (Rigby et al., 1977, J. Mol. Biol. 113:237-51).

Genomic PRV DNA was digested separately with restriction enzymes BamHI, KpnI, PstI and PvuII and duplicate sets of the fragments were resolved by electrophoresis through a 0.7%

agarose gel. DNA fragments were transferred to nitrocellulose by the method of Southern (1975, J. Mol. Biol. 98:503-517). The denatured, labeled plasmid DNA of pDPR7 and pDPR123 were then hybridized to the PRV genomic DNA restriction fragments immobilized on the nitrocellulose.

Figure 1 shows a restriction map of PRV DNA for the enzymes *BamHI* and *KpnI* which was derived by Ladin *et al.* (1982, Virology 116:544-561). The PRV DNA segments carried by both pDPR7 and pDPR123 hybridized to the *BamHI* 2 fragment of PRV DNA as indicated in Figure 1. The hybridization studies indicated that pDPR123 DNA hybridized to the *KpnI* J fragment of PRV DNA which is adjacent to, and slightly overlaps the *BamHI* 2 fragment. Plasmid pDPR7 mapped wholly within the *KpnI* A fragment which is adjacent to *KpnI* J. These data suggested that the plasmids contained PRV DNA inserts that were non-overlapping but adjacent on the PRV DNA genome. Further sub-cloning of the PRV DNA genome allowed localization of the area of hybridization of pDPR7 and pDPR123 to a single 3.6 kb *SphI*/*NcoI* fragment of PRV genomic DNA. The PRV DNA inserts contained in pDPR7 and pDPR123 were found to map within approximately 100 bp from each other. By producing antibodies to the Cro/PRV/β-galactosidase fusion proteins expressed by pDPR7 and pDPR123, the cloned PRV segments were determined to represent different regions of the same gene. Additionally, DNA sequencing indicated that the pDPR7 and pDPR123 inserts formed parts of a continuous open reading frame.

### 6.6.6. PRODUCTION AND ANALYSIS OF ANTISERA RAISED AGAINST Cro/PRV/β-GALACTOSIDASE FUSION PROTEINS EXPRESSED BY PLASMIDS pDPR7 AND pDPR123

Cro-PRV-β-galactosidase fusion proteins were purified from extracts of IPTG-induced cultures of pDPR7 and pDPR123 transformants by the aggregate procedure described in Section 5.5 and SDS-PAGE; electrophoresis was carried out for 3.5

hours at 40 mA as described in Section 6.5.2. Briefly, regions containing the fusion proteins were excised from the gel, and emulsified in Freund's adjuvant and injected into New Zealand white rabbits. Rabbits were inoculated subcutaneously with polyacrylamide gel slices containing approximately 50 μg of PRV fusion protein and the sera were analyzed by Western blot techniques and immunoprecipitation.

Uninfected or PRV infected PK15 cell proteins were subjected to electrophoresis on a 7-17% SDS-PAGE gel and subsequently transferred to nitrocellulose. The immobilized proteins were then reacted with serum raised against pDPR7 and pDPR123 fusion proteins and in duplicate with pre-immune serum. Binding of antibodies to proteins was detected by reaction with $^{125}$I-labeled goat anti-rabbit IgG antibodies and autoradiography. Rabbit anti-sera raised against either fusion protein were found to react with two PRV specific proteins of apparent molecular weights 74,000 and 92,000. Serum taken from rabbits prior to inoculation with these fusion proteins did not react with any PRV-specific proteins.

Goat anti-PRV serum was used as a control in this experiment and $^{125}$I-rabbit anti-goat IgG was used as the second antibody. This Western blot analysis revealed PRV specific proteins ranging in apparent molecular weights from 32,000 to 115,000 including proteins of 74,000 and 92,000 apparent molecular weight.

To immunoprecipitate PRV specific proteins using antiserum raised against either pDPR7 or pDPR123 fusion proteins, it was necessary to more fully denature the PRV proteins. $^{3}$H-glucosamine labeled PRV infected cell extracts were lysed in 1 mℓ of IP buffer, and centrifuged at 40,000 rpm in a Beckman SW41 rotor for 30 minutes at 4°C. The concentration of SDS and dithiothreitol in the supernatant was adjusted to 0.5% and 10 mM respectively. The extract was then boiled for 2 minutes and diluted five-fold with IP buffer. Antisera were then added as described in Section 6.5.3 and immunoprecipitates were analyzed by SDS-PAGE.

Rabbit antisera raised against pDPR7 and pDPR123 fusion proteins immunoprecipitated PRV specific glycoproteins with apparent molecular weights of 74,000 and 92,000 (g74 and g92). The 74,000 molecular weight glycoprotein band appeared sharp, whereas the 92,000 molecular weight glycoprotein band appeared diffuse in both immunoprecipitation and Western blot analysis. Antibodies raised against both pDPR7 and pDPR123 fusion proteins reacted identically in Western blot analysis and under modified immmunoprecipitation conditions. Apparently, antibodies raised against both the pDPR7 and pDPR123 fusion proteins (from denatured, purified gel slices) recognized the g74 and g92 glycoproteins only when the antigen was first denatured. Moreover, these antisera did not neutralize PRV infectivity *in vitro*.

The results of these experiments with antisera raised in rabbits against pDPR7 and pDPR123 fusion proteins indicated that the PRV DNA segments cloned in each plasmid represented coding sequences from the same gene.

### 6.6.7. INSERTION OF *Bam*HI 2 FRAGMENT OR PRV GENOMIC DNA INTO pBR322

As described in section 6.6.5, the PRV DNA inserts of pDPR7 and pDPR123 were mapped to the 20.3 kb *Bam*HI 2 fragment of PRV DNA. This 20.3 kb *Bam*HI 2 fragment was inserted in pBR322 (See Fig. 2). PRV genomic DNA (10 μg) was digested with *Bam*HI and the *Bam*HI 2 fragment was isolated following electrophoresis on a LMP agarose gel. 500 ng of the *Bam*HI 2 fragment of PRV genomic DNA and 100 ng of pBR322 digested with *Bam*HI (4.3 kb) were annealed and ligated and used to transform *E. coli* strain MC1000. Transformants were isolated and plasmid DNA was prepared by the mini-lysate method described in Section 6.1.1. Plasmid pPRV49 was determined to contain the desired fragment (See Fig. 2).

Further analysis of PRV DNA sequences represented in pDPR7 and pDPR123 more precisely located these sequences to an approximately 2.5 kb *Sph*I/*Bam*HI fragment contained within

pPRV49. This fragment was cloned in the SphI/BamHI sites of pBR322 and used to analyse the gene represented by pDPR7 and pDPR123 by subsequent DNA sequencing and S1 and Exonuclease VII analysis.

The 2.5 kb PRV SphI/BamHI DNA fragment from plasmid pPRV49 was isolated on a LMP agarose gel, annealed and ligated to the BamHI/SphI sites of pBR322 (See Fig. 2). The DNA was used to transform *E. coli* MC1000. Transformants were selected on LB agar plates containing 100 µg/mℓ ampicillin. Plasmid DNA prepared by the mini-lysate method was screened by restriction analysis for the desired plasmid and clone p7/123 was selected. Restriction analysis of p7/123 was performed and a restriction map was constructed as shown in Fig. 1.

The PRV glycoprotein encoded by DNA sequences represented in this plasmid is designated gIII (previously referred to in U.S. Pat. Ser. No. 598,073 as g74/g92). The nomenclature used to describe the structural glycoproteins of PRV has been revised according to the classification Hampl *et al.* (1984, J. Virol. 52: 583-590) to be consistent with the terminology used in this field of research.

### 6.6.8. LOCALIZATION AND CHARACTERIZATION OF THE PRV gIII GENE mRNA CODING SEQUENCE

To characterize the PRV gIII glycoprotein gene transcript, mRNA was isolated from PRV infected cells and hybridized to labeled probes using the Northern blot technique.

The size of the mRNA coding for this glycoprotein gene was determined by extracting total cytoplasmic RNA from cells infected with PRV at different times after infection, and a Northern blot analysis was carried out using nick-translated DNA from pDPR7 and pDPR123 as probes. RNA samples (approximately 5µg) of different sizes were electrophoresed through agarose gels containing formaldehyde and transferred to nitrocellulose filters (Maniatis *et al.*, Molecular

Cloning, 1982, Cold Spring Harbor Laboratory pp 202-203). Hybridization of both probes independently to a specific cytoplasmic RNA was detected; the approximate size of the RNA was 1600 bp. The translation of such an RNA could give rise to a polypeptide of maximal molecular weight 58,000.

Mapping the genomic DNA transcribed into the 1.6 kb mRNA sequence was accomplished by nuclease analysis, i.e., using single-strand specific nucleases S1 and Exonuclease VII to map the regions of DNA probes that hybridized to complementary mRNA sequences (Berk and Sharp, 1978, Proc. Natl. Acad. Sci. USA 75:1274), and by sequencing the DNA of the PRV gIII gene (Maxam and Gilbert, 1980, Methods in Enzymology 65:499).

The principle of the S1 mapping technique is to allow duplex formation between RNA and a radiolabeled single stranded DNA (ssDNA) probe (e.g., obtained from p7/123). If the RNA is a mature spliced mRNA, the introns will form a ssDNA loop. The enzyme nuclease S1 digests all ssDNA regions of the radiolabeled DNA that are not protected by duplex formation with RNA, whereas Exonuclease VII digests ssDNA only at the termini and therefore will not digest the ssDNA loops. Comparison of the size of DNA not susceptible to nucleases (by virtue of hybridization to RNA) enables the localization of the 5' end of the gene and the detection of spliced mRNA transcripts. By using appropriate 5' and 3' labeled DNA probes, this method also allows the 5' and 3' boundaries of the transcribed regions of genes to be precisely defined.

In the present invention, to ascertain more precisely the location of the 5' end of the PRV gene represented by pDPR7 and pDPR123, DNA from plasmid p7/123 was digested with restriction enzymes BamHI or SmaI and labeled with $^{32}P$ on either the 5' or 3' termini. Labeled DNA was digested with SphI and subsequently the appropriate DNA fragments were purified by gel electrophoresis for use in an S1 and Exonuclease VII analysis using total cytoplasmic RNA from PRV

infected cells. It should be noted that this procedure resulted in the generation of DNA fragments uniquely labeled at either the BamHI or SmaI terminus.

Using this technique, the 5' end of the gene was located approximately 1500 bp from the BamHI site in p7/123 as shown in Figure 1. As previously described, the size of the mRNA coding for the gene represented by pDPR7 and pDPR123 is approximately 1600 bp. The S1 mapping data indicated that the 3' end of the gene is located outside the SphI/BamHI fragment of p7/123 towards the BamHI site (See Fig. 1). Exonuclease VII digests performed on the DNA/RNA hybrids using the labeled DNA plasmid probes indicated no splicing of the 1.6 kb mRNA transcribed from this PRV gene.

Finally, the PRV glycoprotein gene of p7/123 was sequenced using the method of Maxam and Gilbert (Maxam and Gilbert, 1980, Methods in Enzymology 65:499). Both the coding and non-coding strands were sequenced. Figure 3 represents the DNA sequence obtained for the PRV gIII gene. The DNA sequence data established that there was an open reading frame of approximately 1350 base pairs located downstream from an ATG at position 199. Sequence data upstream from this ATG indicated the presence of the sequences CATTAAATC (residues 112 to 120) and TTTTTAAAA (residues 151 to 159) which may correspond to transcriptional regulatory CAT and TATA signals, respectively (See Fig. 3).

To complete the DNA sequence and fine structure analyses of the PRV gIII gene, the 3' end of the glycoprotein gene which is located on the KpnI J fragment (See Fig. 1) of PRV genomic DNA and overlaps the BamHI 2 fragment, was first cloned in the vector Polink 26.

To construct Polink 26 a synthetic 65 bp DNA fragment containing multiple restriction endonuclease cleavage sites was used to replace the 1850 bp fragment between the EcoRI and SalI site in pBR328 (Bethesda Research Laboratories, Gaithersburg, MD). An approximately 3100 bp fragment was isolated on LMP agarose and ligated to the synthetic 65 bp

DNA fragment. The construction of the Polink 26 vector is described in Fig. 4.

PRV genomic DNA and Polink 26 DNA were digested with KpnI and Polink 26 was subsequently treated with bacterial alkaline phosphatase (BAP) to minimize recircularization of plasmid DNA. The KpnI J fragment of PRV genomic DNA and the linearized Polink 26 DNA fragments were isolated from a 1% LMP agarose gel, ligated and used to transform *E. coli* MC1000. Transformants were isolated and plasmid DNA prepared by the mini-lysate method and screened by Southern blot analysis using nick-translated pDPR123 DNA as a probe. Plasmid pK64 which contained the correct KpnI J fragment of PRV genomic DNA was isolated (See Fig. 4).

DNA sequence and S1 analysis of this clone located the 3' end of the gene approximately 70 bp from the BamHI site in p7/123. Amino acid comparison between the PRV gIII gene and the HSV-1 gC showed limited but significant homology as indicated in Fig. 3.

Similarity alignments between the PRV and HSV-1 proteins were determined using the PROTHOM program of Conrad and Mount as described in Figure 4. The programs were used to locate regions of local sequence similarity in the coding regions of PRV DNA. The programs also substantiated proof that heterologous DNA gene sequences could be used for the localization and identification of yet unmapped herpesvirus genes. This analysis indicated an approximately 28.5% identity of the two sequences for the 467 amino acid sequence compared.

### 6.6.9. CLONING AND EXPRESSION OF THE PRV gIII GENE REPRESENTED BY p7/123

DNA sequence analysis of p7/123, pDPR7 and pDPR123 enabled the translational phasing of the PRV gIII gene represented in p7/123 to be established. Insertion of approximately 96% of the gIII gene coding sequences (NcoI to BamHI of plasmid p7/123) in the correct reading frame

between the cro initiation ATG of pHK412 and the z-gene (i.e., within the BglII, HindIII, SmaI or BamHI cloning sites in PHK412) allowed for the synthesis of a PRV/β-galactosidase fusion protein containing gIII amino acid sequences in cells transformed with this recombinant plasmid.

Plasmid pHK412 was digested with SmaI (resulting in a blunt ended cleaved vector) and BamHI (generating a BamHI cohesive end). The 6.5 kb fragment, containing the promoter, SD sequence, partial cro sequence, initiation ATG and the β-galactosidase gene was isolated by gel electrophoresis. Plasmid p7/123 was digested with NcoI and cohesive ends were filled in using the DNA polymerase Klenow fragment (resulting in a blunt end). After digestion with BamHI, and purification on LMP agarose, the 1400 bp DNA PRV fragment and the 6.5 kb pHK412 fragment were mixed in a 5:1 molar ratio, annealed and ligated, using T4 DNA ligase (Fig. 6). The resultant recombinant plasmids were used to transform E. coli strain NF1829. As previously described, the E. coli strain NF1829 is an overproducer of the lac repressor, hence the lac promoter was transcriptionally silent and must be induced by IPTG or a suitable analog to obtain expression of a gene inserted into the pHK412 plasmid.

### 6.6.10. IDENTIFICATION AND ANALYSIS OF TRANSFORMANTS THAT EXPRESS THE PRV gIII GENE

Transformants were screened by restriction analysis for insertion of the 1.4 kb NcoI to BamHI restriction enzyme fragment of p7/123 DNA after preparation of DNA by the mini-lysate method. A clone designated pNB412 was isolated that contained the correct PRV DNA insert.

To determine if pNB412 transformants were capable of synthesizing PRV/β-galactosidase fusion protein, total cell lysates induced with IPTG were analyzed by SDS-PAGE followed

by Coomassie brilliant blue staining. The bacteria synthesized a high molecular weight polypeptide upon induction of the lac promoter. The PRV/β-galactosidase fusion protein was of the size predicted for a fusion protein based upon data relating to the size of the inserted PRV DNA sequences and the z gene. A low level of fusion protein was produced (less than 1% of total cellular E. coli protein) by pNB412 transformants. In order to increase the level of fusion protein production, the ptac promoter was introduced into pNB412.

### 6.7. CONSTRUCTION OF ptac CLONES

Various tac (i.e., trp-lac) promoter/operator region clones were constructed. The hybrid promoter was constructed by combining the -35 bp region and the -10 bp region (the sequences which are the RNA polymerase binding site) of the E. coli trp and the lac promoters. (See European Patent Application 67,540 to DeBoer, filed May 18, 1982).

#### 6.7.1. CONSTRUCTION OF ptacNB

The expression vector p830 contains the strong tac (trp-lac) promoter. (Plasmid p830 also contains cro sequences ligated to the tac promoter and parvovirus sequences and is described in detail in U.S. Pat. Application, Ser. No. 564,567 to Halling et al., filed December 22, 1983.) This plasmid was digested with PstI and BglII and a 1300 bp fragment was isolated on LMP agarose. Plasmid pNB412 was also digested with PstI and BglII and a 6.8 kb fragment was isolated on LMP agarose. The 6.8 kb pNB412 fragment and the 1.3 kb p830 fragment were mixed in a 1:4 molar ratio, annealed and ligated using T4 DNA ligase (See Fig. 7). The ligation mixture was used to transform E. coli NF1829.

Transformants were selected for ampicillin resistance and plasmid DNA was prepared using the alkaline mini-lysate preparation described in Section 6.1.2. Plasmid DNA was screened by restriction endonuclease digestion with PstI and BglII to identify a plasmid containing the DNA sequences of the tac promoter. One clone, designated ptacNB was selected and examined for production of fusion protein by analysis of total *E. coli* proteins from cells harboring this expression plasmid as described *supra*. The fusion protein was produced at approximately 5-10% of total cellular *E. coli* protein as determined by Coomassie brilliant blue staining of SDS-PAGE gels containing total cell lysates of transformants induced with IPTG.

### 6.7.2. WESTERN BLOT ANALYSIS OF THE Cro/PRV gIII β-GALACTOSIDASE FUSION PROTEIN EXPRESSED BY ptacNB

The fusion protein produced by ptacNB specifically interacted with goat anti-PRV protein and rabbit serum directed against PRV g74 protein. The fusion protein expressed by ptacNB in the IPTG induced cells was separated by SDS-PAGE and transferred to nitrocellulose for Western blot analysis as described *supra*. The nitrocellulose was treated with goat anti-PRV serum or rabbit anti-PRV g74 serum followed by the appropriate $^{125}$I-labeled second antibody.

Autoradiographs of the protein blots clearly demonstrated that goat anti-PRV serum or rabbit anti-PRV g74 serum reacted strongly with the fusion protein expressed by ptacNB and not with other β-galactosidase fusion proteins containing unrelated virus protein sequences.

6.7.3. CLONING AND EXPRESSION OF THE PRV gIII GENE WITH ITS NATURAL TERMINATOR

Plasmid pNT7/123 was constructed to contain PRV gIII gene sequences and the natural terminator signal sequence TGA for the gene (see Fig. 8). Plasmid ptacNB was partially digested with NcoI and then digested to completion with XhoI. An approximately 4.8 kb NcoI/XhoI DNA fragment that contained an intact NcoI site at the ATG for the gIII gene and in which the β-galactosidase gene was deleted was isolated and purified in LMP agarose. Plasmid pK64 was digested with XhoI and NcoI and an approximately 1100 bp fragment which contained the natural TGA termination sequence for the gene was isolated in LMP agarose. The two fragments were ligated and the ligation mixture was used to transform E. coli NF1829.

Plasmid DNA was prepared using the mini-lysate method described supra. DNA was screened by restriction endonuclease digestion with HindIII and XhoI or NcoI and XhoI to identify a plasmid containing the correct insert. One clone, pNT7/123 with the correct insertion was selected. This clone was examined for production of PRV protein.

Plasmid pNT7/123 transformants induced with IPTG synthesized no detectable levels of Cro-PRV fusion protein. This plasmid contains the natural terminator sequence and a sequence which may be analogous to the transmembrane sequence found in other Herpes Simplex Virus glycoprotein genes. The fact that no detectable levels of inducible protein were observed may be due to the presence of these transmembrane sequences as is the case with the expression of HSV-1 gD protein in E. coli (See U.S.Pat. Appln. Atty. Dock. No. 2697-046, filed February 29, 1985 to Watson et al.) It should also be noted that some E. coli proteins would migrate in a protein gel in a position similar to that expected for the Cro-PRV hybrid protein and may therefore obscure the detection of the Cro-PRV protein.

### 6.7.4. CONSTRUCTION OF p7/123AM WHICH PRODUCED BOTH Cro/PRV gIII FUSION PROTEIN AND Cro/PRV gIII β-GALACTOSIDASE FUSION PROTEIN

Recombinant plasmids were constructed that can direct the production of both Cro/PRV gIII and Cro/PRV gIII β-galactosidase fusion proteins in appropriate host cells.

For example, as described below, if the recombinant plasmid ptacNB was cleaved at the junction of the PRV gene sequence and the Z-gene sequence and subsequently ligated in the presence of an appropriate DNA linker sequence containing an amber codon termination sequence, TAG, the resultant recombinant plasmids would contain an amber codon termination sequence in phase with the translational reading frames of both the PRV gene and Z-gene.

Plasmid ptacNB DNA was digested with BamHI resulting in cleavage of the plasmid at its unique restriction endonuclease site located at the junction of between the PRV gene and the Z-gene. The cleaved plasmid was re-ligated using T4 DNA ligase in the presence of a DNA linker characterized by an XbaI site and an amber sequence, TAG:

```
   ↓XbaI
GATCTAGATCTA
   ATCTAGATCTAG
            ↑
```

Each complementary strand of the DNA linker was synthesized by the solid phase method reported by Chow et al. 1981, Nucleic Acids Res. 9 (12): 2807-17.

Introduction of the linker sequence destroyed the BamHI site but introduced a BglII and XbaI site and two amber codons. As a result of the ligation, the two amber sequences were in phase with the translational reading frame of the PRV gIII gene and the Z-gene. The resulting plasmid was designated p7/123AM.

6.7.5. p7/123AM TRANSFORMANTS

Plasmid p7/123AM was used to transform E. coli NF1829. When induced with IPTG, the ampicillin resistant transformants synthesized an approximately 60,000 dalton Cro/PRV gIII fusion protein as analyzed using the mini-aggregate procedure.

The following mini-aggregate procedure was used to isolate host cell aggregates for screening analysis:

(1) Duplicate culture tubes containing 5 mℓ of fresh Luria broth containing 100 μg/mℓ ampicillin were inoculated with 200 μℓ of cells obtained from an overnight culture and grown for 90 minutes at 37°C. One of each duplicate was induced by the addition of 1mM IPTG (final concentration). The inocula were then grown with shaking at 37°C, and aliquots were taken at different time-points post-induction an described in (2);

(2) The cells contained in a 3 mℓ aliquot of the culture were pelleted by centrifugation in a microcentrifuge (12,000 x g) for 2 minutes. (N.B., the total volume of a microcentrifuge tube is 1.5 mℓ, therefore, a 1.5 mℓ aliquot was first pelleted and the supernatant was drawn off; another 1.5 mℓ aliquot was added to the same microcentrifuge tube and pelleted in the same tube.)

(3) The cell pellet was resuspended in 50 μℓ of 25% sucrose containing 50mM Tris-HCl, pH8, and the suspension was frozen by placing the tube in a dry ice/ethanol bath.

(4) The frozen suspension was thawed, 50 μℓ of 10mg/mℓ lysozyme in 0.25M Tris-HCl pH8 was added, and the suspension was incubated for 10 to 15 minutes on ice.

(5) After the 10 to 15 minute incubation 400 μℓ of TET buffer (100mM Tris-HCl, pH8, 50mM EDTA, 2% Triton X-100; Triton X-100 is a non-ionic detergent: polyoxyethylene (9-10) p-tert-octyl phenol) was added, the suspension was gently mixed and incubated for 5 minutes on ice. Then 500

μℓ of 2xRIPA (2xRIPA is 20mM Tris-HCl, pH7.4, 300mM NaCl, 2% sodium deoxycholate, 2% NP-40, 0.2% SDS) was added and the suspension was mixed gently and incubated for 5 minutes on ice.

(6) The cells in the suspension were then sonicated for 10 seconds using a microprobe, and the suspension was cleared by centrifugation in a Sorvall SS34 rotor for 10 minutes at 20,000 r.p.m. (47,800xg).

(7) The supernatant was decanted and the pellet, which contains the aggregated proteins, was resuspended in 100 μℓ distilled $H_2O$ to which 90 μℓ of 2x Sample Buffer (2x Sample Buffer is composed of 10% SDS, 40mM Tris-HCl, pH6.8, 2% β-ME, and 0.02 volumes saturated solution of bromophenol blue) and 10 μl β-ME was added and mixed well. The mixture was boiled for 5 minutes and 25 μℓ aliquots were applied to 10% SDS-polyacrylamide gels for electrophoresis.

The amber protein specified by the tac promoter/operator construct was produced at approximately 3% of total cellular *E. coli* protein as determined by Coomassie brilliant blue staining of SDG-PAGE gels.

### 6.7.6. INSERTION OF PRV DNA SEQUENCES INTO A HIGH COPY NUMBER PLASMID

To enhance expression levels of the PRV gene modifications of plasmid p7/123AM were made using derivatives of the high copy number plasmid pOP1Δ6. This construction replaced ptac with plac and also replace the wild type colE1 origin of replication with an "ori" mutation that causes plasmid over replication (Fig. 9).

Plasmid p7/123AM was digested with EcoRI and the fragment encoding ptac and cro/PRV/β-galactosidase coding sequences was isolated. Plasmid pHV13 ( See, U.S. Pat. Appln. Attorney Docket No. 2697-046 to Watson *et al*., filed February 27, 1985) which contains the ori mutation was digested with EcoRI and the DNA fragment containing the origin of replication and the $amp^r$ gene was isolated. The

two fragments were ligated using $T_4$ DNA ligase and the resultant mixture used to transform E. coli NF1829. Plasmid p7/123AM + ori was isolated from these transformed cells.

Plasmid p7/123AM + ori was then digested with HindIII and PstI and the DNA fragment encoding the PRV gIII β-galactosidase sequences and the origin of replication of the plasmid were isolated. The expression vector pHK412 was also digested with PstI and HindIII and the DNA fragment containing the plac promoter was isolated. The two fragments were ligated and used to transform E. coli NF1829. Ampicillin resistant colonies were selected, plasmid DNA isolated, and one plasmid pHK 7/123AM + ori was selected for the presence of both the PRV insert and the high copy number phenotype. The PRV protein expressed by this plasmid was produced at the rate of approximately 3% of total cellular E. coli protein.

## 6.8. CONSTRUCTION OF RECOMBINANT VACCINIA VIRUSES

Vaccinia virus expression vectors were used in the cloning and expression of the gIII gene sequences of PRV. Construction of recombinant vaccinia virus was achieved in two stages. First, a plasmid was constructed that contained the gIII gene of PRV located downstream from a vaccinia promoter (the early 7.5 K promoter). Second, this chimeric gene containing the vaccinia promoter and the PRV gIII coding sequence was inserted into the genome of vaccinia virus by homologous recombination *in vivo*.

### 6.8.1. CONSTRUCTION OF AN E. COLI PLASMID CONTAINING THE VACCINIA/PRV gIII RECOMBINANT GENE

Five micrograms of pNT7/123 plasmid DNA was digested to completion with NcoI and the resulting fragments were resolved on a low-melting point agarose gel (see Fig. 10). A 2.5 kb PRV-specific fragment was isolated. This fragment

contains the entire coding sequence of the PRV gIII gene and approximately 900 bp of the 3' non-coding sequences. The initiation codon of the PRV gIII gene is believed to be encoded by the ATG sequence located in the 5' single-stranded region generated by the NcoI digested. This region was made double stranded by the action of Klenow enzyme in the presence of excess deoxyribonucleotide triphosphates.

The resulting blunt-ended fragment, was ligated to 0.5 ug of pGS20 (MacKett et al., 1984, J. Virol. 49: 857-864) DNA which was previously linearized with BamHI and made blunt-ended with the Klenow enzyme. Ligation was allowed to proceed for 16 hours at 12°C. The ligation mixture was then used to transform E. coli strain MC1000. Plasmid DNA from individual transformants was analyzed for the correct orientation of the PRV insert and the regeneration of the BamHI sites at the ligation junctions. The confirmed structure of the desired plasmid pGSPR1 is shown in Fig. 10.

### 6.8.2. CONSTRUCTION OF A RECOMBINANT VACCINIA VIRUS EXPRESSING THE PRV gIII GENE OF PRV

Recombinant vaccinia viruses were generated by transfection of the pGSPRI plasmid into cells infected with vaccinia virus (See Fig. 10)

Insertion of the vaccinia-PRV chimeric gene into vaccinia virus genome was achieved by in vivo recombination. This was made possible by the fact that the chimeric genes in plasmid pGSPR1 was flanked by vaccinia sequences coding for the thymidine kinase (TK) gene. Introduction of this plasmid into vaccinia-infected cells allows recombination to occur between the TK sequence on the plasmid and the homologous sequence in the viral genome. Insertion of the chimeric gene will only occurr as the result of double recombinations in both of the flanking sequences. Such recombinants have the chimeric gene inserted in the vaccinia

TK gene and, as a result, were phenotypically $TK^-$. These $TK^-$ recombinants can therefore be selected for growth on $TK^-$ host cells in medium supplemented with 5-bromo-deoxyuridine (BUdR). The general principle of this procedure has been described by MacKett et al. (1984, J. Virol. 49: 857-864).

A 100 mm dish of 80% confluent African Green Monkey kidney cells (strain B-SC-40) (supplied by Dr. R Condit, State University of New York, Buffalo, NY) were infected with vaccinia virus (strain WR) at a low multiplicity (0.05 plaque-forming units per cell). After 4 hours of incubation at 37°C, infected cells were overlaid with a calcium phosphate precipitate of plasmid DNA pGSPR1. The precipitates were prepared by adding 0.5 mℓ of 2X DNA-$CaCl_2$ solution dropwise to 0.5 mℓ of 2X HeBS (Hepes Buffered Saline) solution. [2X DNA-$CaCl_2$ solution contains 20 ug of plasmid DNA in 0.5 mℓ of 0.24 M $CaCl_2$. 2X HeBS contains (per mℓ) 16 mg of NaCl, 0.74 mg of KCl, 0.25 mg of $Na_2HPO_4 2H_2O$, 2 mg of dextrose, and 10 mg of HEPES at pH 7.08.] Precipitation was allowed to proceed at room temperature for 15-30 minutes.

Four hours following the overlay of precipitates, the cells were washed once with 1X HeBS, incubated at 37°C for 3 minutes in the presence of 2 mℓ 15% glycerol in 1X HeBS which was aspirated off the cells. The cell were washed once with 1X HeBS and then incubated with 10 mℓ of growth medium containing DME (Dulbecco's Modified Eagles Medium, GIBCO) and 10% FCS (fetal calf serum, GIBCO). Two days later, infected cells were harvested and virus stock were prepared from these cells by 2 cycles of freezing and thawing, followed by sonication.

Recombinants were selected by plating 0.1 mℓ of a $10^{-3}$ dilution of vaccinia viral stocks on 60 mm dishes of confluent human $TK^-$ 143 cells (supplied by Dr. R Condit) overlaid with 1% agar + DME + 5% FCS. Individual plaques were picked and the virus suspensions used to infect human $TK^-$ 143 cells in 16 mm diameter wells under selective

conditions. Recombinants were detected by DNA plaque hybridization. Infected cells were collected on nitrocellulose filters and the presence of PRV specific sequence in these samples was assayed by dot-blot hybridization as described by MacKett et al. (1982, Proc. Natl. Acad. Sci., U.S.A. 79:7415-7419). After denaturation, neutralization and fixation of the DNA, the DNA was hybridized with $^{32}P$-labeled plasmid pNT7/123 DNA as probe. Recombinants that gave positive hybridization to this probe were further plaque-purified twice on TK-143 cells under selective conditions and again on B-SC-40 cells under non-selective conditions. After final confirmation by another "dot-blot" hybridization, viral stocks were prepared from the three times purified plaques on B-SC-40 cells and used for characterization of recombinant virus, VPRI as described below.

### 6.8.3. ANALYSIS OF RECOMBINANT VPRI VIRUS

High titer stocks of recombinant VPRI were grown on B-SC-40 cells (2 x $10^9$ pfu/mℓ). These virus stocks were used to infect PK15 cells at 10pfu/cell. The infected cells were overlayed with 5mℓ DME, 5% FCS and 100μC/mℓ $^3H$ - glucosamine (Amersham). At 7-8 hours post-infection, the cells were harvested and immunoprecipitations using cell lysates were carried out as described previously (See Sec 6.4.1.). The results indicated that goat anti-PRV serum, serum raised against the Cro/PRV gIII protein expressed by p7/123AM and a monoclonal antibody against the PRV gIII gene all immunoprecipated a PRV specific glycoprotein of 92,000 apparent molecular weight. Pre-immune or control sera did not immunoprecipitate any glycoproteins from VPRI infected all extracts. As a control PK15 cells were infected with wild type vaccinia virus and labeled with $^3H$-glucosamine as described supra. Using wild type vaccinia infected cell extracts labeled with $^3H$-glucosamine no immunoprecipitation

of the PRV specific glycoproteins was observed using the same set of antibodies.

The level of expression of the PRV gIII protein in cells infected with the recombinant virus VPRI is as yet unknown. Immunological experiments are now in progress to assess the ability of the recombinant virus VPRI to induce an immune response in mice and pigs capable of protecting them from a challenge with PRV.

### 6.9. USE OF HETEROLOGOUS DNA PROBES TO LOCALIZE GLYCOPROTEIN GENES WITHIN THE PRV GENOME

Since Herpes simplex virus (HSV) and pseudorabies virus (PRV) have similar morphological and biochemical characteristics, and genomic DNA from HSV and PRV share homologous sequences (an estimated 8% of the sequences of the genomes of HSV and PRV; Ludwig et al., 1972, Virology 49: 95-101), it seemed likely that glycoprotein genes of these two viruses may be evolutionarily conserved and exhibit some sequence identity. Therefore, HSV DNA fragments containing glycoprotein genes were used to localize glycoprotein genes within the PRV genome. PRV genomic DNA was digested with a number of restriction enzymes and subjected to electrophoresis in a 0.7% agarose gel. DNA fragments were subsequently transferred to nitrocellulose filters according to the method of Southern.

Cloned, nick-translated DNA sequences corresponding to HSV-1 gB (DeLuca et al., 1982, Virology 122: 411-423), HSV-1 gC (Frink, 1981, J. Virology 39: 559-572) and HSV-1 gD (U.S. Pat. Application, Ser. No. 510,551 to Watson et al., filed July 6, 1983) were hybridized to restriction fragments of PRV DNA fixed to nitrocellulose filters. The conditions for hybridization were as follows: 50% formamide, 5X SSC (10X SSC = 1.5 M NaCl, 0.15 M sodium citrate), 10mM PIPES (1,4-Piperazinediethane Sulfonic Acid) pH6.5, 5X Denhardt's (1x Denhardt's = 0.2% BSA, 0.02% Ficoll, 0.2%

polyvinylpyrolidine) solution, 200 μg/mℓ calf thymus DNA and $10^6$ cpm/mℓ of the $^{32}$P-labeled probe at 37°C for 20 hours. Under these conditions, only specific hybridization of HSV-1 gB DNA to PRV genomic DNA was observed. The region of hybridization was localized to a 9.5 kb SalI 3 fragment of PRV genomic DNA (See Fig. 11). 200 ng of the SalI 3 fragment and 100 ng of pBR328 digested with SalI were annealed and ligated and used to transform E. coli MC1000 as described in Fig. 11. A clone, designated pPRS3 was isolated that contains the PRV SalI 3 DNA fragment.

Further Southern blot analysis localized the region of hybridization of HSV-1 gB DNA to PRV DNA to a 3.4 kb SphI/SalI fragment of plasmid pPRS3. Plasmid pPRS3 was partially digested with SphI and completely digested with SalI and the 3.4 kb fragment was isolated after electrophoresis through LMP agarose. 100 ng of the 3.4 kb fragment and 100 ng of pBR322 digested with SalI and SphI were annealed and ligated and used to transform E. coli NF 1829 (See Fig. 11). Clone pssgB was isolated and a restriction map of this plasmid was constructed (See Fig. 12). The SphI site which should have been regenerated by ligation of the two SphI termini was lost during the cloning procedure.

The size of the mRNA coding for this PRV glycoprotein gene was determined by Northern blot analysis as described in Section 6.6.8. Total cytoplasmic RNA was extracted from cells infected with PRV at different times after infection, and a Northern blot analysis was carried out using the 2.7 kb SphI/SalI DNA fragment from pPRS3 purified from LMP agarose and subsequently nick-translated. Hybridization of the nick-translated probe to an RNA species of approximately 3000 bases was detected. The size of the mRNA encoding HSV-1 gB is estimated to be approximately 3000 bases.

To determine the direction of transcription and the location of the 5' termini for this putative glycoprotein

gene, S1 analysis was carried out as described in Section 6.6.8.

Plasmid DNA from clone pssgB was digested with SalI or BstEII and labeled on either the 5' or 3' strand. The DNA was then digested with BamHI and subjected to electrophoresis in a 1% LMP agarose gel. DNA fragments of 3.4 kb (BamHI/SalI) and 1.7 kb (BamHI/BstEII) were purified from LMP agarose. S1 analysis indicated that the 5' end of the gene was located approximately 800 bp from the BstEII site (1400 on the restriction map) towards the BamHI site in pssgB. The 3' end of the gene is most likely located external to the SalI 3 fragment.

Another herpesvirus, bovine Herpes Virus I (BHV I) shares a homologous region of its genome with the HSV-1 gB gene and the PRV gene represented in plasmid pssgB. The homology between the BHV I and PRV homolog of the HSV-1 gB gene is greater than that for either BHV I or PRV with the HSV-1 gB gene. The BHV-1 homolog of the HSV-1 gB gene has been determined to encode for a family of related glycoproteins of 135,000, 75,000 and 54,000 apparent molecular weights, using monospecific serum raised against authentic glycoproteins of BHV-1. (The monospecific sera was supplied by L. Babiuk, Saskatchewan, Canada).

Indirect evidence as to the PRV specific glycoproteins encoded by the HSV-1 gB homolog of PRV was obtained using this monospecific sera to carry out immunoprecipitations using PRV infected cell lysates labeled with $^3$H-glucosamine. This monospecific sera raised against authentic BHV-1 glycoproteins immunoprecipitated PRV specific glycoproteins of 110,000, 68,000 and 55,000 apparent molecular weights.

Direct evidence indicating which PRV glycoproteins are encoded by the PRV homolog of the HSV-1 gB gene was obtained by using sera raised in rabbits against Cro/PRV/β-galactosidase fusion protein expressed by the plasmid pXXgB (See section 6.9.3). Sera raised against this fusion protein immunoprecipitated PRV specific glycoproteins of 110,000,

68,000 and 55,000 apparent molecular weights from PRV infected cell extracts labeled with $^3H$-glucosamine. The HSV-1 gB homolog of PRV has thus been identified as the PRV gII glycoprotein gene complex (Hampl et al.; Lukacs et al., 1985, J. Virol. 53:166-173).

The PRV gII gene was sequenced using the method of Maxam and Gilbert. The entire PRV gII gene was cloned into pBamHI-1 (i.e., the BamHI 1 fragment, as shown in Fig. 1). Figure 13 represents most of the DNA sequences of the PRV gII gene.

Similarity alignments between PRV and HSV-1 gB were also determined using the PROTHOM program and the PRTALN program as described supra and in Fig. 14. The programs were used to locate regions of local sequence similarity in the coding regions of PRV DNA and HSV-1 gB DNA and to confirm the specific DNA sequence of the PRV gII gene. This program indicated an approximately 49.2% identity for the 900 amino acids sequence compared.

Cloning and expression of the PRV gII gene is described below.

### 6.9.1. CLONING OF THE PRV gII GENE REPRESENTED BY PLASMID pssgB

A 1300 bp XhoI from plasmid pssgB fragment was inserted in the expression vector pHK412. 500 ng of pssgB DNA was digested with the restriction enzyme XhoI and subsequently incubated with all four dNTP's and the Klenow fragment of DNA polymerase I. The DNA was then subjected to electrophoresis on a 1% LMP agarose gel, and the XhoI fragment of 1300 bp was excised from the gel. One µg of the expression vector pHK412 was digested with the restriction enzyme SmaI and subsequently incubated with 0.6 units of calf intestinal alkaline phosphatase (CIAP, Boehringer-Mannheim Corp., Indianapolis, IN) and 0.5mM $ZnCl_2$ at 37°C for 30 minutes to dephosphorylate the DNA at the termini. The DNA was then incubated at 65°C for 10 minutes and subjected to electrophoresis on a 1% LMP agarose gel.

Linear DNA of approximately 6500 bp was excised from the gel.

Approximately 50 ng of the XhoI 1300 bp fragment of pssgB and 100 ng of the 6500 bp fragment of pHK412 were ligated and transformed into E. coli NF1829. A clone, designated pssgB-1 was isolated which contained the 1300 bp XhoI DNA fragment of pssgB.

Subsequent immunological analysis of the protein expressed by this clone and DNA sequence analysis (See Fig. 13) indicated that the PRV DNA in plasmid pssgB-1 was not in the correct reading frame to express the PRV gII gene. DNA sequence analysis had indicated the presence of two open reading frames in the 1300 bp XhoI restriction enzyme fragment represented in the plasmid pssgB in the pssgB gene insert.

### 6.9.2. CLONING AND EXPRESSION OF PRV gII GENE SEQUENCES

To express the PRV DNA sequences in the correct translational reading frame as determined from DNA sequence analysis, several new plasmid constructions were made as described below.

pxxgB

The following procedure was used to place the 5' end of a portion of the PRV gII gene in the correct phase for expression of the PRV gII gene sequence. A 766 bp DNA fragment was isolated from pssgB by digestion of 20 µg of the plasmid with XmaI (Fig. 15). The expression vector pJS413 (5 µg) was digested with XmaI and treated with 0.6 units of CIAP. The digested DNA were subjected to electrophoresis on a 1% LMP agarose gel and the 6500 bp XmaI linear DNA fragment from pJS413 and the 766 bp XmaI fragment from pssgB excised from the gel. Approximately 100 ng of each fragment were ligated and transformed into E. coli NF1829.

Recombinant plasmids were purified using the alkaline mini-preparation mentio (Sec. 6.7.5.) and analyzed by restriction endonuclease mapping for the correct construction. Plasmids in which the PRV sequences were in the correct orientation and in-frame with the Cro ATG of pJS413 should yield an approximately 766 bp DNA fragment upon digestion with restriction enzymes BamHI and XhoI.

A plasmid designated pJSXma contained a 766 bp PRV DNA insert in the proper orientation to establish the correct reading frame with the Cro ATG. The PRV sequences were, however, not in frame with the lacZ gene. To phase the PRV gII gene sequence with lacZ, the BglII/BamHI fragment from pJSXma which encodes PRV gII gene sequences was inserted in plasmid pHK414.

The expression plasmid pHK414 (5 μg) was digested with BamHI and BglII and treated with 0.6 units of CIAP. Plasmid pJSXma (20 μg) was also digested with BglII and BamHI. The DNA fragments were electrophoresed on a 1% LMP agarose gel and the 6.8 kb linear DNA fragment from pHK414 and the 766 bp PRV DNA fragments from plasmid pJSXma were excised from the gel and subsequently ligated using $T_4$ DNA ligase (approximately 100 ng of each fragment). Recombinant plasmids resulting from this ligation were used to transform E. coli NF1829.

Ampicillin resistant clones were selected and a plasmid pXXgB which contained the appropriate BglII/BamHI PRV DNA insert was isolated. Expression plasmid pXXgB was examined for the expression of a Cro/PRV gII β-galactosidase fusion protein as described supra. Total E. coli cell extracts either induced using IPTG or uninduced were subjected to electrophoresis on SDS-PAGE gels. Coomassie blue staining of these gels indicated that a Cro/PRV gII β-galactosidase fusion protein of approximately 145,000 apparent molecular weight was produced upon induction with IPTG as compared to the uninduced control. The yield of Cro/PRV gII β-

galactosidase fusion protein was approximately 5% of total cell protein.

pPR10

Plasmid pssgB was digested with XhoI and then treated with the Klenow fragment of DNA polymerase I in the presence of the four dNTPs to fill-in the cohesive ends (Fig. 16). A 1183 bp XhoI fragment from pssgB was purified on a 1% LMP agarose gel. Plasmid pHK412 was digested with BglII and SmaI and treated with the Klenow fragment of DNA polymerase I in the presence of the four dNTPs. The 6.5kb linear DNA fragment of pHK412 was purified on a 1% LMP agarose gel, excised and subsequently ligated to the 1183 XhoI fragment from pssgB using $T_4$ DNA ligase. These recombinant plasmids were used to transform E. coli NF1829. Plasmid DNA from ampicillin resistant colonies was screened by restriction endonuclease digestion with BglII and BamHI (the BglII site is reconstituted when the blunt ended BglII site is ligated to the XhoI blunt end) for the correct insert. A plasmid, pXh412 was isolated that contained an 1183 bp PRV DNA insert.

To construct a plasmid expressing a Cro/PRV gII fusion protein, the 1183 bp BglII/BamHI DNA fragment of pXh412 was inserted into the plasmid p470/1 (Fig. 16). Plasmid p470/1 is organized as follows: pTAC/Olac/Cro/TIC/TTS/TI Term/ori/amp$^r$. TIC is an artificial translation initiation complex located 3' to the (Cro)ATG and consists of the sequence 5'-ATGGCTACTGTTAT-3'. TTS is the triple translation terminator sequence. TI Term is a transcription terminator from the E. coli rrnB operon.

Plasmid pXh412 was digested with BglII and BamHI and a 1183 bp DNA fragment encoding PRV gII DNA sequences was purified on a 1% LMP agarose gel. p470/1 was also digested with BamHI and BglII, treated with CIAP and the linear DNA fragment was purified on a 1% LMP agarose gel. The two DNA fragments were excised from the gel and subsequently ligated

using $T_4$ DNA ligase. Recombinant plasmids were used to transform E. coli NF1829. Ampicillin resistant transformants were screened for plasmids containing the 1183 bp BglII/BamHI PRV gII DNA fragment. Clones containing this PRV DNA insert were screened for the production of a Cro/PRV gII fusion protein as described, supra. A plasmid designated pPR10 was selected that expressed a Cro/PRV gII fusion protein of approximately 46,000 daltons upon induction with IPTG. Plasmid pPR10 produced about 5 mg/liter of this Cro/PRV gII fusion protein.

pPR14 and pPR15

Plasmid pssgB was partially digested with XhoI and the cohesive ends were filled-in using the Klenow fragment of DNA polymerase I and the four dNTPs (FIG. 17). An 1870 XhoI partial DNA fragment was excised from a 1% LMP agarose gel and inserted into the SmaI site of the expression vector pJS413. After ligation of the two DNA fragments using $T_4$ DNA ligase, recombinant plasmids were used to transform E. coli NF1829. Ampicillin resistant colonies were selected and screened for plasmid DNA containing the approximately 1870 bp PRV gII DNA insert. A clone, designated pxxXhJS was found to contain the PRV XhoI partial fragment of pssgB in the correct orientation.

Plasmid p470/1 was digested with BglII and BamHI, treated with CIAP, and the linear DNA fragment was purified on a 1% LMP agarose gel. Plasmid pxxXhJS was digested with BglII and BamHI and an 1870 bp DNA fragment was purified on a 1% LMP agarose gel. The DNA fragments were excised from the gel, ligated using $T_4$ DNA ligase, and the ligation mixture was used to transform E. coli NF1829. Ampicillin resistant transformants were screened for plasmids containing the 1870 bp PRV DNA insert. A clone, designated pPR14, was found to contain the appropriate DNA insert.

Plasmid pPR14 is not in phase with the TIC DNA sequence but is in phase with TTS/TI TERM and therefore does not

express a Cro/PRV gII fusion protein. To adjust the reading frame of the PRV DNA insert at the TIC DNA sequence of this plasmid, pPR14 was digested with BglII and the cohesive ends were filled-in using the Klenow fragment of DNA polymerase I. The plasmid was then re-ligated using $T_4$ DNA ligase and resulting plasmids used to transform E. Coli NF1829. Ampicillin resistant colonies were screened for plasmids lacking the BglII site, and a clone designated pPR15 was isolated. This clone was analyzed for the expression of a Cro/PRV gII fusion protein as described, supra. Plasmid pPR15 was found to express a 70,000 dalton Cro/PRV gII fusion protein by analysis of mini-aggregate protein extracts on SDS-PAGE.

### 6.9.3. WESTERN BLOT ANALYSIS OF Cro/PRV gII FUSION PROTEINS

Aggregate fusion proteins expressed by pxxgB, pPR10, and pPR15 were isolated from cultures induced with IPTG and applied to a denaturing 7.5% SDS-PAGE gel. The proteins were transferred to nitrocellulose paper for Western blot analysis as described supra in Sec. 6.6.4. The immobilized fusion proteins were reacted with goat anti-PRV sera or a mixture of two PRV gII monoclonal antibodies (designated M2 and M3) directed against PRV glycoproteins (See, Hampl et al.). Antigen/antibody interactions were visualized using an appropriate rabbit antisera and $^{125}$I-labeled protein A. The nitrocellulose was then exposed to XAR5 film.

Autoradiographs clearly demonstrated that goat anti-PRV serum reacted strongly with fusion proteins expressed by plasmids pxxgB, pPR10, and pPR15 and not with another fusion protein containing unrelated virus protein sequences. Autoradiographs of the Western blot also demonstrated that the mixture of two PRV gII monoclonals reacted with the pPR15 fusion protein. The gII monoclonals did not react with the pPR10 fusion protein. The gII monoclonals reacted very weakly or not at all with the pxxgB fusion protein.

# 7. VACCINATION

## 7.1. PROTECTION OF MICE AGAINST LETHAL PRV INFECTION USING FUSION PROTEIN EXPRESSED BY ptacNB IN A VACCINE FORMULATION

Four groups of Webster Swiss Balb/c mice each were injected intramuscularly (IM) with the following preparations: Group 1 received 250 μg of undenatured fusion protein expressed by ptacNB, mixed with formaldehyde and aluminum hydroxide (Reheis Chemical Company, Berkeley Height, NJ); Group 2 received 250 μg of undenatured fusion protein expressed by ptacNB (in phosphate buffered saline); Group 3 received 250 μg of urea-solubilized fusion protein expressed by ptacNB (isolated by the denaturing aggregate purification method described in Section 5.6; fusion protein expressed by ptacNB isolated by this method precipitated when the urea was removed by dialysis), mixed with formaldehyde and aluminum hydroxide; and group 4 received 250 μg of urea-solubilized ptacNB fusion protein in phosphate buffered saline. At day 21 all mice were injected IM with a further dose of 50 μg of fusion protein expressed by ptacNB prepared as above.

The mice were bled retro-orbitally at day 20 and day 28. Fourteen days after the second injection the mice were challenged with approximately 5 times $LD_{50}$ of PRV. The PRV was suspended in cell culture medium and injected IV. Survival of the challenged mice indicated protection. Results are shown in Table I. The mouse protection data indicate that immunization with fusion protein produced by the clone ptacNB was capable of protecting mice from a lethal challenge with PRV.

TABLE I

PRV-M-1 CHALLENGE OF VACCINATED MICE

Number of Mice Surviving After Challenge with PRV[1]

| | Day 0 | Day 4 | Day 8 | Mean time to death (hrs) | Protective efficacy on day 8 |
|---|---|---|---|---|---|
| Group 1 | 5/5 | 5/5 | 3/4 | 186 | 60% |
| Group 2 | 5/5 | 5/5 | 4/5 | 180 | 80% |
| Group 3 | 5/5 | 5/5 | 3/5 | 156 | 60% |
| Group 4 | 5/5 | 5/5 | 3/5 | 120 | 60% |
| Control[1] | 5/5 | 0/5 | 0/5 | 74 | 0% |
| Control[2] | 5/5 | 0/5 | 0/5 | 70 | 0% |

No deaths occurred between day 8 and day 12. Mice were killed and bled for serum on day 12.

[1] Challenge with $10^{4.5}$ PFU Becker Strain PRV.

[2] Challenge with $10^{4.0}$ PFU Becker Strain PRV.

## 7.2. INOCULATION OF PIGS USING FUSION PROTEIN EXPRESSED BY ptacNB IN A VACCINE FORMULATION

Six groups of 3 pigs each were inoculated with the preparations described in Table II for PRV-P-1 formulations. Pigs were injected with 2 mℓ of the vaccine formulation intramuscularly (IM) and observed for local reactions and pyrogenicity. All pigs were bled at day 0, boosted and bled at 21 days, and bled again at days 28 and 42. Four pigs were held as uninoculated controls.

TABLE II

PRV-P-1 FORMULATIONS

| Group | 1.5 mg/mℓ Aggregate | 1.5 mg/mℓ Soluble | 37% Formaldehyde | 3% $Al(OH)_3$ | PBS |
|---|---|---|---|---|---|
| A | | 3.32 mℓ | | | 16.68 mℓ |
| B | | 3.32 mℓ | 100 μℓ | | 16.58 mℓ |
| C | | 3.32 mℓ | 100 μℓ | 234 μℓ | 16.35 mℓ |
| D | 3.32 mℓ | | | | 16.68 mℓ |
| E | 3.32 mℓ | | 100 μℓ | | 16.58 mℓ |
| F | 3.32 mℓ | | 100 μℓ | 234 μℓ | 16.35 mℓ |
| G | CONTROLS | | | | |

None of the pigs showed any local reaction to injection or exhibited the effects of pyrogenicity.

None of the pigs inoculated with the fusion protein expressed by ptacNB seroconverted, and none of the pigs were protected against a challenge with PRV. Therefore, further constructions of plasmids and vaccine formulations were developed as described in Section 6 and below to provide protection against PRV.

### 7.3. PROTECTION OF MICE AGAINST LETHAL PRV INFECTION USING THE Cro/PRV gIII FUSION PROTEIN EXPRESSED BY p7/123AM IN A VACCINE FORMULATION

Twelve groups of mice were inoculated intramuscularly (IM) with 250 μg of Cro/PRV gIII fusion protein. The protein was treated as described in Table III. Three weeks after the initial injection, the mice were boosted with a

second 250 µg dose of protein. Control mice received no treatment.

The mice were bled retro-orbitally 7 days after the boost. Seven days after the second injection (Day 0) the mice were challenged with $10^5$ pfu of PRV per mouse. The results are shown in Table III. The mouse protection data indicated that immunization of the mice with the Cro/PRV gIII fusion protein solubilized with urea and treated with aluminum hydroxide and formaldehyde provided the best protection against challenge with PRV. The majority of mice did seroconvert as detected by immunoprecipitation of PRV specific glycoproteins of molecular weight 92,000 and 74,000 (gIII glycoprotein) from PRV infected cells labeled with $^3$H-glucosamine.

**TABLE III**

**VACCINATION AND CHALLENGE OF MICE WITH PRV-M-5,M6**

| Group | Formulation[1] | Deaths/Total Day 0 | Deaths/Total Day 4 | Deaths/Total Day 12 | Mean Time to Deaths (days) | % Protected[2] 4 day | % Protected[2] 12 day |
|---|---|---|---|---|---|---|---|
| A | Urea, Al, Form | 0/8 | 0/8 | 2/8 | 5.0** | 100 | 75.0 |
| B | Urea | 0/8 | 1/8 | 6/8 | 6.2** | 87.5 | 25.0 |
| C | Urea, L121 | 0/3 | 3/3 | 3/3 | 3.8** | 0 | 0 |
| D | Agg, Al, Form | 0/9 | 4/9 | 7/9 | 4.3* | 55.5 | 22.2 |
| E | Agg | 0/7 | 4/7 | 6/7 | 5.2 | 42.8 | 14.3 |
| F | Agg,L121 | 0/3 | 3/3 | 3/3 | 3.8** | 0 | 0 |
| G | Base, Al, Form | 0/9 | 0/9 | 5/9 | 7.0** | 100 | 44.4 |
| H | Base | 0/10 | 4/10 | 7/10 | 4.2 | 60 | 30.0 |
| I | Base L121 | 0/7 | 0/7 | 2/7 | 6.0** | 100 | 71.4 |
| J | Base,Al,Form,L121 | 0/9 | 0/9 | 4/9 | 7.0** | 100 | 55.5 |
| K | Al, Form | 0/10 | 10/10 | 10/10 | 3.1 | 0 | 0 |
| L | L121 | 0/10 | 9/10 | 10/10 | 3.1 1.1±.6 | 10 3.3 | 0 |
| Control | --- | 0/10 | 10/10 | 10/10 | 3.3 | 0 | 0 |

* = Significantly different from controls (K, L, Controls) at $p = .05$
** + Significantly different from controls (K, L, Controls) at $p = .01$

[1] Al is Aluminum Hydroxide, $Al(OH)_3$; Form is formeldehyde, Agg is aggregate protein isolated by the aggregate purification method; Base is 50 mM NaOH; and L121 is adjuvant.

[2] All days represent days after challenge with $10^5$ pfu of PRV per mouse.

0162738

### 7.4. PROTECTION OF SWINE USING THE Cro/PRV gIII FUSION PROTEIN EXPRESSED BY p7/123AM IN A VACCINE FORMULATION

The object of these experiments was to determine the efficacy of a swine vaccination in response to a challenge with PRV in dosage levels of 2 mg and 5 mg per injection and in combination with five different adjuvants.

Cro/PRV gIII fusion protein was prepared by the aggregate purification method as described supra from plasmid p7/123AM. The Cro/PRV gIII fusion protein was solubilized in SDS as follows: 100 mℓ of 0.1% SDS in 10mM Tris, pH9, 500 mM DTT was added to the protein pellet; the mixture was heated at 60°C for 30 minutes, centrifuged at 9000 x g for 25 minutes at room temperature; the protein was dialyzed against 10mM Tris, pH9. The preparation contained approximately 40% pure Cro/PRV gIII fusion protein expressed by p7/123AM.

Forty-five pigs were inoculated intramuscularly (2 mℓ/dose) in the right hind leg with the preparations described in Tables IV and V. Adjuvants used in these experiments included: Pluronic Polyol L121 (BASF Wyandette Corporation, Wyandotte, MI), DDA (dimethyldioctadecyl-ammonium bromide; Eastman Kodak Company, Rochester, NY); Avridine (CP-20,961; Pfizer, Inc., NY), Alhydrogel (Aluminum Hydroxide Gel; Superfos a/s, Denmark); and Freund's Complete Adjuvant (Difco Laboratories, Detroit, MI). Pigs were injected with the Cro/PRV gIII fusion protein expressed by p7/123AM in vaccine formulations, boosted after 2 weeks and were observed for local reactions and pyrogenicity. Temperatures were recorded, saliva and blood samples taken and clinical observations (respiratory, enteric, neurologic problems, weight gain rate, and death were carefully made. Pigs remained essentially normal throughout this period (Table IV).

Fourteen days after the first injection, pigs were challenged intranasally with approximately $10^7$ pfu of Becker strain PRV. The pig survival data is presented in Table V.

Table IV

**Summary of PRV-S4: Dose Effect**

| Vaccine Dose | Average total Clinical score[1] | Live/total(%) | Weight Gain Rate[2] | 2-5 day Post Challenge Temperature[3] | Health Score[4] | Number/total Seropositive[5] |
|---|---|---|---|---|---|---|
| 2mg | 13.1 $\pm$ 17.7 | 12/14 (85.7) | 0.31 $\pm$ 1.8 | 41.4 $\pm$ 0.6 | 1.1 | 12/14 |
| 5mg | 6.9 $\pm$ 4.8 | 14/14 (100) | 0.57 $\pm$ 1.8 | 41.5 $\pm$ 0.6 | 2.0 | 9/14 |
| 0mg | 45.4 $\pm$ 12.6 | 3/13 (23.0) | -0.92 $\pm$ 1.7 | 41.5 $\pm$ 0.6 | -0.7 | 0/13 |
| killed[6] | 18.0 $\pm$ 8.5 | 2/2 (100) | 0.96 $\pm$ 2.0 | 41.6 $\pm$ 0.7 | 1.5 | 2/2 |

1. A subjective scoring system based upon respiratory, neurologic, and enteric disease symptoms and death.
2. Weight gain rate is averaged over the 14 days post challenge.
3. Average temperature (degrees C) before challenge = 39.7 $\pm$ .3 (103.5°).
4. Health score = reciprocal of clinical disease score (x10) plus weight gain rate.
5. Serologic test is plaque-reduction.
6. Vaccinated with killed virus vaccine, Norden laboratories.

Table V

**Summary of PRV-S4: Adjuvant Effect**

| Vaccine Adjuvant | Average total Clinical score[1] | Live/total(%) | Weight Gain Rate[2] | 2-5 day Post Callenge Temperature[3] | Health Score[4] | Number/total Seropositive[5] |
|---|---|---|---|---|---|---|
| Freund's | 12.8 ± 23.7 | 5/6 (83.3) | 0.92 ± 2.2 | 41.1 ± 0.6 | 1.7 | 6/6 |
| L121 | 2.3 ± 0.5 | 4/4 (100) | 0.94 ± 1.9 | 41.5 ± 0.4 | 5.4 | 4/4 |
| DDA | 13.7 ± 15.2 | 5/6 (83.3) | -0.04 ± 2.0 | 41.4 ± 0.6 | 0.7 | 5/6 |
| Avridine | 8.0 ± 6.2 | 6/6 (100) | 0.19 ± 1.6 | 41.5 ± 0.5 | 1.4 | 4/6 |
| Alhydro | 10.5 ± 4.6 | 6/6 (100) | 0.44 ± 1.7 | 41.6 ± 0.4 | 1.4 | 2/6 |
| Control[6] | 45.4 ± 12.6 | 3/13 | -0.92 ± 1.7 | 41.5 ± 0.6 | -0.7 | 0/13 |

1. A subjective scoring system based upon respiratory, neurologic, and enteric disease symptoms and death.

2. Weight gain rate is averaged over the 14 days post challenge.

3. Average temperature (degrees C) before challenge = 39.7 ± .3 (103.5°).

4. Health score = reciprocal of clinical disease score (x10) plus weight gain rate.

5. Serologic test is plaque-reduction.

6. Control was placebo including average of effect of all adjuvants without PRV antigen.

See text for description of the adjuvants.

It can be concluded from these experiments that the PRV antigen raised the survival rate of pigs by approximately 60% when compared to the non-antigen treated animals. PRV antigens administered with avridine or $AlOH_3$ caused the lease degree of reactions, whereas FCA, L121 and DDA were the most severe.

## 8. DEPOSIT OF MICROORGANISMS

The following *E. coli* strains carrying the listed plasmids have been deposited with the Agricultural Research Culture Collection (NRRL), Peoria, IL, or with In Vitro International, Inc. (IVI), Ann Arbor, MI and have been assigned the following accession numbers:

| E. Coli Strain | Plasmid | Accession Numbers |
|---|---|---|
| K12, NF1829 | ptacNB | NRRL B-15760 |
| K12, MC1000 | pPRV49 | NRRL B-15762 |
| K12, NF1829 | pssgB | NRRL B-15763 |
| K12, NF1829 | pNT7/123 | IVI-10049 |
| K12, MC1000 | pBamHI-1 | IVI-10048 |

The present invention is not to be limited in scope by the microorganisms deposited, since the deposited embodiment is intended as single illustration of one aspect of the invention and any microorganisms which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claim.

It is also to be understood that all base pair sizes given for nucleotides are approximate and are used for purpose of description.

WE CLAIM:

1. A process for producing a polypeptide having an immunoreactive and antigenic determinant of a Pseudorabies virus glycoprotein, comprising:

(a) culturing a unicellular organism containing a recombinant vector comprising a DNA sequence coding for a polypeptide having an immunoreactive and antigenic determinant of Pseudorabies virus protein and capable of being replicated, transcribed and translated in the unicellular organism; and

(b) isolating said polypeptide from the culture.

2. The process according to claim 1, wherein the polypeptide is immunogenic.

3. The process according to claim 1, wherein the recombinant vector was introduced into the unicellular organism by transformation.

4. The process according to claim 1, wherein the recombinant vector was introduced into the unicellular organism by transduction.

5. The process according to claim 1, wherein the recombinant vector was introduced into the unicellular organism by transfection.

6. The process according to claim 1, wherein the information contained in the DNA sequence was obtained from a Pseudorabies virus genome.

7. The process according to claim 6, wherein the information contained in the DNA sequence was obtained from a Pseudorabies virus.

8. The process according to claim 1, wherein the DNA sequence is obtained from a DNA vector containing a Pseudorabies virus DNA sequence.

9. The process according to claim 1, wherein the DNA sequence codes for a glycoprotein of Pseudorabies virus.

10. The process according to claim 1, wherein the unicellular organism comprises a eucaryotic organism.

11. The process according to claim 1, wherein the recombinant vector comprises a vaccinia virus.

12. The process according to claim 1, wherein the unicellular organism comprises a procaryotic organism.

13. The process according to claim 12, wherein the procaryotic organism comprises *Escherichia coli*.

14. The process according to claim 13, wherein the *Escherichia coli* has accession NRRL No. B-15760

15. The process according to claim 13, wherein the *Escherichia coli* has accession NRRL No. B-15763

16. The process according to claim 13, wherein the *Escherichia coli* has accession NRRL No. B-15762

17. The process according to claim 13, wherein the *Escherichia coli* has accession IVI No. 10049.

18. The process according to claim 13, wherein the Escherichia coli has accession IVI No. 10048.

19. A process for preparing a unicellular organism having a DNA sequence coding for a polypeptide having an immunoreactive and antigenic determinant of a Pseudorabies virus protein, comprising: introducing a recombinant vector into a unicellular organism, said recombinant vector comprising a DNA sequence coding for a polypeptide having an immunoreactive and antigenic determinant of a Pseudorabies virus protein and capable of being replicated, transcribed and translated in the unicellular organism.

20. A recombinant vector, comprising: a DNA sequence coding for an immunoreactive and antigenic determinant of a Pseudorabies virus protein.

21. The recombinant DNA vector according to claim 20, wherein the DNA sequence codes for a Pseudorabies virus glycoprotein as depicted in FIG. 3, or any portion thereof coding for a polypeptide having an immunoreactive and antigenic determinant of a Pseudorabies virus glycoprotein.

22. The recombinant DNA vector according to claim 21, wherein the vector comprises pPRV49, or a mutant, recombinant, or genetically engineered derivative thereof.

23. The recombinant DNA vector according to claim 20, wherein the DNA sequence codes for a Pseudorabies virus glycoprotein as partially depicted in Fig. 13, or any portion thereof coding for a polypeptide having an immunoreactive and antigenic determinant of a Pseudorabies virus glycoprotein.

24. The recombinant DNA vector according to claim 23, wherein the vector comprises pBamHI-1, or a mutant, recombinant, or genetically engineered derivative thereof.

25. The recombinant DNA vector according to claim 20, wherein said DNA sequence is under the control of expression control elements.

26. The recombinant DNA vector according to claim 25 wherein the vector comprises ptacNB, or a mutant recombinant, or genetically engineered derivative thereof.

27. The recombinant DNA vector according to claim 20, further comprising the essential portions of the pBR322 replicon.

28. The recombinant DNA vector according to claim 20, wherein said DNA sequence is connected in the correct translational reading frame to a second DNA sequence coding for a protein.

29. The recombinant DNA vector according to claim 25, wherein the vector comprises pNT/123, or a mutant recombinant, or genetically engineered derivative thereof.

30. The recombinant DNA vector according to claim 25, wherein the vector comprises pssgB or a mutant, recombinant, or genetically engineered derivative thereof.

31. A unicellular organism containing the recombinant DNA vector of claim 20.

32. A unicellular organism containing the recombinant DNA vector of claim 21.

33. A unicellular organism containing the recombinant DNA vector of claim 23.

34. A unicellular organism containing the recombinant DNA vector of claim 25.

35. A unicellular organism containing the recombinant DNA vector of claim 27.

36. A unicellular organism containing the recombinant DNA vector of claim 28.

37. An Escherichia coli bacterium containing the recombinant DNA vector of claim 20.

38. An Escherichia coli bacterium containing the recombinant DNA vector of claim 21.

39. An Escherichia coli bacterium containing the recombinant DNA vector of claim 23.

40. An Escherichia coli bacterium containing the recombinant DNA vector of claim 25.

41. An Escherichia coli bacterium containing the recombinant DNA vector of claim 27.

42. An Escherichia coli bacterium containing the recombinant DNA vector of claim 28.

43. An Escherichia coli bacterium of claim 40 deposited with the NRRL and assigned NRRL accession No. B-15760 and mutants, recombinants, and genetically engineered derivatives thereof.

44. An Escherichia coli bacterium of claim 40 deposited with the NRRL and assigned NRRL accession No. B-15763 and mutants, recombinants, and genetically engineered derivatives thereof.

45. An Escherichia coli bacterium of claim 40 deposited with the IVI and assigned IVI accession No.

10049 and mutants, recombinants, and genetically engineered derivatives thereof.

46. An *Escherichia coli* bacterium of claim 37 deposited with the NRRL and assigned NRRL accession No. B-15762 and mutants, recombinants, and genetically engineered derivatives thereof.

47. An *Escherichia coli* bacterium of claim 37 deposited with the IVI and assigned IVI accession No. 10048 and mutants, recombinants, and genetically engineered derivatives thereof.

48. A polypeptide having an immunoreactive and antigenic determinant of a Pseudorabies virus protein produced by *Escherichia coli* of claim 43.

49. A polypeptide having an immunoreactive and antigenic determinant of a Pseudorabies virus protein produced by *Escherichia coli* of claim 44.

50. A polypeptide having an immunoreactive and antigenic determinant of a Pseudorabies virus protein produced by *Escherichia coli* of claim 45.

51. A polypeptide produced according to the process of claim 2.

52. A vaccine formulation comprising the polypeptide of claim 51 which when used as an immunogen, is capable of eliciting antibodies against virus.

53. A vaccine formulation according to claim 52 further comprising a compatible pharmaceutical carrier therefor.

54. A vaccine formulation comprising a lysate of the unicellular organism of claim 34.

55. The vaccine formulation according to claim 54 further comprising a compatible pharmaceutical carrier therefor.

56. A vaccine formulation comprising a lysate of the <u>Escherichia coli</u> of claim 40.

57. The vaccine formulation according to claim 56 further comprising a compatible pharmaceutical carrier therefor.

58. A vaccine formulation comprising a lysate of the <u>Escherichia coli</u> of claim 43.

59. The vaccine formulation according to claim 58 further comprising a compatible pharmaceutical carrier therefor.

60. A vaccine formulation comprising a lysate of the <u>Escherichia coli</u> of claim 44.

61. The vaccine formulation according to claim 60 further comprising a compatible pharmaceutical carrier therefor.

62. A vaccine formulation comprising a lysate of the <u>Escherichia coli</u> of claim 45.

63. The vaccine formulation according to claim 62 further comprising a compatible pharmaceutical carrier therefor.

64. A vaccine formulation comprising a cell extract of the unicellular organism of claim 34.

65. The vaccine formulation according to claim 64 further comprising a compatible pharmaceutical carrier therefor.

66. A vaccine formulation comprising a cell extract of the _Escherichia_ _coli_ of claim 40.

67. The vaccine formulation according to claim 66 further comprising a compatible pharmaceutical carrier therefor.

68. A vaccine formulation comprising a cell extract of the _Escherichia_ _coli_ of claim 43.

69. The vaccine formulation according to claim 68 further comprising a compatible pharmaceutical carrier therefor.

70. A vaccine formulation comprising a cell extract of the _Escherichia_ _coli_ of claim 44.

71. The vaccine formulation according to claim 70 further comprising a compatible pharmaceutical carrier therefor.

72. A vaccine formulation comprising a cell extract of the _Escherichia_ _coli_ of claim 45.

73. The vaccine formulation according to claim 72 further comprising a compatible pharmaceutical carrier therefor.

74. An aggregate protein comprising an aggregate protein derived from the unicellular organism of claim 34.

75. An aggregate protein comprising an aggregate protein derived from the Escherichia coli of claim 40.

76. An aggregate protein comprising an aggregate protein derived from the Escherichia coli of claim 43.

77. An aggregate protein comprising an aggregate protein derived from the Escherichia coli of claim 44.

78. An aggregate protein comprising an aggregate protein derived from the Escherichia coli of claim 45.

79. A vaccine formulation comprising the aggregate protein of claim 74.

80. The vaccine formulation according to claim 79 further comprising a compatible pharmaceutical carrier therefor.

81. A vaccine formulation comprising the aggregate protein of claim 75.

82. The vaccine formulation according to claim 81 further comprising a compatible pharmaceutical carrier therefor.

83. A vaccine formulation comprising the aggregate protein of claim 76.

84. The vaccine formulation according to claim 83 further comprising a compatible pharmaceutical carrier therefor.

85. A vaccine formulation comprising the aggregate protein of claim 77.

86. The vaccine formulation according to claim 85 further comprising a compatible pharmaceutical carrier therefor.

87. A vaccine formulation comprising the aggregate protein of claim 78.

88. The vaccine formulation according to claim 87 further comprising a compatible pharmaceutical carrier therefor.

89. A polypeptide comprising a substantially pure polypeptide having an immunoreactive and antigenic determinant of a Pseudorabies virus protein.

90. A vaccine formulation comprising the polypeptide of claim 89.

91. The vaccine according to claim 90 further comprising a compatible pharmaceutical carrier therefor.

92. The polypeptide according to claim 89 in which the polypeptide is fused to a second polypeptide.

93. A vaccine formulation comprising the polypeptide of claim 92.

94. The vaccine according to claim 93 further comprising a compatible pharmaceutical carrier therefor.

95. The polypeptide according to claim 89 in which the polypeptide is capable of forming aggregates.

96. A vaccine formulation comprising the polypeptide of claim 95.

97. The vaccine according to claim 96 further comprising a compatible pharmaceutical carrier therefor.

98. A vaccine formulation according to claim 52 comprising a live virus vaccine.

99. The vaccine formulation according to claim 98 wherein the virus comprises a recombinant virus.

100. The vaccine formulation according to claim 99 further comprising a vaccinia virus recombinant.

101. A method for localizing genes encoding antigenic viral proteins within large DNA virus genomes, comprising:

(a) fragmenting genomic DNA of a virus and ligating the DNA fragments into DNA expression vectors to form recombinant DNA expression vectors that direct host cell synthesis of fusion proteins;

(b) introducing the recombinant DNA expression vectors into host cells and culturing the host cells so that fusion proteins are produced;

(c) separating the fusion proteins produced by the host cells in step (b) and contacting the separated fusion proteins with specific antibody directed against a denatured antigenic protein of the virus of step (a) under conditions

sufficient to allow the antibody to bind to antigen;

(d) detecting the bound antibody and identifying a host cell which produced a fusion protein that bound to the antibody;

(e) isolating the recombinant DNA vector from the host cell identified in step (d);

(f) contacting a portion of the denatured recombinant DNA vector isolated in step (e) with denatured restriction fragments of DNA obtained from the genomic DNA isolated from the virus of step (a) under conditions that allow hybridization of complementary DNA sequences; and

(g) detecting hybridization and identifying the restriction fragments of the genomic viral DNA which hybridized so that the areas of hybridization are identified on the genomic map of the virus DNA.

102. The method according to claim 101 wherein the DNA virus genome comprises a Pseudorabies virus genome.

FIG. 1

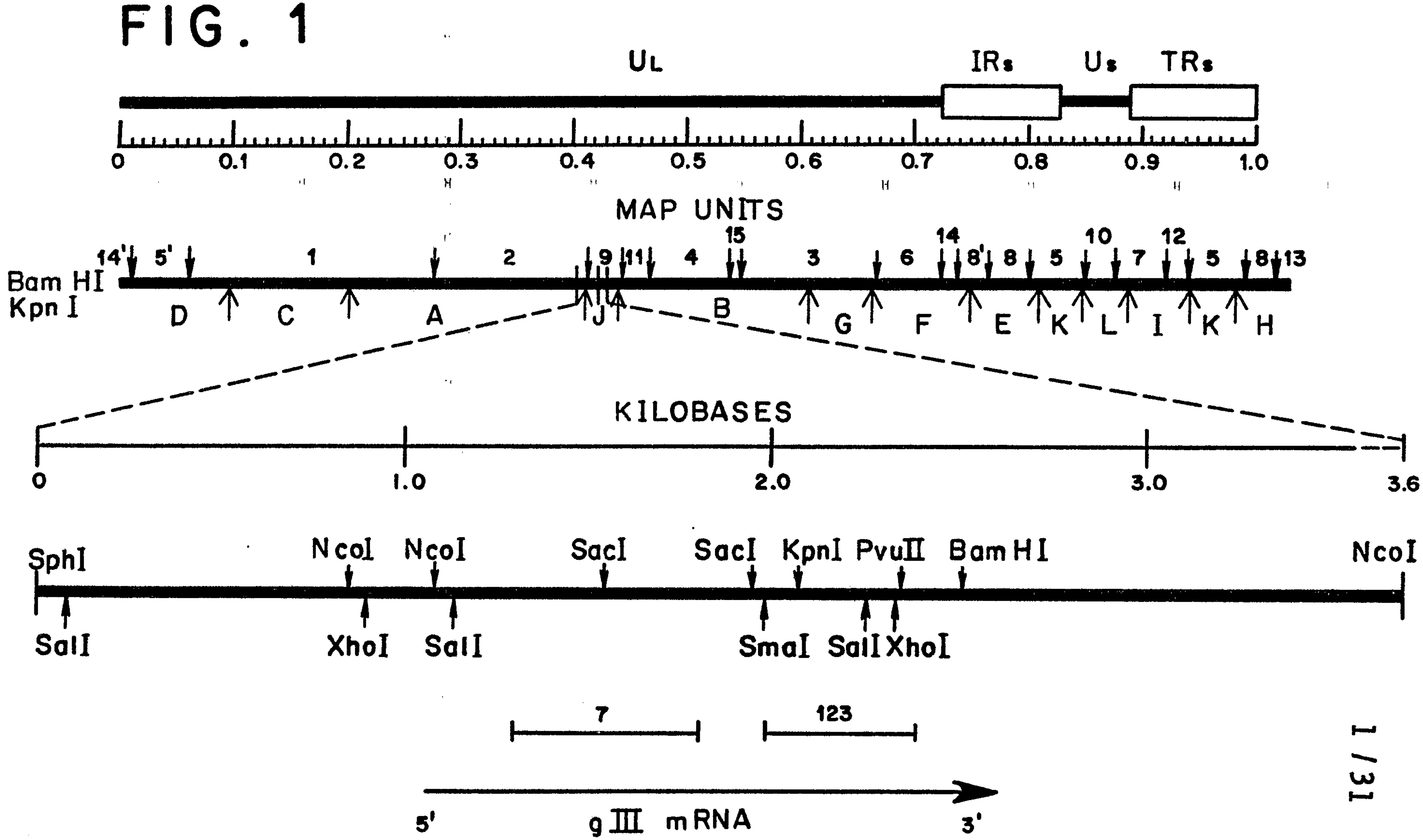
UL
IRs
Us
TRs
0
0.1
0.2
0.3
0.4
0.5
0.6
0.7
0.8
0.9
1.0
MAP UNITS
Bam HI
Kpn I
14'
5'
1
2
9
11
4
15
3
6
14
8'
8
5
10
7
12
5
8
13
D
C
A
J
B
G
F
E
K
L
I
K
H
KILOBASES
0
1.0
2.0
3.0
3.6
SphI
NcoI
NcoI
SacI
SacI
KpnI
PvuII
BamHI
NcoI
SalI
XhoI
SalI
SmaI
SalI
XhoI
7
123
5'
gIII mRNA
3'

# FIG. 2A

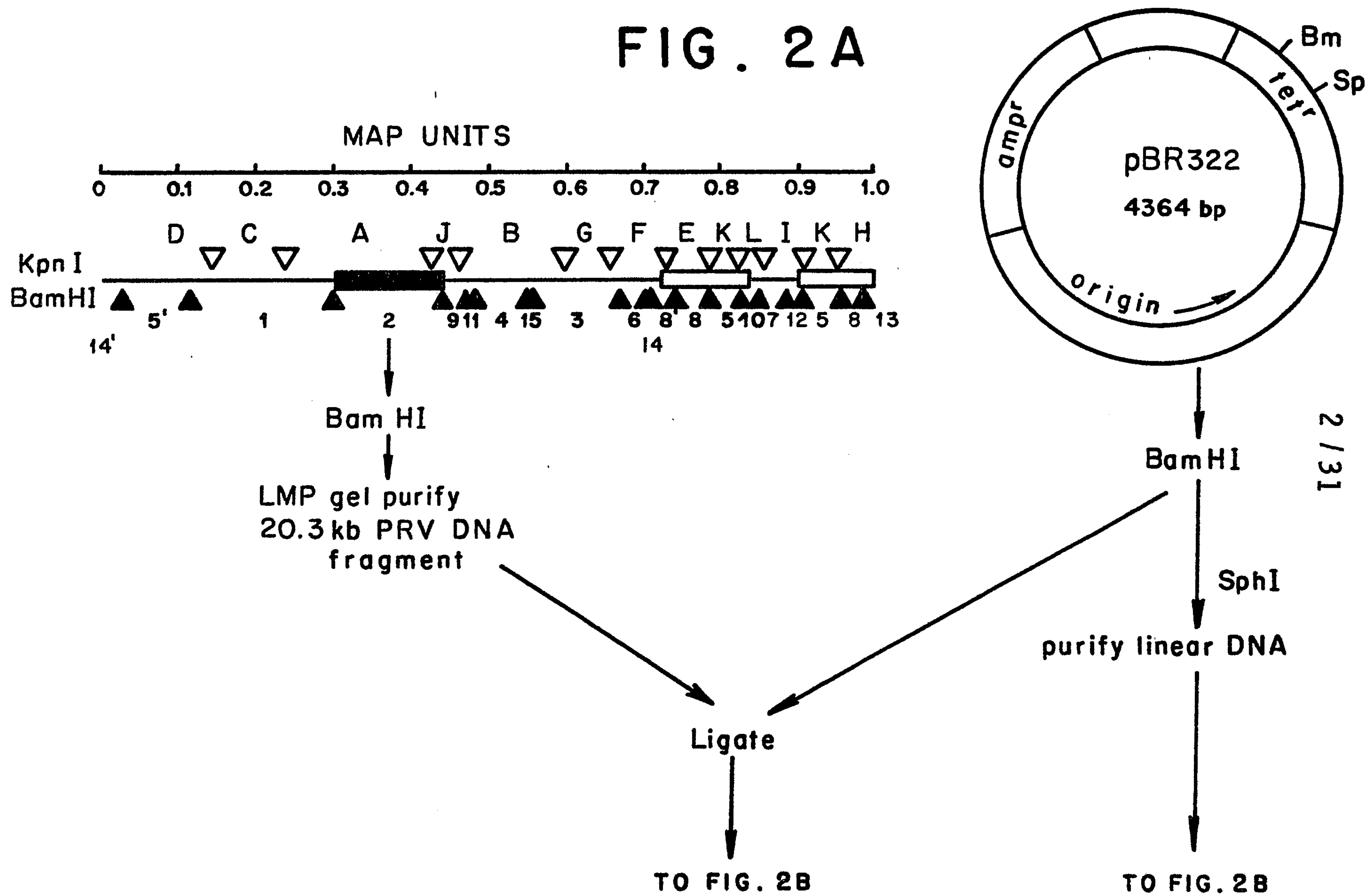

MAP UNITS
0
0.1
0.2
0.3
0.4
0.5
0.6
0.7
0.8
0.9
1.0
D C A J B G F E K L I K H
Kpn I
BamHI
5'
1
2
9 11
4
15
3
6
8'
8
5
10
7
12
5
8
13
14'
14
Bam HI
LMP gel purify
20.3 kb PRV DNA
fragment
pBR322
4364 bp
ampr
tetr
origin
Bm
Sp
BamHI
SphI
purify linear DNA
Ligate
TO FIG. 2B
TO FIG. 2B

FIG. 2B

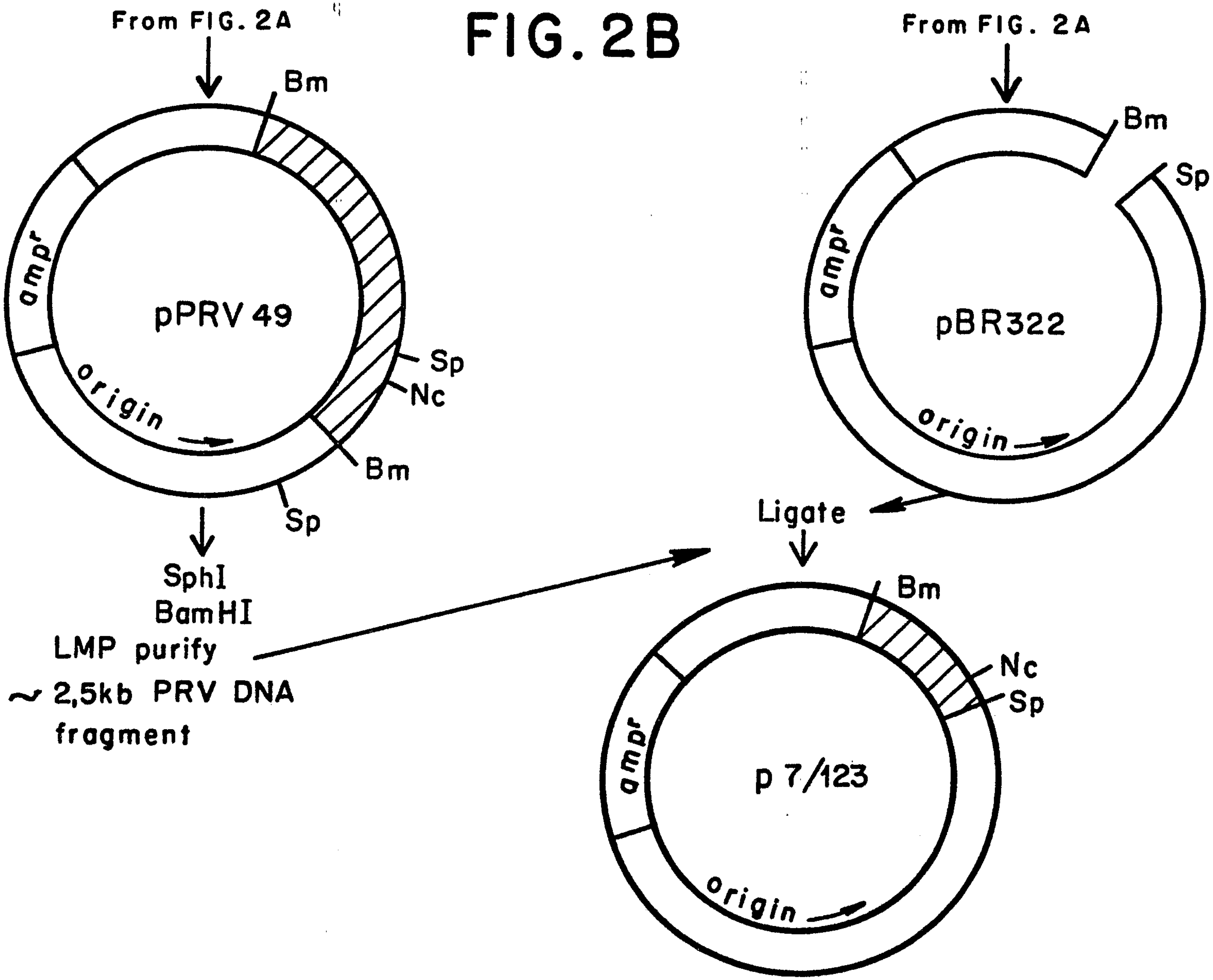
From FIG. 2A
Bm
pPRV 49
ampr
origin
Sp
Nc
Bm
Sp
SphI
BamHI
LMP purify
~ 2,5kb PRV DNA
fragment
From FIG. 2A
Bm
Sp
pBR322
ampr
origin
Ligate
Bm
Nc
Sp
p 7/123
ampr
origin

# FIG. 3A



```
                                    AGG CGG ACC ACG TCC GCT GCG CCA CAC   27

    CCG CGC GTA CCG GCT CGC CGC CGC GCA CGT GAC GCG GGC CCT   69

    GCT GGT GCA GGC GTA CGT GAC CGT CGC CAT GTG CGC CAC TAG  111

     CAT
    CAT TAA ATC CGT TTC CTG ATT CAC GCC CAC GCT CGC GCG TTT  153

     TATA
    TTA AAA CCG CGA TGG GGG GAC GGG GGG CCA TTC GCA CGC GCC  195

     NcoI
    ATG GCC TCG CTC GCG CGT GCG ATG CTC GCT CTG CTG GCG CTC  237
1   MET Ala Ser Leu Ala Arg Ala Met Leu Ala Leu Leu Ala Leu

                                            SalI
    TAC GCG GCG GCC ATC GCC GCG GCG CCG TCG ACC ACG ACG GCG  279
15  Tyr Ala Ala Ala Ile Ala Ala Ala Pro Ser Thr Thr Thr Ala

    CTC GAC ACG ACG CCC AAC GGG GGC GGC GGC GGC AAC AGC AGC  321
29  Leu Asp Thr Thr Pro Asn Gly Gly Gly Gly Gly Asn Ser Ser

                        → pDPR7
    GAG GGA GAA CTC TCG CCC TCT CCG CCC CCG ACC CCC GCG CCC  363
43  Glu Gly Glu Leu Ser Pro Ser Pro Pro Pro Thr Pro Ala Pro

    GCC TCG CCC GAG GCG GGC GCG GTC TCG ACG CCC CCG GTC CCG  405
57  Ala Ser Pro Glu Ala Gly Ala Val Ser Thr Pro Pro Val Pro

    CCG CCC TCG GTC TCG CGC AGG AAG CCC CCG CGG AAC AAC AAC  447
71  Pro Pro Ser Val Ser Arg Arg Lys Pro Pro Arg Asn Asn Asn

    CGG ACG CGC GTC CAC GGC GAC AAG GCC ACC GCG CAC GGG CGC  489
85  Arg Thr Arg Val His Gly Asp Lys Ala Thr Ala His Gly Arg

    AAG CGC ATC GTG TGC CGG GAG CGG CTG TTC TCG GCG CGG GTG  531
99  Lys Arg Ile Val Cys Arg Glu Arg Leu Phe Ser Ala Arg Val

    GGG GAC GCG GTC AGC TTC GGG TGC GCC GTC TTC CCG CGC GCC  573
113 Gly Asp Ala Val Ser Phe Gly Cys Ala Val Phe Pro Arg Ala

    GGG GAG ACC TTC GAG GTC CGC TTC TAC CGC CGC GGG CGC TTC  615
127 Gly Glu Thr Phe Glu Val Arg Phe Tyr Arg Arg Gly Arg Phe
```

# FIG. 3B



```
    CGC TCG CCC GAC GCC GAC CCC GAG TAC TTT GAC GAG CCC CCG  657
141 Arg Ser Pro Asp Ala Asp Pro Glu Tyr Phe Asp Glu Pro Pro

            SacI
    CGC CCG GAG CTC CCG CGG GAG CGG CTC CTC TTC AGC TCC GCC  699
155 Arg Pro Glu Leu Pro Arg Glu Arg Leu Leu Phe Ser Ser Ala

    AAC GCC TCC CTC GCC CAC GCG GAC GCG CTC GCG CCC GTC GTC  741
169 Asn Ala Ser Leu Ala His Ala Asp Ala Leu Ala Pro Val Val

    GTC GAG GGC GAG CGC GCG ACC GTC GCC AAC GTC TCG GGC GAG  783
183 Val Glu Gly Glu Arg Ala Thr Val Ala Asn Val Ser Gly Glu

    GTG TCC GTG CGC GTG GCC GCG GCG GAC GCC GAG ACC GAG GGC  825
197 Val Ser Val Arg Val Ala Ala Ala Asp Ala Glu Thr Glu Gly

    GTC TAC ACG TGG CGC GTG CTG TCC GCC AAC GGC ACC GAG GTC  867
211 Val Tyr Thr Trp Arg Val Leu Ser Ala Asn Gly Thr Glu Val

                                                pDPR7
    CGG AGC GCC AAC GTC TCG CTC CTC CTG TAC AGC CAG CCC GAG  909
225 Arg Ser Ala Asn Val Ser Leu Leu Leu Tyr Ser Gln Pro Glu

    TTC GGC CTG AGC GCG CCG CCC GTC CTC TTC GGT GAG CCC TTC  951
239 Phe Gly Leu Ser Ala Pro Pro Val Leu Phe Gly Glu Pro Phe

    CGG GCG GTG TGC GTC GTC CGC GAC TAC TAC CCG CGG CGC AGC  993
253 Arg Ala Val Cys Val Val Arg Asp Tyr Tyr Pro Arg Arg Ser

    GTG CGC CTG CGC TGG TTC GCG GAC GAG CAC CCG GTG GAC GCC 1035
267 Val Arg Leu Arg Trp Phe Ala Asp Glu His Pro Val Asp Ala

                                            SacI
    GCC TTC GTG ACC AAC AGC ACC GTG GCC GAC GAG CTC GGG CGC 1077
281 Ala Phe Val Thr Asn Ser Thr Val Ala Asp Glu Leu Gly Arg

    CGC ACG CGC GTC TCC GTG GTG AAC GTG ACG CGC GCG GAC GTC 1119
295 Arg Thr Arg Val Ser Val Val Asn Val Thr Arg Ala Asp Val

     SmaI      pDPR123
    CCG GGC CTC GCG GCC GCG GAC GCC GCG GAC GCG CTC GCG CCG 1161
309 Pro Gly Leu Ala Ala Ala Asp Ala Ala Asp Ala Leu Ala Pro
```

# FIG. 3C



KpnI
AGC CTG CGC TGC GAG GCC GTG TGG TAC CGC GAC AGC GTG GCC 1203
323 Ser Leu Arg Cys Glu Ala Val Trp Tyr Arg Asp Ser Val Ala

TCG CAG CGC TTC TCC GAG GCC CTG CGC CCC CAC GTC TAC CAC 1245
337 Ser Gln Arg Phe Ser Glu Ala Leu Arg Pro His Val Tyr His

CCG GCG GCG GTC TCG GTG CGC TTC GTC GAG GGC TTC GCC GTC 1287
351 Pro Ala Ala Val Ser Val Arg Phe Val Glu Gly Phe Ala Val

TGC GAC GGC CTC TGC GTG CCC CCG GAG GCG CGC CTC GCC TGG 1329
365 Cys Asp Gly Leu Cys Val Pro Pro Glu Ala Arg Leu Ala Trp

TCC GAC CAC GCC GCC GAC ACC GTC TAC CAC CTC GGC GCC TGC 1371
379 Ser Asp His Ala Ala Asp Thr Val Tyr His Leu Gly Ala Cys

GCG GAG CAC CCC GGC CTG CTC AAC GTG CGG AGC GCC CGC CCG 1413
393 Ala Glu His Pro Gly Leu Leu Asn Val Arg Ser Ala Arg Pro

SalI XhoI
CTG TCG GAC CTC GAC GGG CCC GTC GAC TAC ACC TGC CGC CTC 1455
407 Leu Ser Asp Leu Asp Gly Pro Val Asp Tyr Thr Cys Arg Leu

PvuII pDPR123
GAG GGC CTG CCC TCG CAG CTG CCC GTC TTC GAG GAC ACG CAG 1497
421 Glu Gly Leu Pro Ser Gln Leu Pro Val Phe Glu Asp Thr Gln

CGC TAC GAC GCC TCC CCC GCG TCC GTG AGC TGG CCC GTC GTG 1539
435 Arg Tyr Asp Ala Ser Pro Ala Ser Val Ser Trp Pro Val Val

BamHI
AGC AGC ATG ATC GTC GTC ATC GCC GGC ATC GGG ATC CTG GCC 1581
449 Ser Ser Met Ile Val Val Ile Ala Gly Ile Gly Ile Leu Ala

ATC GTG CTG GTC ATC ATG GCG ACG TGC GTC TAC TAC CGC CAG 1623
463 Ile Val Leu Val Ile Met Ala Thr Cys Val Tyr Tyr Arg Gln

GCG GGG CCG TGA CGT CCC GCG CGT CCC CCC CCA CGT CGA ATC 1665
477 Ala Gly Pro ***

AAT AAA CGA CAG CGA GTC CGA CCC GCG CCC TCG CGC TTG TGT 1707

GTG TCG CGC GCG CCC 1722

# FIG. 4

```
     10        20        30        40        50
MAPGRVGLAVVLWGLLWLGAGVAGGSETASTGPTITAGAVTNASEAPTSGSPGSAASPEV
                  : :  :      :    : :   :     :    :  : :
                 MASL-AR-AMLALLALYAAAIAAAPSTTTALDTTPNGGGGGNSSEG
                          10        20        30        40

      70        80        90        100       110
TPTSTPNPNNVTQNKTTPTEPASPPTTPKPTSTPKSPPTSTPDPKPK--NNTTPAKSGRP
    : :         ::  ::::     :::  :: :    ::   :: :
ELSPSPPP--------TPA-PASPEAGAV--STPPVPPPSVSRRKPPRNNNRTRVHGDKA
    50                 60         70        80        90

120       130       140       150       160       170
TKPPGPVWCDR-RDPLARYGSRVQIRCRFRNSTRMEFRLQIWRYSMGPSPPIAPAPDLEE
:       : :  :: : :   :         :    :    ::   :   :
TAHGRKRIVCRERLFSARVGDAVSFGCAVFPRAGETFEVRFYRRGRFRSPDADPEYFDEP
   100       110       120       130       140       150

 180       190                 200       210       220
VLTNITAP--PGGLLVYDSAPN-------LTDPHVLWAEGAGPGADPPLYSV--------
          :  :   :  ::          :  : :   : :       ::
PR-----PELPRERLLFSSANASLAHADALA-PVVVEGERATVANVSGEVSVRVAAADAE
           160       170        180       190       200

      230       240       250       260       270
TGPLPTQRLIIGEVTPATQGMYYLAWGRMDSPHEYGTWVRVRMFRPPSLTLQPHAVMEGQ
:  : :     :         :     :  : :                :   :  :
TEGVYTWRVLSANGTEVRSANVSLLLY---SQPEFG-------------LSAPPVLFGE
 210       220       230       240

      290       300       310       320       330
PFKATCTAAAYYPRNPVEFDWFEDDRQVFNPGQIDTQTHEHPDGFTTVSTV--TSEAVGG
:: : :   ::::  :   ::  :   :             : :: :  :   :  : :
PFRAVCVVRDYYPRRSVRLRWFADEHPVDAAFVTNSTVADELGRRTRVSVVNVTRADVPG
      260       270       280       290       300

340                 350       360       370       380
QVP--------P--RTFTCQMTWHRDSVTFSRRNATGLALVLPRPTITMEFGVRHV----
           :   :   :   : ::::   :       ::  :           :: :
LAAADAADALAPSLR---CEAVWYRDSVASQRFSE---ALR-PHVYHPAAVSVRFVEGFA
      320             330       340           350       360

   390       400       410       420       430
VCTAGCVPEGVTFAWFLGDDPSPAAKSAVTAQESCD-HPGLATVRSTLPISY-DYSE-YI
::  :::    ::       :  :   :     :   ::::  :::  : :  :     :
VCDGLCVPPEARLAW------SDHAADTVYHLGACAEHPGLLNVRSARPLSDLDGPVDYT
   370                 380       390       400       410

      450       460       470       480       490
CWLTGYPAGIPVLEHHGSHQPPPRDPTERQVIEAIEWVGIGIGVLAAGVLVVTAI-VYVV
: : : :    ::  :         :      :    :  :   :::  ::  :::   :  ::
CRLEGLPSQLPVFEDTQRYDASPASVSWPVVSSMIV-VIAGIGILAI-VLVIMATCVYY-
 420       430       440       450           460       470

      510       520
RTSQSRQRIGGNARPPVTFLISI                    HSV-1 gC
      ::
-----RQAGP                                 PRV gIII
```

0162738

8 / 31

FIG. 5A

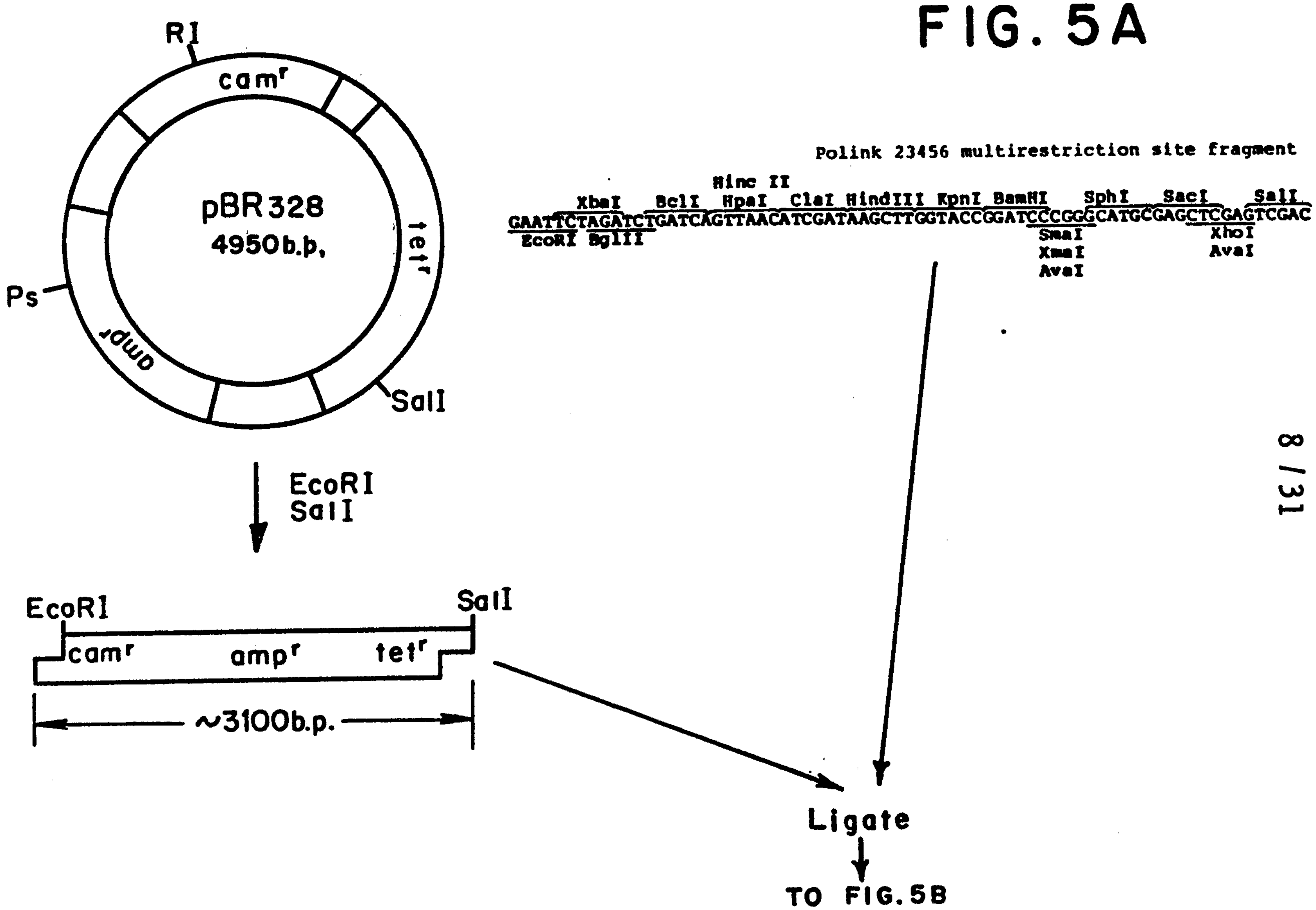

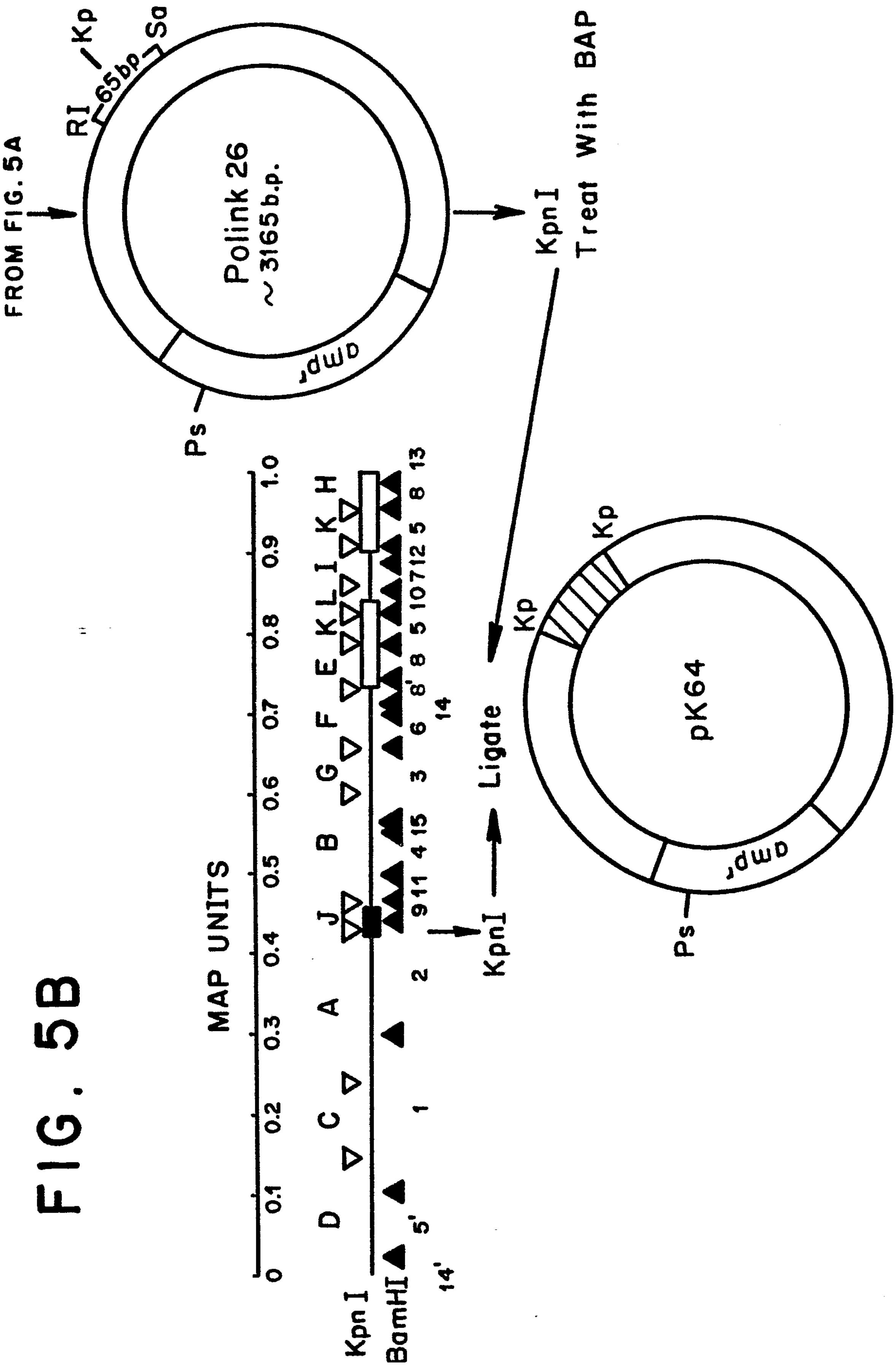
FROM FIG. 5A
RI
65bp
Kp
Sa
Polink 26
~3165 b.p.
ampr
Ps
KpnI
Treat With BAP
MAP UNITS
0
0.1
0.2
0.3
0.4
0.5
0.6
0.7
0.8
0.9
1.0
D C A J B G F E K L I K H
KpnI
BamHI
14'
5'
1
2
9 11
4 15
3
6
8'
14
8
5 10 7 12 5
8 13
KpnI
Ligate
Kp
Kp
pK64
ampr
Ps

FIG. 5B

FIG. 6

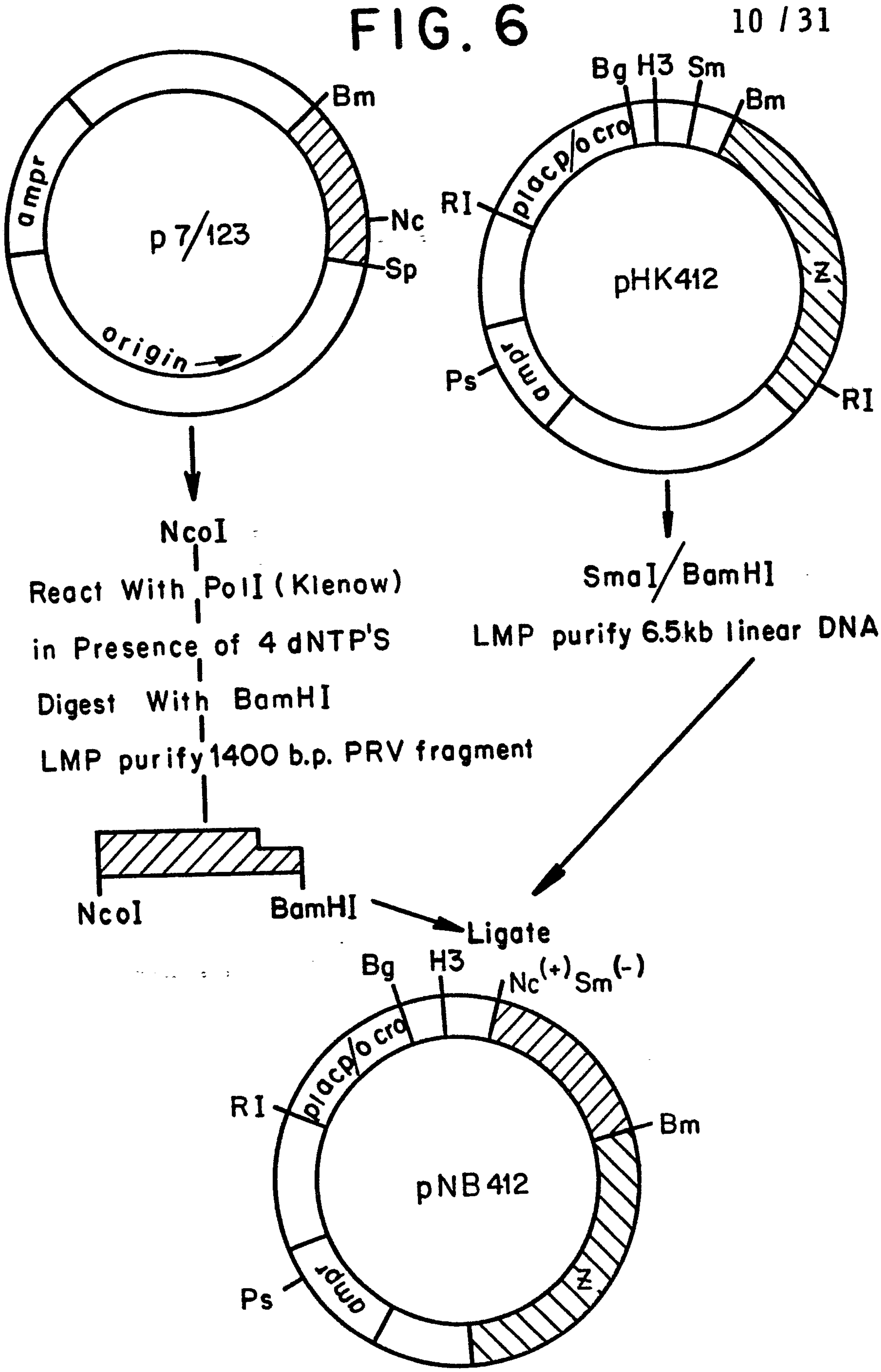
Bm
ampr
p 7/123
Nc
Sp
origin
Bg H3 Sm
Bm
placp/o cro
RI
pHK412
Z
ampr
Ps
RI
NcoI
React With PolI (Klenow)
in Presence of 4 dNTP'S
Digest With BamHI
LMP purify 1400 b.p. PRV fragment
SmaI/BamHI
LMP purify 6.5kb linear DNA
NcoI
BamHI
Ligate
Bg
H3
Nc(+) Sm(-)
placp/o cro
RI
Bm
pNB412
ampr
Z
Ps

FIG. 7



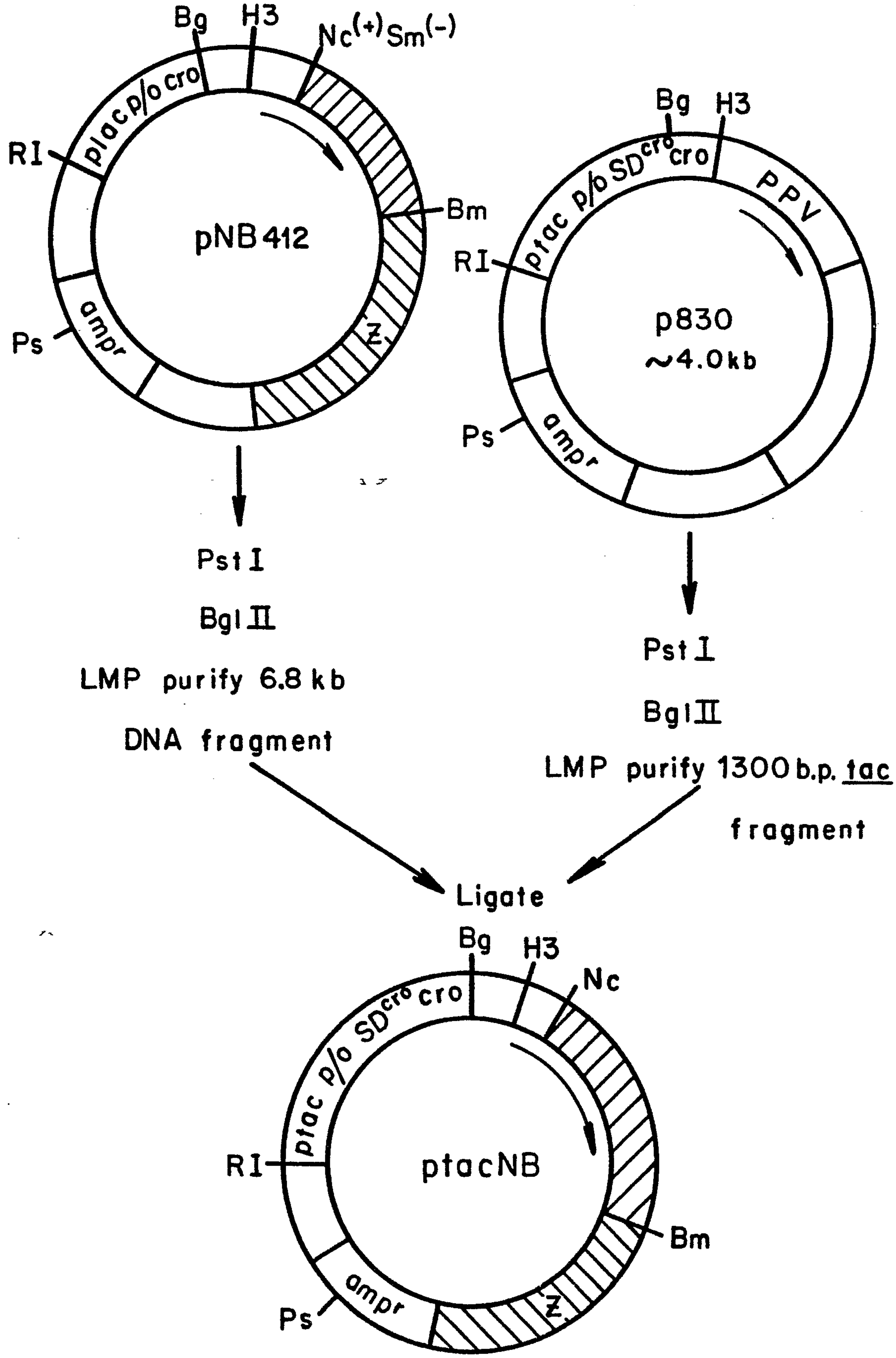
Bg
H3
Nc(+) Sm(-)
plac p/o cro
RI
Bm
pNB412
Ps
ampr
Z
Bg
H3
ptac p/o SDcro cro
PPV
RI
p830
~4.0kb
Ps
ampr
Pst I
Bgl II
LMP purify 6.8 kb
DNA fragment
Pst I
Bgl II
LMP purify 1300 b.p. tac
fragment
Ligate
Bg
H3
Nc
ptac p/o SDcro cro
RI
ptacNB
Bm
ampr
Ps
Z

FIG. 8



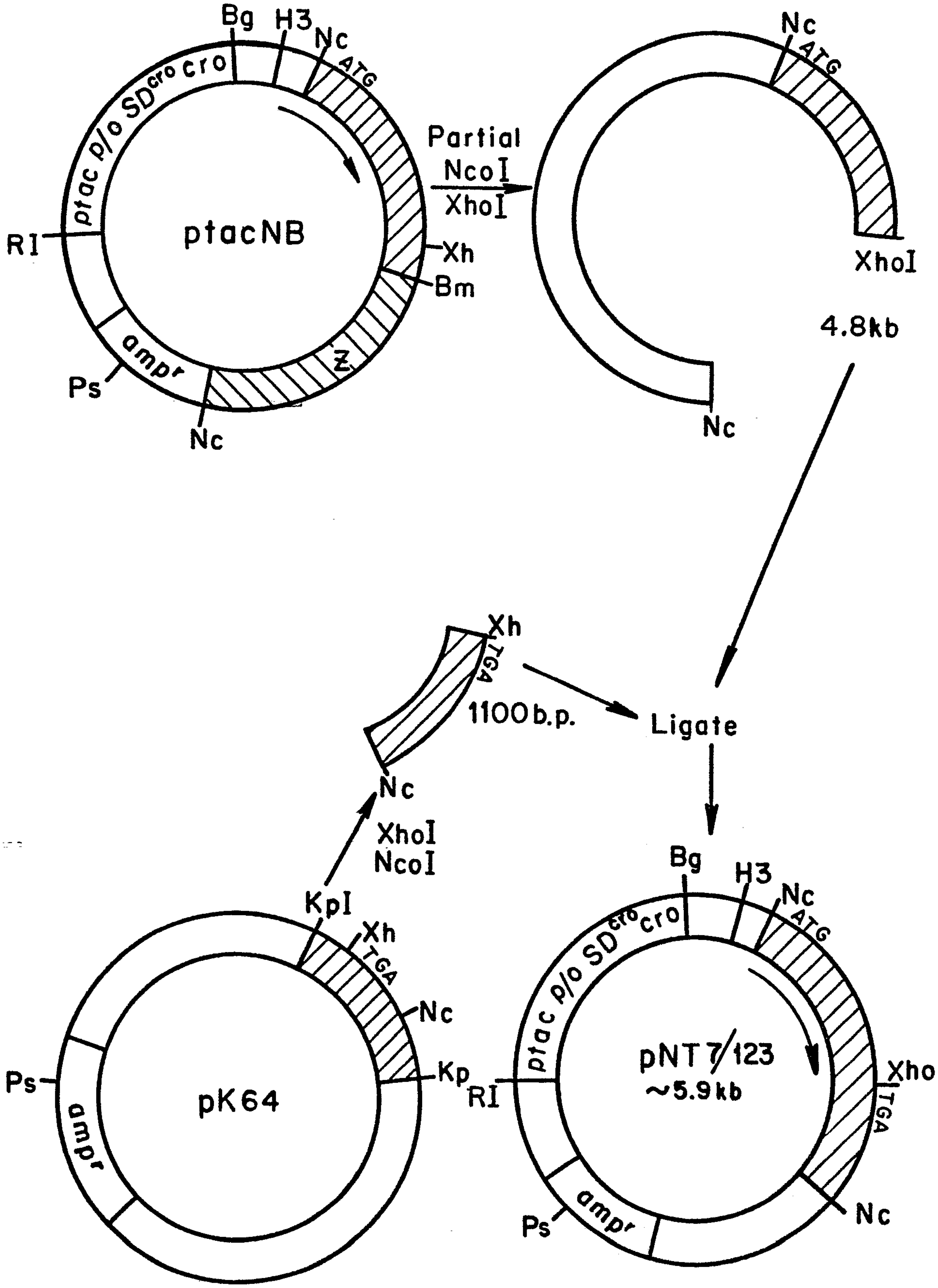

Bg
H3
Nc
ATG
cro
ptac p/o SD$^{cro}$
ptacNB
RI
Xh
Bm
Z
ampr
Ps
Nc
Partial
NcoI
XhoI
Nc
ATG
XhoI
4.8kb
Nc
Xh
TGA
1100b.p.
Ligate
Nc
XhoI
NcoI
KpI
Xh
TGA
Nc
Kp
Ps
ampr
pK64
Bg
H3
Nc
ATG
cro
ptac p/o SD$^{cro}$
RI
pNT 7/123
~5.9kb
Xho
TGA
Nc
ampr
Ps

FIG. 9A



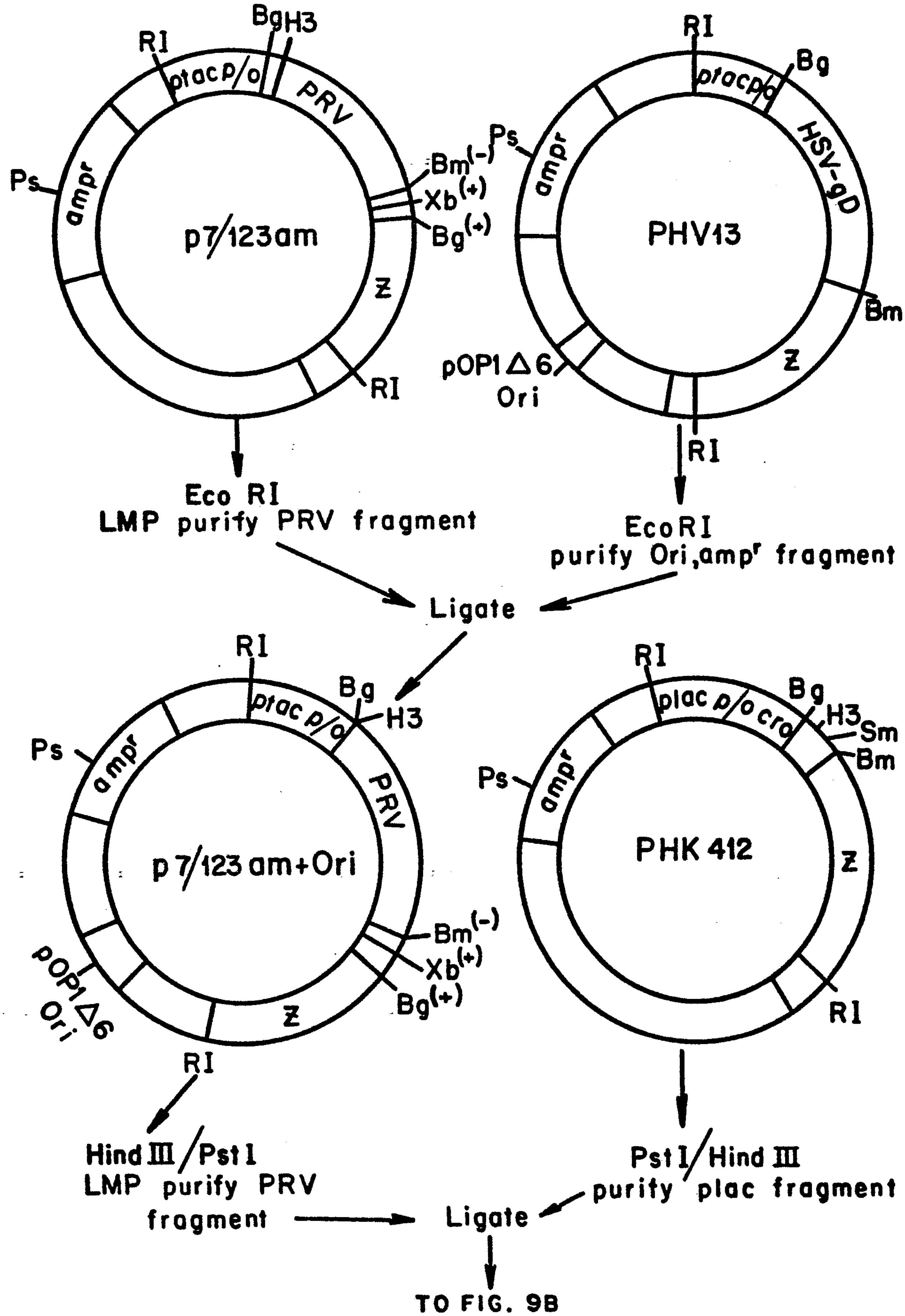
RI
Bg
H3
ptac p/o
PRV
Ps
ampr
p7/123am
Bm(-)
Xb(+)
Bg(+)
Z
RI
Eco RI
LMP purify PRV fragment
RI
Bg
ptac p/o
HSV-gD
Ps
ampr
PHV13
Bm
Z
pOP1 Δ6
Ori
RI
EcoRI
purify Ori,ampr fragment
Ligate
RI
Bg
H3
ptac p/o
PRV
Ps
ampr
p 7/123 am+Ori
Bm(-)
Xb(+)
Bg(+)
Z
pOP1 Δ6
Ori
RI
RI
plac p/o cro
Bg
H3
Sm
Bm
Ps
ampr
PHK 412
Z
RI
Hind III/Pst1
LMP purify PRV
fragment
Pst I/Hind III
purify plac fragment
Ligate
TO FIG. 9B

# FIG. 9B

FROM FIG. 9A

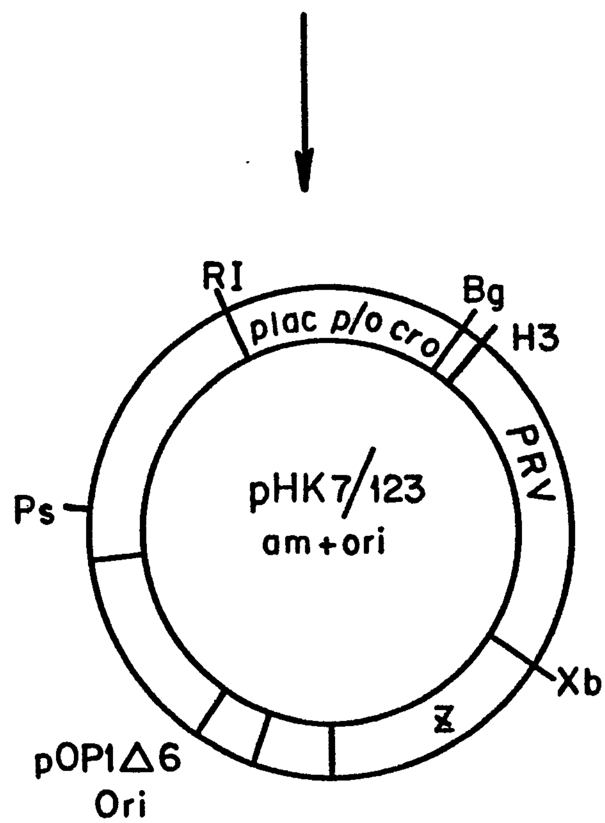

# FIG. 10



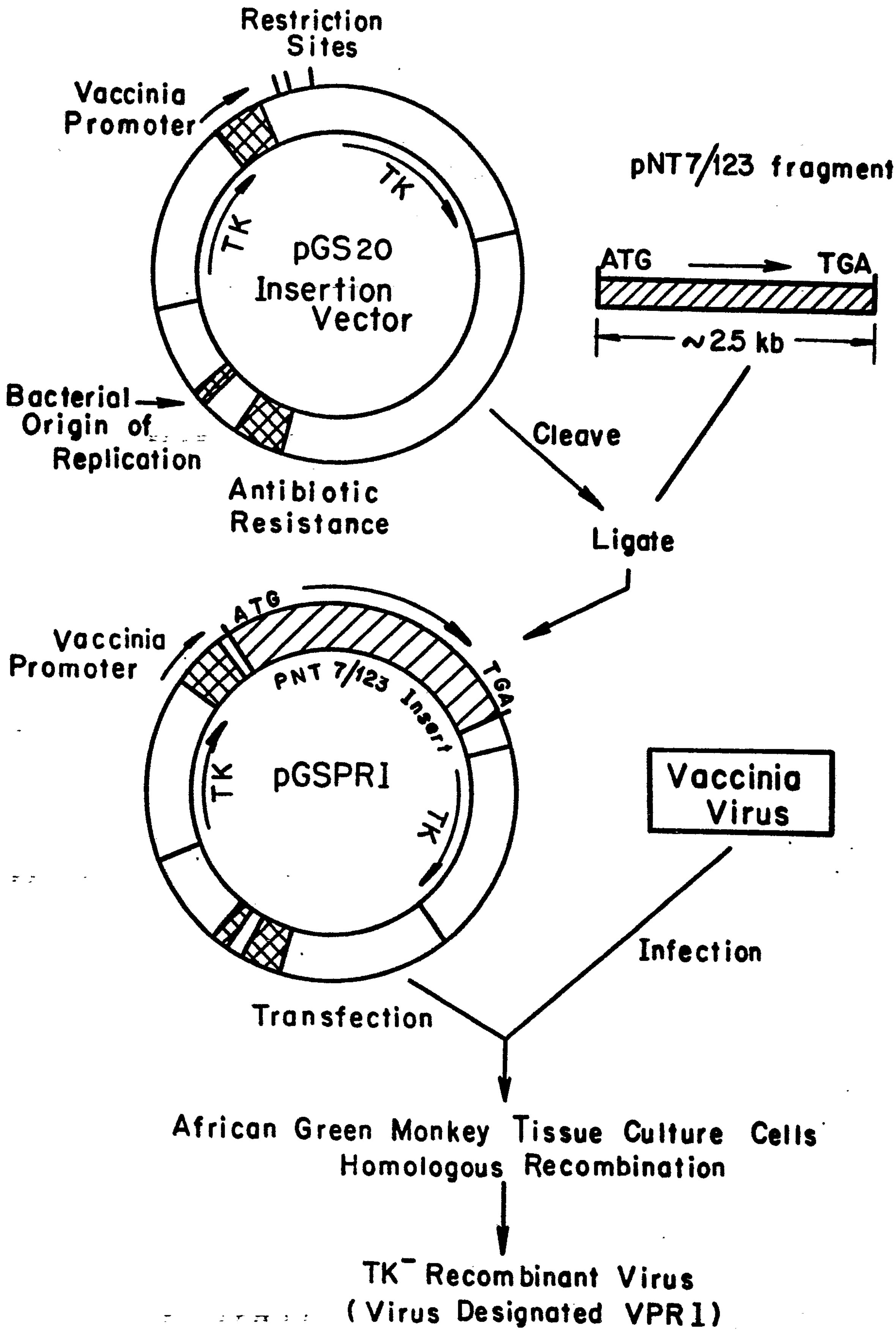
Restriction Sites
Vaccinia Promoter
TK
TK
pGS20 Insertion Vector
Bacterial Origin of Replication
Antibiotic Resistance
pNT7/123 fragment
ATG
TGA
~2.5 kb
Cleave
Ligate
Vaccinia Promoter
ATG
TGA
PNT 7/123 Insert
TK
pGSPRI
TK
Vaccinia Virus
Infection
Transfection
African Green Monkey Tissue Culture Cells
Homologous Recombination
TK⁻ Recombinant Virus
(Virus Designated VPR1)

# FIG. 11A

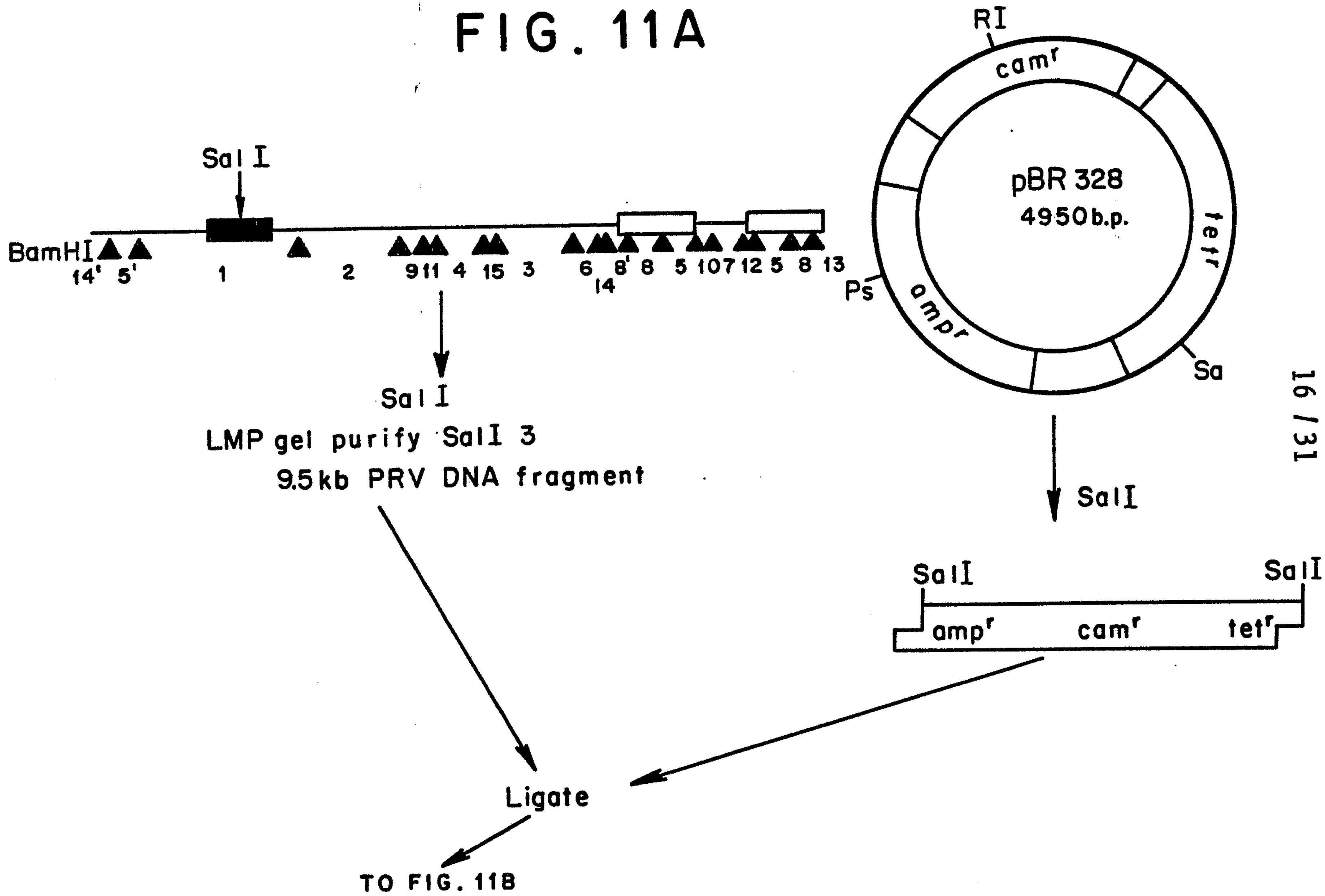
BamHI
14'
5'
Sal I
1
2
9 11
4
15
3
6
14
8'
8
5
10 7
12
5
8
13
Sal I
LMP gel purify SalI 3
9.5kb PRV DNA fragment
RI
camr
pBR 328
4950 b.p.
tetr
Sa
ampr
Ps
SalI
SalI
SalI
ampr
camr
tetr
Ligate
TO FIG. 11B

# FIG. 11B



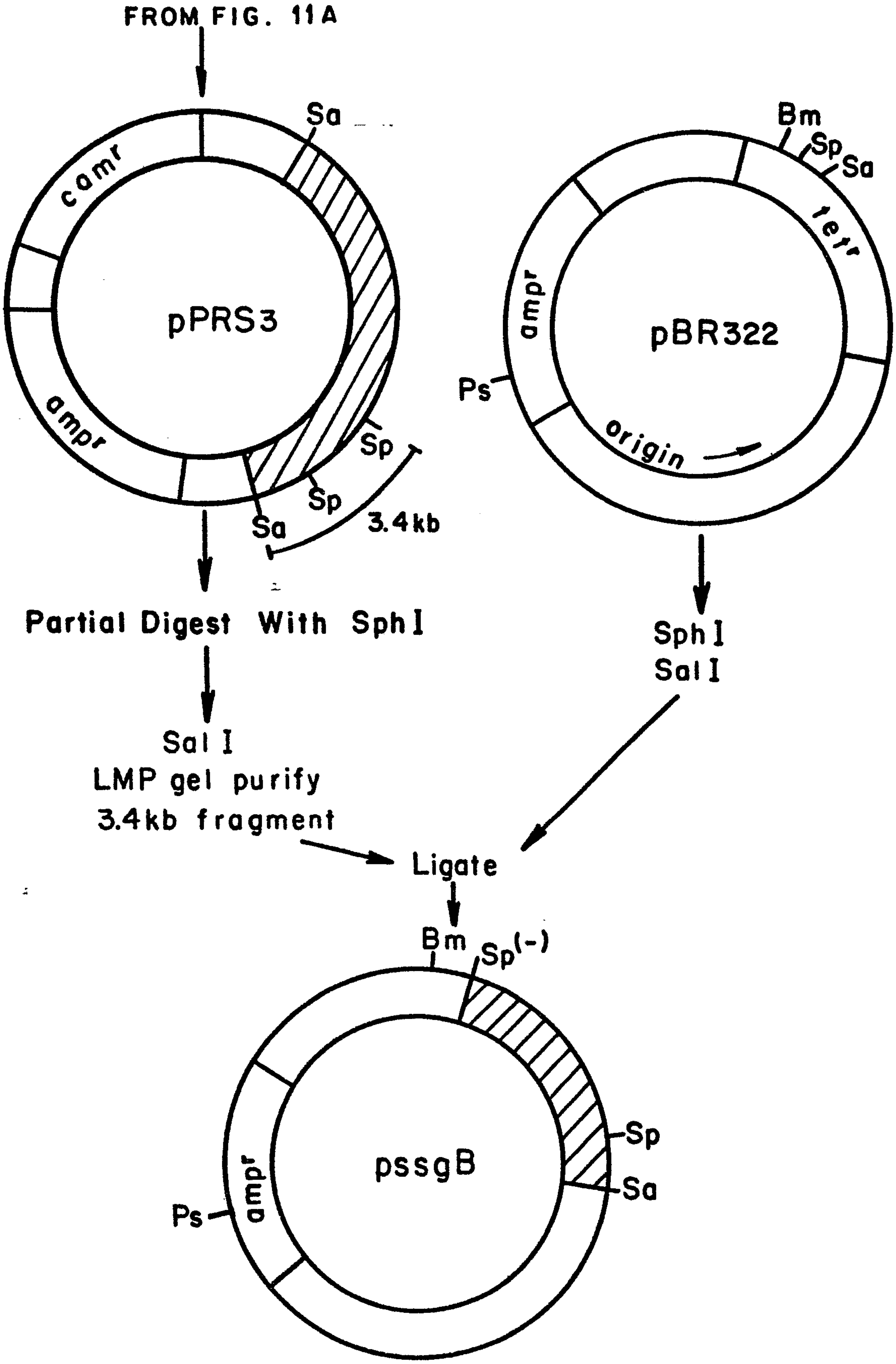
FROM FIG. 11A
pPRS3
Sa
camr
ampr
Sp
Sp
Sa
3.4kb
pBR322
Bm
Sp
Sa
tetr
ampr
Ps
origin
Partial Digest With SphI
SphI
SalI
SalI
LMP gel purify
3.4kb fragment
Ligate
pssgB
Bm
Sp(-)
Sp
Sa
ampr
Ps

FIG. 12

PRV pssgB Restriction Sites (Approximate)

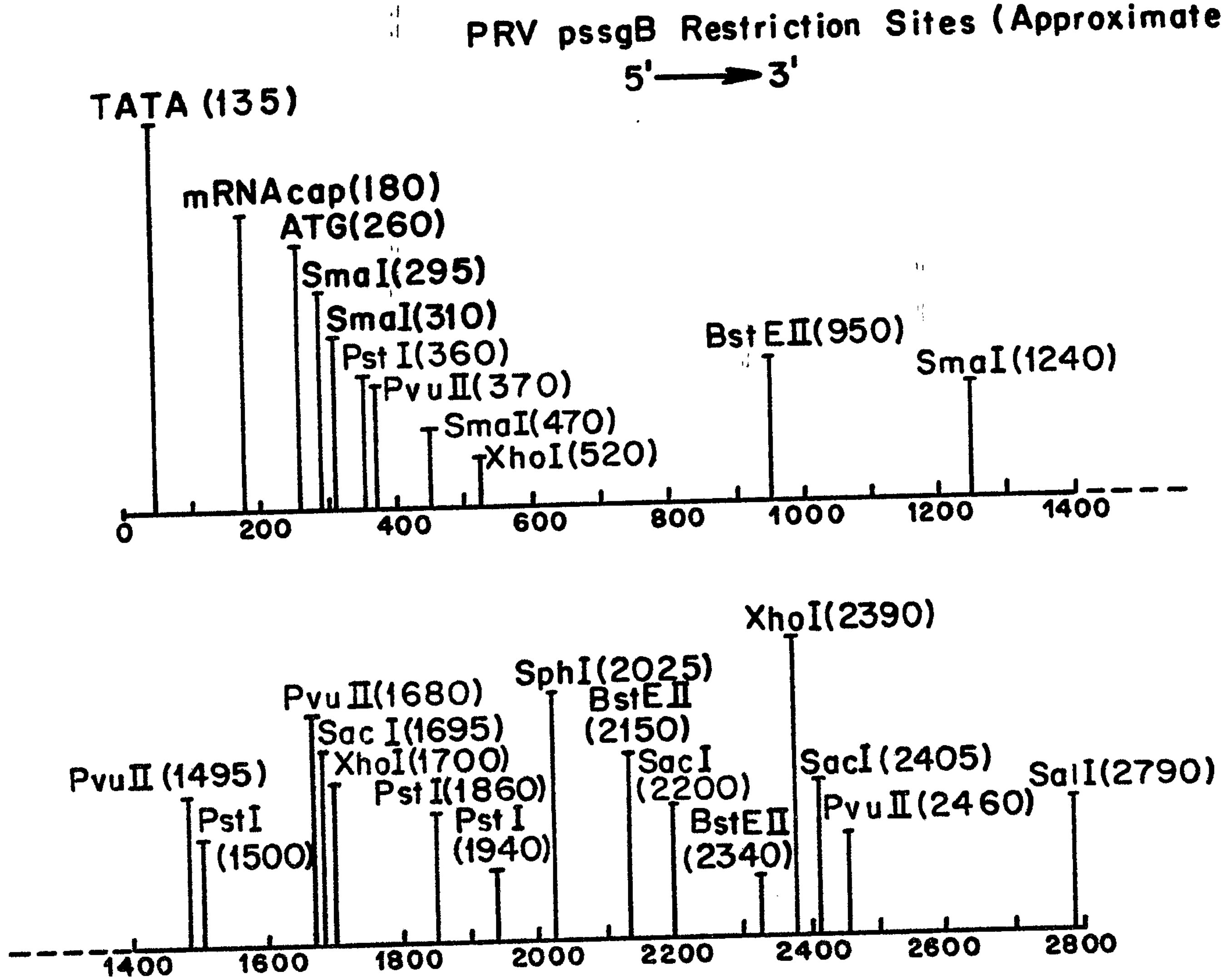

# FIG. 13A



```
                                                   AAC GTG GCC     9

TCC TGC CGC ACC TGA AGG AGG GCT GGC GCG CTT CAT GGT GGC    51

CCG CGA TTG GTG CGT CAG CGA GTT CCG CGG CTT CTA CCG CTT    93

CCA GAC GGC CGG CGT AAC CGC CAC CCA GCG GCA GGC CTG GCG   135

  TATA
ATA TAT CCG CGA GCT GGT GCT GGC GGT TGC AGT CTT CAG GTC   177

CGT CTT CCA CTG CGG GGA CGT CGA GGT CCT CCG CGC GGA TCG   219

CTT CGC CGG ACG CGA CGG GCT GTA CCT GAC CTA CGA GGC GTC   261

                                        SmaI
  ATG CCC GCT GGT GGC GGT CTT TGG CGC GGG CCC CGG GGG CAT   303
1 MET Pro Ala Gly Gly Gly Leu Trp Arg Gly Pro Arg Gly His

       SmaI
   CGG CCC GGG CAC CAC GGC GGT GCT GGC CTC GGA CGT CTT TGG   345
15 Arg Pro Gly His His Gly Gly Ala Gly Leu Gly Arg Leu Trp

                       PstI PvuII
   CCT GCT CCA CAC CAC GCT GCA GCT GCG CGG GGC GCC GTC GCG   387
29 Pro Ala Pro His His Ala Ala Ala Ala Arg Gly Ala Val Ala

   CTA GCG CTG CTG CTG CTG GCG CTC GCC GCG GCC CCG CCG TGC   429
43 Leu Ala Leu Leu Leu Leu Ala Leu Ala Ala Ala Pro Pro Cys

   GGC GCG GCG GCC GTG ACG CGG GCC GCC TCG GCC TCG CCG ACG   471
57 Gly Ala Ala Ala Val Thr Arg Ala Ala Ser Ala Ser Pro Thr

    SmaI
   CCC GGG ACG GGC GCC ACC CCC AAC GAC GTC TCC GCG GAG GCG   513
71 Pro Gly Thr Gly Ala Thr Pro Asn Asp Val Ser Ala Glu Ala

        XhoI
   TCC CTC GAG GAG ATC GAG GCG TTC TCC CCC GGC CCC TCG GAG   555
85 Ser Leu Glu Glu Ile Glu Ala Phe Ser Pro Gly Pro Ser Glu

   GCC CCC GAC GGC GAG TAC GGC GAC CTG GAC GCG CGG ACG GCC   597
99 Ala Pro Asp Gly Glu Tyr Gly Asp Leu Asp Ala Arg Thr Ala
```

# FIG. 13B



GTG CGC GCG GCC GCG ACC GAG CGG GAC CGC TTC TAC GTC TGC 639
113 Val Arg Ala Ala Ala Thr Glu Arg Asp Arg Phe Tyr Val Cys

CCG CCG CCG TCC GGC TCC ACG GTG GTG CGG CTG GAG CCC GAG 681
127 Pro Pro Pro Ser Gly Ser Thr Val Val Arg Leu Glu Pro Glu

CAG GCC TGC CCC GAG TAC TCG CAG GGG CGC AAC TTC ACG GAG 723
141 Gln Ala Cys Pro Glu Tyr Ser Gln Gly Arg Asn Phe Thr Glu

GGG ATC GCC GTG CTC TTC AAG GAG AAC ATC GCC CCG CAC AAG 765
155 Gly Ile Ala Val Leu Phe Lys Glu Asn Ile Ala Pro His Lys

TTC AAG GCC CAC ATC TAC TAC AAG AAC GTC ATC GTC ACG ACC 807
169 Phe Lys Ala His Ile Tyr Tyr Lys Asn Val Ile Val Thr Thr

GTG TGG TCC GGG AGC ACG TAC GCG GCC ATC ACG AAC CGC TTC 849
183 Val Trp Ser Gly Ser Thr Tyr Ala Ala Ile Thr Asn Arg Phe

ACA GAC CGC GTG CCC GTC CCC GTG CAG GAG ATC ACG GAC GTG 891
197 Thr Asp Arg Val Pro Val Pro Val Gln Glu Ile Thr Asp Val

ATC GAC CGC CGC GGC AAG TGC GTC TCC AAG GCC GAG TAC GTG 933
211 Ile Asp Arg Arg Gly Lys Cys Val Ser Lys Ala Glu Tyr Val

BstEII
CGC AAC AAC CAC AAG GTG ACC GCC TTC GAC CGC GAC GAG AAC 975
225 Arg Asn Asn His Lys Val Thr Ala Phe Asp Arg Asp Glu Asn

CCC GTC GAG GTG GAC CTG CGC CCC TCG CGC CTG AAC GCG CTC 1017
239 Pro Val Glu Val Asp Leu Arg Pro Ser Arg Leu Asn Ala Leu

GGC ACC CGC GGC TGG CAC ACC ACC AAC GAC ACC TAC ACC AAG 1059
253 Gly Thr Arg Gly Trp His Thr Thr Asn Asp Thr Tyr Thr Lys

ATC GGC GCC GCG GGC TTC TAC CAC ACG GGC ACC TCC GTC AAC 1101
267 Ile Gly Ala Ala Gly Phe Tyr His Thr Gly Thr Ser Val Asn

TGC ATC GTC GAG GAG GTG GAG GCG CGC TCC GTG TAC CCC TAC 1143
281 Cys Ile Val Glu Glu Val Glu Ala Arg Ser Val Tyr Pro Tyr

# FIG. 13C



```
    GAC TCC TTC GCC CTG TCC ACG GGG GAC ATT GTG TAC ATG TCC 1185
295 Asp Ser Phe Ala Leu Ser Thr Gly Asp Ile Val Tyr Met Ser

    CCC TTC TAC GGC CTG CGC GAG GGG GCC CAC GGG GAG CAC ATC 1227
309 Pro Phe Tyr Gly Leu Arg Glu Gly Ala His Gly Glu His Ile

                SmaI
    GGC TAC GCG CCC GGG CGC TTC CAG CAG GTG GAG CAC TAC TAC 1269
323 Gly Tyr Ala Pro Gly Arg Phe Gln Gln Val Glu His Tyr Tyr

    CCC ATC GAC CTG GAC TCG CGC CTC CGC GCC TCC GAG AGC GTG 1311
337 Pro Ile Asp Leu Asp Ser Arg Leu Arg Ala Ser Glu Ser Val

    ACG CGC AAC TTT CTA CGC ACG CCG CAC TTC ACG GTG GCC TGG 1353
351 Thr Arg Asn Phe Leu Arg Thr Pro His Phe Thr Val Ala Trp

    GAC TGG GCC CCC AAG ACG CGG CGC GTG TGC AGC CTG GCC AAG 1395
365 Asp Trp Ala Pro Lys Thr Arg Arg Val Cys Ser Leu Ala Lys

    TGG CGC GAG GCC GAG GAG ATG ACC CGC GAC GAG ACG CGC GAC 1437
379 Trp Arg Glu Ala Glu Glu Met Thr Arg Asp Glu Thr Arg Asp

    GGC TCC TTC CGC TTC ACG TCG CGG GCC CTG GGC GCC TCC TTC 1479
393 Gly Ser Phe Arg Phe Thr Ser Arg Ala Leu Gly Ala Ser Phe

                        PvuII       PstI
    GTC AGC GAC GTC ACG CAG CTG GAC CTG CAG CGC GTG CAC CTG 1521
407 Val Ser Asp Val Thr Gln Leu Asp Leu Gln Arg Val His Leu

    GGC GAC TGC GTC CTC CGC GAG GCC TCG GAG GCC ATC GAC GCC 1563
421 Gly Asp Cys Val Leu Arg Glu Ala Ser Glu Ala Ile Asp Ala

    ATC TAC CGG CGG CGC TAC AAC AGC ACG CAC GTG CTG GCC GGC 1605
435 Ile Tyr Arg Arg Arg Tyr Asn Ser Thr His Val Leu Ala Gly

    GAC AGG CCC GAG GTG TAC CTC GCC CGC GGG GGC TTC GTG GTG 1647
449 Asp Arg Pro Glu Val Tyr Leu Ala Arg Gly Gly Phe Val Val

                                                PvuII
    GCC TTC CGC CCG CTG ATC TCG AAC GAG CTG GCG CAG CTG TAC 1689
463 Ala Phe Arg Pro Leu Ile Ser Asn Glu Leu Ala Gln Leu Tyr
```

# FIG. 13D



```
                  SacI XhoI
    GCG CGC GAG CTC GAG CGC CTC GGC CTC GCC GGC GTC GTG GGC 1731
477 Ala Arg Glu Leu Glu Arg Leu Gly Leu Ala Gly Val Val Gly

    CCC GCG GCC CCC GCG GCC GCC CGT CGG GCC CGG CGC TCC CCC 1773
491 Pro Ala Ala Pro Ala Ala Ala Arg Arg Ala Arg Arg Ser Pro

    GGC CCG GCG GGG ACG CCC GAG CCG CCG GCC GTC AAC GGC ACG 1815
505 Gly Pro Ala Gly Thr Pro Glu Pro Pro Ala Val Asn Gly Thr

    GGG CAC CTG CGC ATC ACC ACG GGC TCG GCG GAG TTT GCG CGC 1857
519 Gly His Leu Arg Ile Thr Thr Gly Ser Ala Glu Phe Ala Arg

    PstI
    CTG CAG TTC ACC TAC GAC CAC ATC CAG GCG CAC GTG AAC GAC 1899
533 Leu Gln Phe Thr Tyr Asp His Ile Gln Ala His Val Asn Asp

                                                PstI
    ATG CTG GGC CGC ATC GCG GCC GCC TGG TGC GAG CTG CAG AAC 1941
547 Met Leu Gly Arg Ile Ala Ala Ala Trp Cys Glu Leu Gln Asn

    AAG GAC CGC ACC CTG TGG AGC GAG ATG TCG CGC CTG AAC CCC 1983
561 Lys Asp Arg Thr Leu Trp Ser Glu Met Ser Arg Leu Asn Pro

     SphI
    AGC GCC GTG GCC ACG GCC GCG CTC GGC CAG CGC GTC TCG GCG 2025
575 Ser Ala Val Ala Thr Ala Ala Leu Gly Gln Arg Val Ser Ala

    CGC ATG CTC GGC GAC GTG ATG GCC ATC TCG CGG TGC GTG GAG 2067
589 Arg Met Leu Gly Asp Val Met Ala Ile Ser Arg Cys Val Glu

    GTG CGC GGC GGC GTG TAC GTG CAG AAC TCC ATG CGC GTG CCC 2109
603 Val Arg Gly Gly Val Tyr Val Gln Asn Ser Met Arg Val Pro

                                                BstEII
    GGC GAG CGC GGC ACG TGC TAC AGC CGC CCG CTG GTC ACC TTC 2151
617 Gly Glu Arg Gly Thr Cys Tyr Ser Arg Pro Leu Val Thr Phe

    GAG CAC AAC GGC ACG GGC GTG ATC GAG GGC CAG CTC GGC GAC 2193
631 Glu His Asn Gly Thr Gly Val Ile Glu Gly Gln Leu Gly Asp

            SacI
    GAC AAC GAG CTC CTC ATC TCG CGC GAC CTC ATC GAG CCC TGC 2235
645 Asp Asn Glu Leu Leu Ile Ser Arg Asp Leu Ile Glu Pro Cys
```

# FIG. 13E



```
    ACC GGC AAC CAC CGG CGC TAC TTT AAG CTG GGG AGC GGG TAC 2277
659 Thr Gly Asn His Arg Arg Tyr Phe Lys Leu Gly Ser Gly Tyr

    GTG TAC TAC GAG GAC TAC AAC TAC GTG CGC ATG GTG GAG GTG 2319
673 Val Tyr Tyr Glu Asp Tyr Asn Tyr Val Arg Met Val Glu Val

                                  BstEII
    CCC GAG ACG ATC AGC ACG CGG GTG ACC CTG AAC CTG ACG CTG 2361
687 Pro Glu Thr Ile Ser Thr Arg Val Thr Leu Asn Leu Thr Leu

                                     XhoI
    CTG GAG GAC CGC GAG TTC CTG CCC CTC GAG GTG TAC ACG CGC 2403
701 Leu Glu Asp Arg Glu Phe Leu Pro Leu Glu Val Tyr Thr Arg

       SacI
    GAG GAG CTC GCC GAC ACG GGC CTC CTG GAC TAC AGC GAG ATC 2445
715 Glu Glu Leu Ala Asp Thr Gly Leu Leu Asp Tyr Ser Glu Ile

                    PvuII
    CAG CGC CGC AAC CAG CTG CAC GCG CTC AAG TTC TAC GAC ATC 2487
729 Gln Arg Arg Asn Gln Leu His Ala Leu Lys Phe Tyr Asp Ile

    GAC CGC GTG GTC AAG GTG GAC CAC AAC GTG GTG CTG CTG CGC 2529
743 Asp Arg Val Val Lys Val Asp His Asn Val Val Leu Leu Arg

    GGC ATC GCC AAC TTC TTC CAG GGC CTC GGC GAC GTG GGC GCC 2571
757 Gly Ile Ala Asn Phe Phe Gln Gly Leu Gly Asp Val Gly Ala

    GCC GTC GGC AAG GTG GTC CTG GGT GCC ACG GGG GCC GTG ATC 2613
771 Ala Val Gly Lys Val Val Leu Gly Ala Thr Gly Ala Val Ile

    TCG GCC GTC GGC GGC ATG GTG TCC TTC CTG TCC AAC CCC TTC 2655
785 Ser Ala Val Gly Gly Met Val Ser Phe Leu Ser Asn Pro Phe

    GGG GCG CTC GCC ATC GGG CTG CTG GTG CTG GCC GGC CTG GTC 2697
799 Gly Ala Leu Ala Ile Gly Leu Leu Val Leu Ala Gly Leu Val

    GCG GCC TTC CTG GCC TAC CGG CAC ATC TCG CGC CTG CGC CGC 2739
813 Ala Ala Phe Leu Ala Tyr Arg His Ile Ser Arg Leu Arg Arg

    AAC CCC ATG AAG GCC CTG TAC CCC GTC ACG ACG AAG ACG CTC 2781
827 Asn Pro Met Lys Ala Leu Tyr Pro Val Thr Thr Lys Thr Leu

    AAG GAG GAC GGC GTC 2796
845 Lys Glu Asp Gly Val
```

# FIG. 14A



```
                              10                  20
                        MHQGAPSWGRRWF-----------VVWALLGLTL------GVL
                         :  ::   :: :         :  ::: : :     :
MPAGGGLWRGPRGHRPGHHGGAGL-GRLWPAPHHAAAARGAVALALLLLALAAAPPCGAA
        10        20        30        40        50

    30        40        50        60        70        80
-VASAAPSSPGTPGVARDPGGERGPCHSGAAALGAAPTGDPKPKKNKKPKNPTPPRPAGD
 :  ::  :: :::    :        :  :  :    : :          :   ::
AVTRAASASP-TPGTGATPNDV-----SAEASLEEIEAFSPGPSEA-------PDGEYGD
60         70        80             90                  100

    90       100       110       120       130       140
NATVAAGHATLREHLRDIKAENTDANFYVCPPPTGATVVQFEQPRRCPTRPEGQNYTEGI
    :   :   :  :          ::::::: : :::  :     ::   : : ::::
LDARTAVRAAATERDR----------FYVCPPPSGSTVVRLEPEQACPEYSQGRNFTEGI
   110       120                    130       140       150

   150       160       170       180       190       200
AVVFKENIAPYKFKATMYYKDVTVSQVWFGHRYSQFMGIFEDRAPVPFEEVIDKINAKGV
:: ::::::: ::::  ::: : :  :: :  :      : :: :::  :  :  :   :
AVLFKENIAPHKFKAHIYYKNVIVTTVWSGSTYAAITNRFTDRVPVPVQEITDVIDRRGK
   160       170       180       190       200       210

   210       220       230       240       250       260
CRSTAKYVRNNLETTAFHRDDHETDMELKPANAATRTSRGWHTTDLKYNPSRVEAFHRYG
: : : :::::    ::: ::       : :       ::::::   :          : :
CVSKAEYVRNNHKVTAFDRDENPVEVDLRPSRLNALGTRGWHTTNDTYTKIGAAGFYHTG
   220       230       240       250       260       270

   270       280       290       300       310       320
TTVNCIVEEVDARSVYPYDEFVLATGDFVYMSPFYGYREGSHTEHTTYAADRFKQVDGFY
: :::::::: :::::::: : : ::: :::::::: ::: : ::  ::  :: ::   :
TSVNCIVEEVEARSVYPYDSFALSTGDIVYMSPFYGLREGAHGEHIGYAPGRFQQVEHYY
   280       290       300       310       320       330

   330       340       350       360       370       380
ARDLTTKARATAPTTRNLLTTPKFTVAWDWVPKRPSVCTMTKWQEVDEMLRSEYG-GSFR
 ::     ::      ::: : :: ::::::: ::    ::    :: :  :: : :  ::::
PIDLDSRLRASESVTRNFLRTPHFTVAWDWAPKTRRVCSLAKWREAEEMTRDETRDGSFR
   340       350       360       370       380       390

   390       400       410       420       430       440
FSSDAISTTFTTNLTEYPLSRVDLGDCIGKDARDAMDRIFARRYNATHIKVGQ-PQYYLA
: : :    :    :   : :: ::::   :  : : :  :::: ::   :   :  :::
FTSRALGASFVSDVTQLDLQRVHLGDCVLREASEAIDAIYRRRYNSTHVLAGDRPEVYLA
   400       410       420       430       440       450
```

# FIG. 14B



```
     450       460       470                       480
NGGFLIAYQPLLSNTLAELYVREHLREQS----------------RKP-PNPTPPPPGAS
::: :  :: :: :: :: :: :                      : : :  :: :: :
RGGFVVAFRPLISNELAQLYARELERLGLAGVVGPAAPAAARRARRSPGPAGTPEPP-AV
   460       470       480       490       500       510

   490       500       510       520       530       540
ANASVERIKTTSSIEFARLQFTYNHIQRHVNDMLGRVAIAWCELQNHELTLWNEARKLNP
      :: :: : ::::::::: ::: :::::::: : :::::::   ::: :    :::
NGTGHLRI-TTGSAEFARLQFTYDHIQAHVNDMLGRIAAAWCELQNKDRTLWSEMSRLNP
    520       530       540       550       560       570

   550       560       570       580       590       600
NAIASVTVGRRVSARMLGDVMAVSTCVPVAADNVIVQNSMRISSRPGACYSRPLVSFRYE
 : :      : ::::::::::::: : :: :     : ::::::     : ::::::: :  :
SAVATAALGQRVSARMLGDVMAISRCVEVRGG-VYVQNSMRVPGERGTCYSRPLVTF--E
    580       590       600        610       620       630

   610       620       630       640       650       660
DQGPLV-EGQLGENNELRLTRDAIEPCTVGHRRYFTFGGGYVYFEEYAYSHQLSRADIT-
  :  : :::::  :::   :: :::::  :::::  : :::: : : :      :
HNGTGVIEGQLGDDNELLISRDLIEPCTGNHRRYFKLGSGYVYYEDYNYV----RMVEVP
        640       650       660       670       680

      670       680       690       700       710       720
-TVSTFIDLNITMLEDHEFVPLEVYTRHEIKDSGLLDYTEVQRRNQLHDLRFADIDTVIH
 : ::   :: : ::: :: ::::::: :   : ::::: : ::::::: : : ::: :
ETISTRVTLNLTLLEDREFLPLEVYTREELADTGLLDYSEIQRRNQLHALKFYDIDRVVK
  690       700       710       720       730       740

      730       740       750       760       770       780
ADANAAMFAGLGAFFEGMGDLGRAVGKVVMGLVGGVVSAVSGVSSFMSNPFGALAVGLLV
: :       :   :: : :: : :::::: :  : : ::: :  : :::::::: ::::
VDHNVVLLRGIANFFQGLGDVGAAVGKVVLGATGAVISAVGGMVSFLSNPFGALAIGLLV
  750       760       770       780       790       800

      790       800       810       820       830       840
LAGLAAAFFAFRYVMRLQSNPMKALYPLTTKELKNPTNPDASGEGEEGGDFDEAKLAEAR
:::: ::: : :    ::  :::::::: ::: ::                 : :
LAGLVAAFLAYRHISRLRRNPMKALYPVTTKTLK------------EDGV
  810       820       830       840

      850       860       870       880       890       900
EMIRYMALVSAMERTEHKAKKKGTSRLLSAKVTDMVMRKRRNTNYTQVPNKDGDADEDDL
```

HSV-1 gB

PRV gII

FIG. 15A

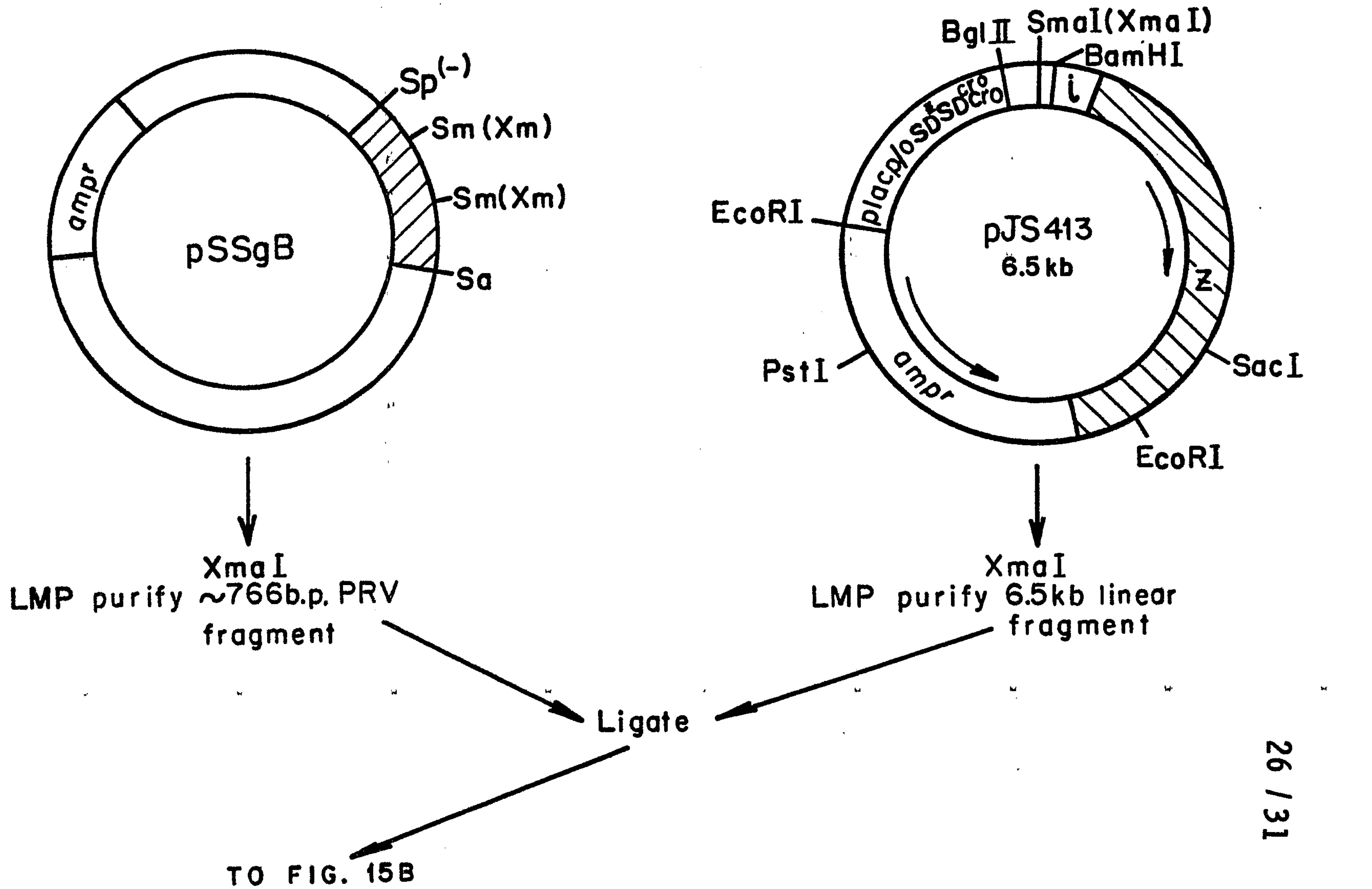
pSSgB
ampr
Sp(-)
Sm (Xm)
Sm(Xm)
Sa
Xma I
LMP purify ~766b.p. PRV fragment
pJS413
6.5 kb
BglII
SmaI(XmaI)
BamHI
i
placp/oSDSDcro
EcoRI
PstI
ampr
z
SacI
EcoRI
XmaI
LMP purify 6.5kb linear fragment
Ligate
TO FIG. 15B

# FIG. 15B



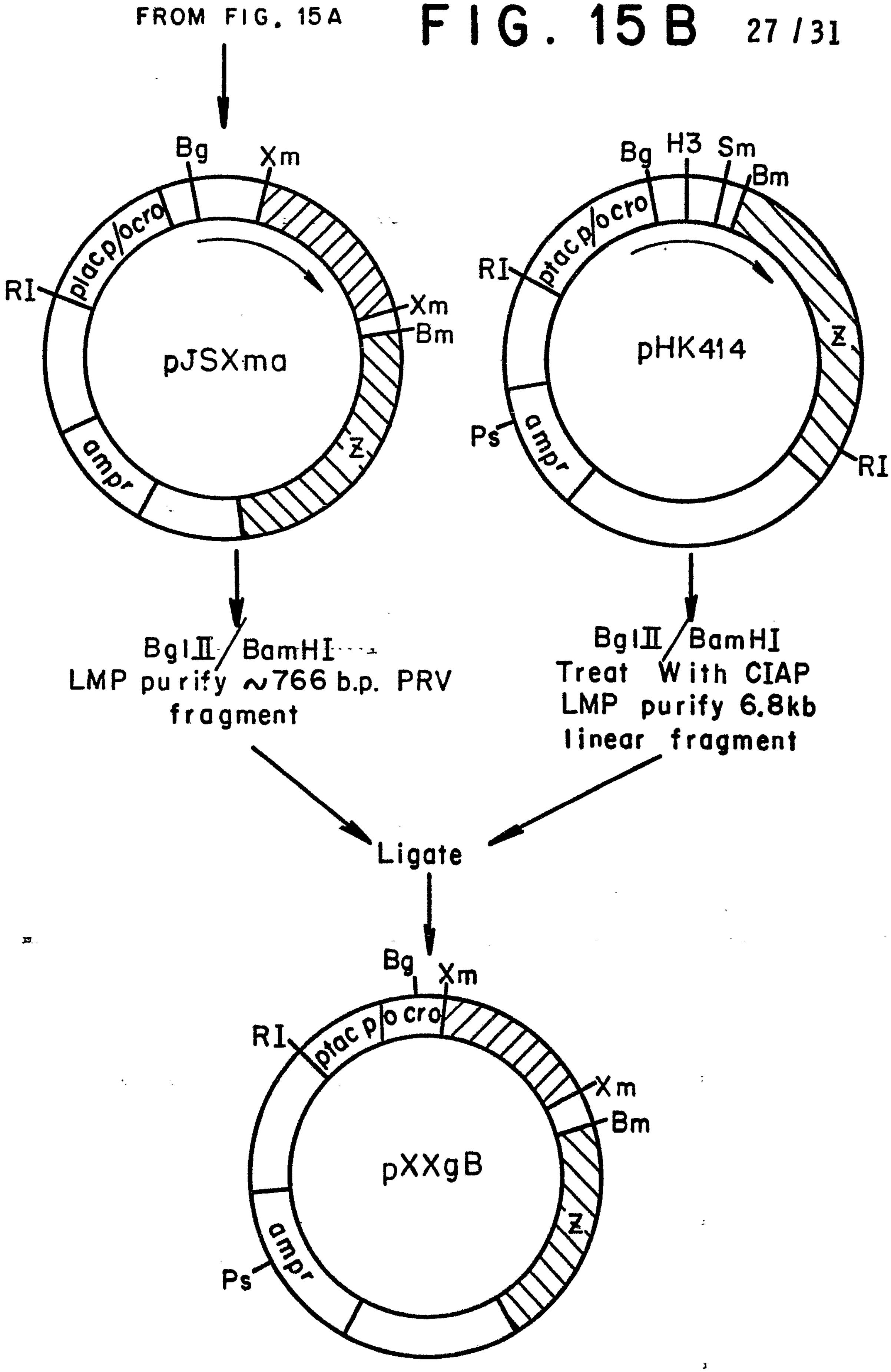
FROM FIG. 15A
Bg
Xm
plac p/o cro
RI
Xm
Bm
pJSXma
Z
ampr
Bg
H3
Sm
Bm
RI
ptac p/o cro
pHK414
Z
Ps
ampr
RI
BglII/BamHI
LMP purify ~766 b.p. PRV fragment
BglII/BamHI
Treat With CIAP
LMP purify 6.8kb linear fragment
Ligate
Bg
Xm
RI
ptac p o cro
Xm
Bm
pXXgB
Z
Ps
ampr

FIG. 16A

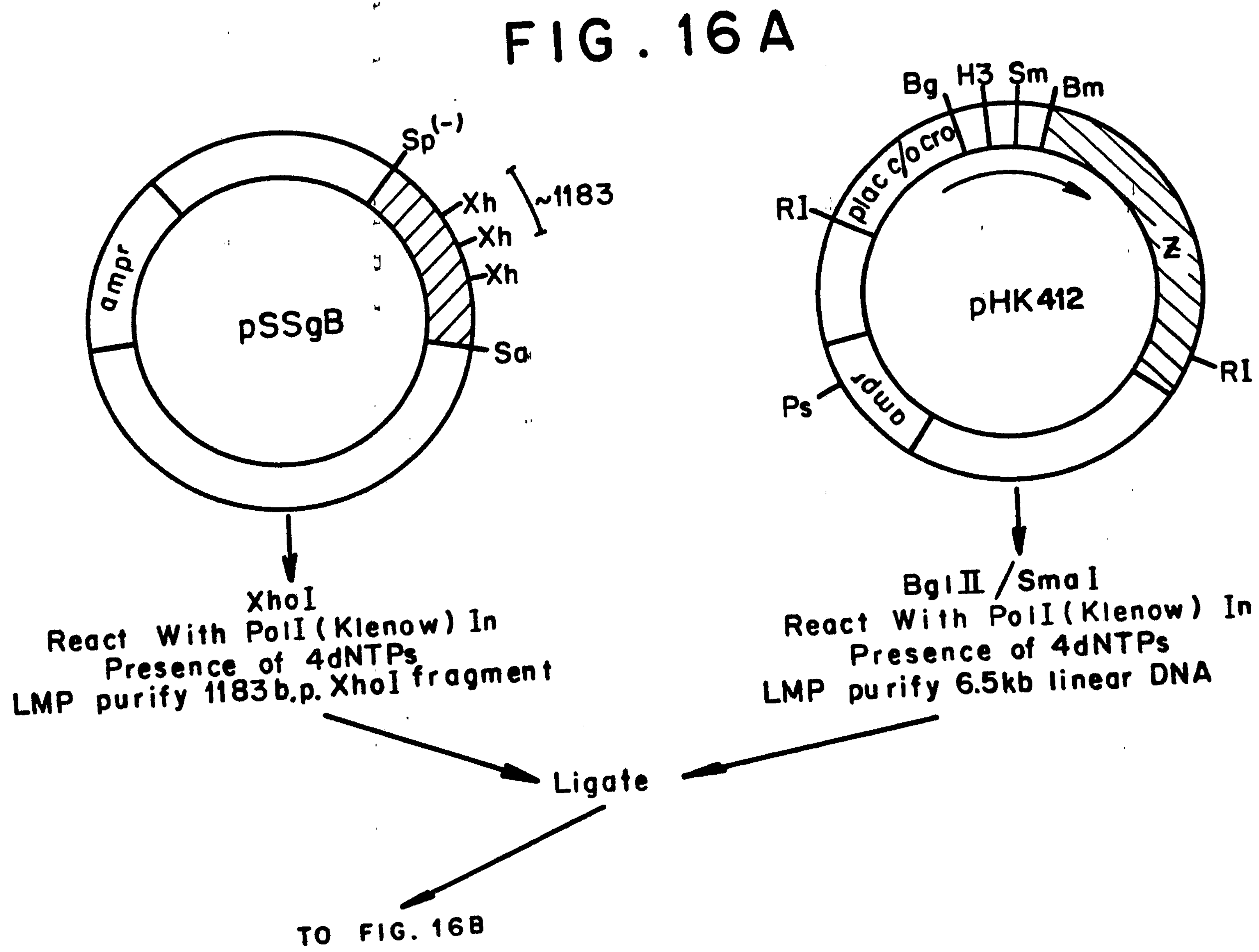
pSSgB
ampr
Sp(-)
Xh
Xh
Xh
Sa
~1183
pHK412
plac c/o cro
Bg
H3
Sm
Bm
Z
RI
RI
Ps
ampr
XhoI
React With PolI (Klenow) In
Presence of 4dNTPs
LMP purify 1183 b.p. XhoI fragment
BglII / SmaI
React With PolI (Klenow) In
Presence of 4dNTPs
LMP purify 6.5kb linear DNA
Ligate
TO FIG. 16B

FROM FIG. 16A

# FIG. 16B



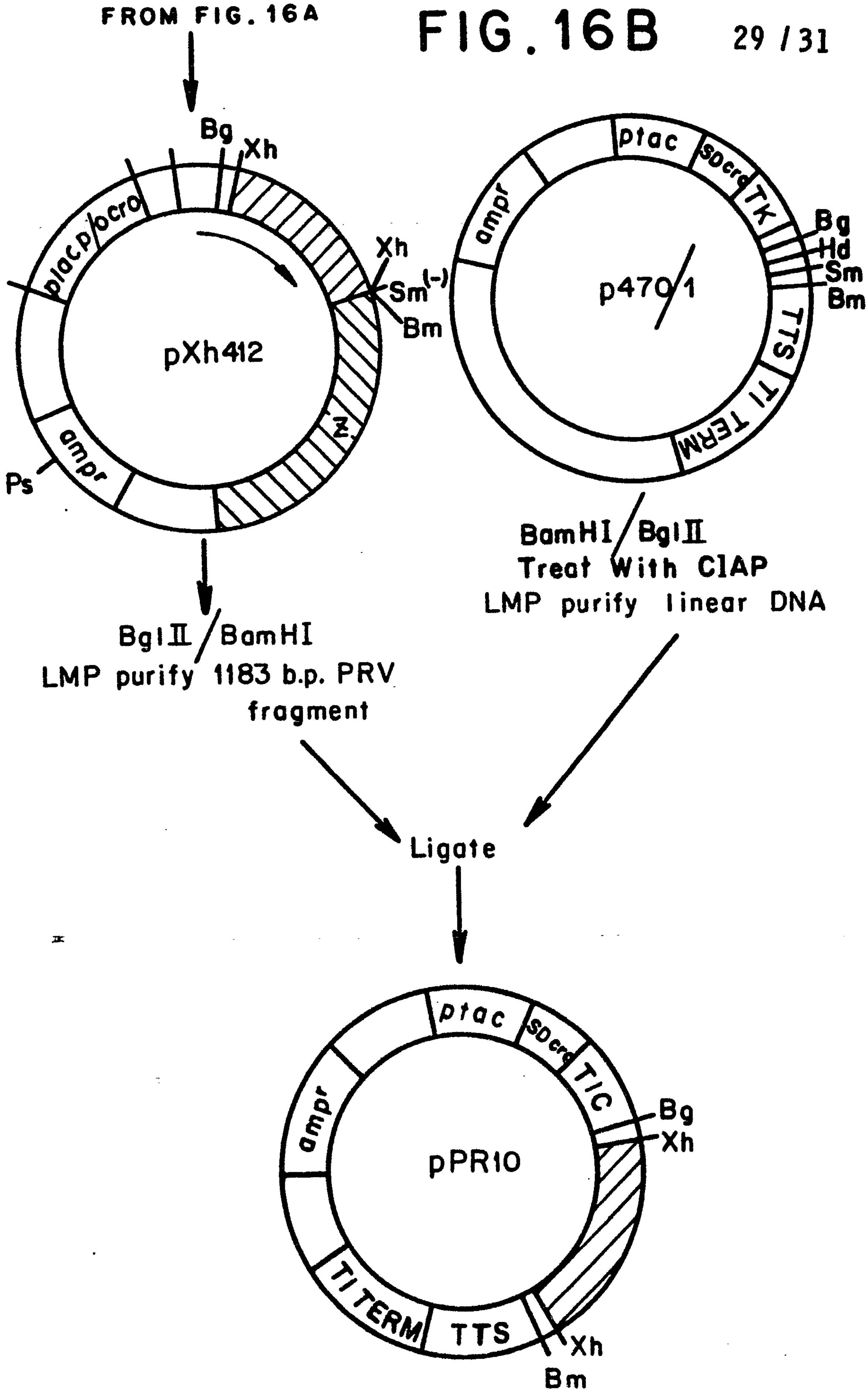

FIG. 17A

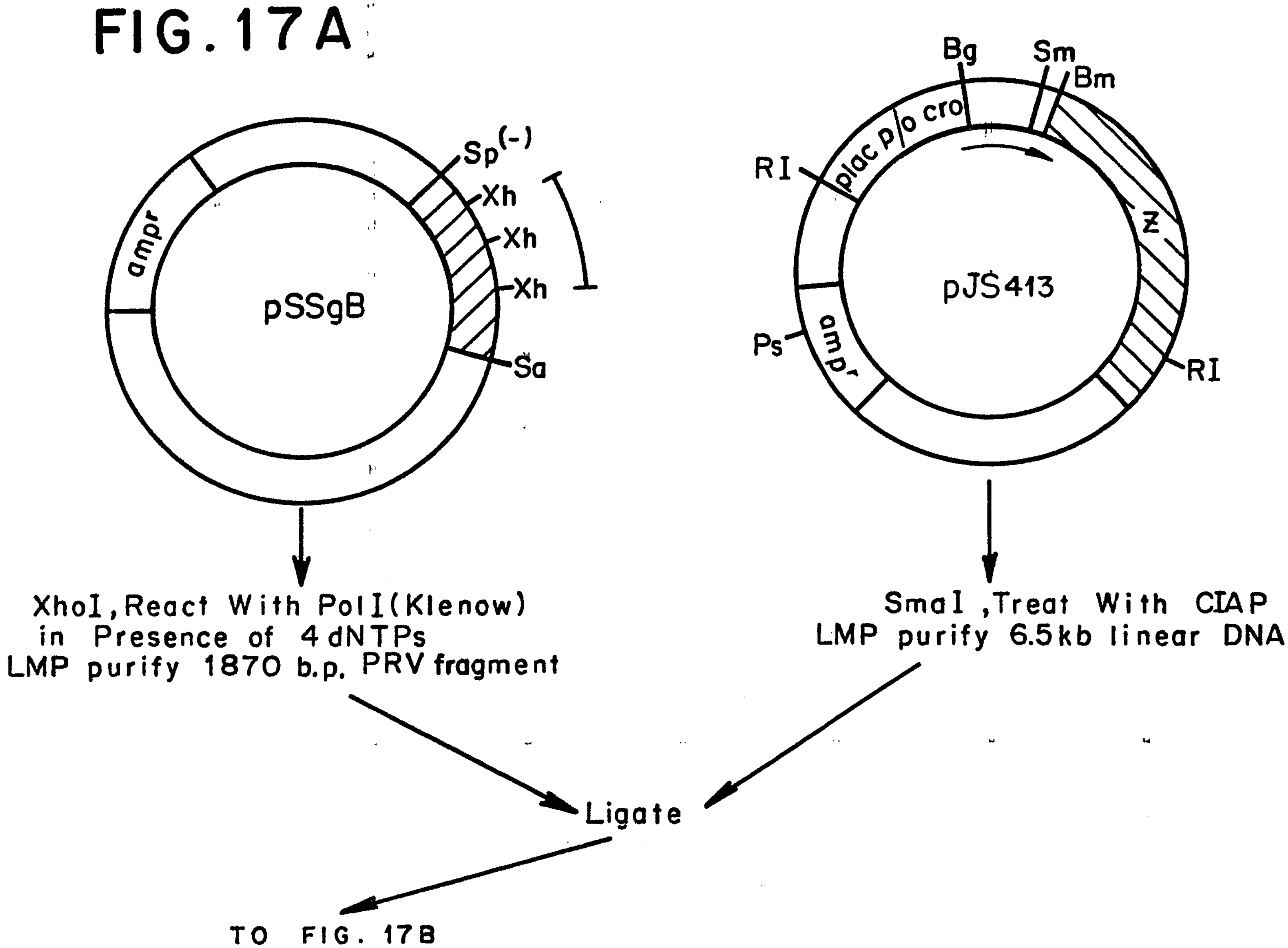
pSSgB
ampr
Sp(-)
Xh
Xh
Xh
Sa
pJS413
Bg
Sm
Bm
RI
plac p/o cro
Z
RI
Ps
ampr
XhoI, React With PolI (Klenow)
in Presence of 4 dNTPs
LMP purify 1870 b.p. PRV fragment
SmaI, Treat With CIAP
LMP purify 6.5kb linear DNA
Ligate
TO FIG. 17B

FROM FIG.17A

# FIG. 17B



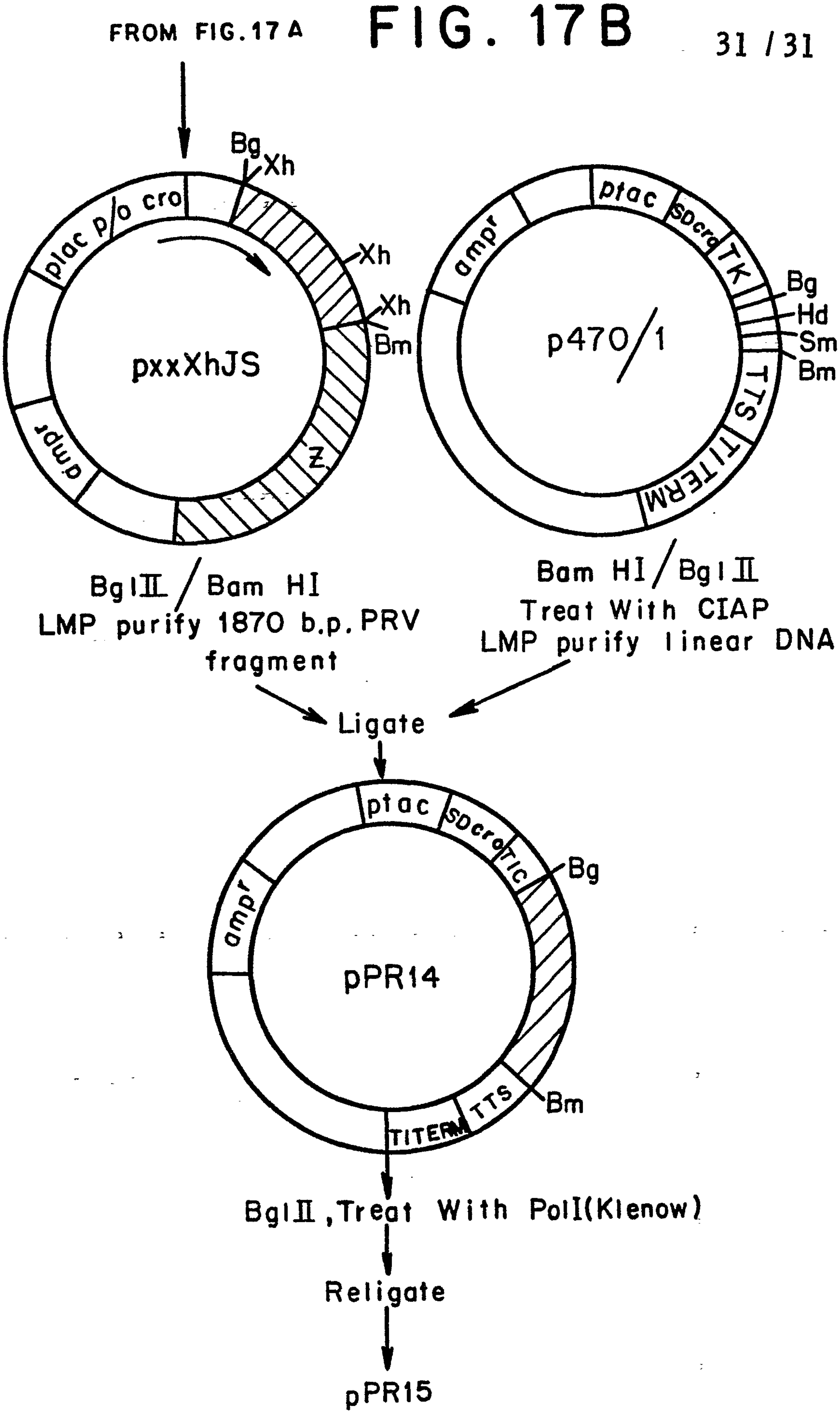

European Patent Office

# EUROPEAN SEARCH REPORT

0162738
Application number

EP 85400704.4

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | WO - A1 - 83/04 052 (TRANSGENE S.A.)<br>* Claims 1,20-24,26 *<br>-- | 1,2,12, 13,20, 31,37, 51,52, 89,90 | C 12 N 15/00<br>C 12 P 21/00<br>C 07 K 15/14<br>C 12 N 7/00<br>C 12 N 1/20<br>A 61 K 39/205<br>G 01 N 33/53<br>//C 12 R 1:19 |
| A,D, P | JOURNAL OF VIROLOGY, vol. 52, no. 2, November 1984, Washington D.C.<br>H. HAMPL et al. "Characterization of the envelope proteins of pseudorabies virus"<br>pages 583-590<br>* Totality *<br>---- | 1,89 | |
| | | | TECHNICAL FIELDS SEARCHED (Int. Cl.4) |
| | | | C 12 N<br>C 12 P<br>C 07 K<br>A 61 K<br>G 01 N |

The present search report has been drawn up for all claims

| Place of search | Date of completion of the search | Examiner |
|---|---|---|
| VIENNA | 10-07-1985 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03 82